# EUROPÄISCHE PATENTANMELDUNG

(11) **EP 4 745 232 A2**
(43) Veröffentlichungstag der Anmeldung: **20.05.2026**
(21) Anmeldenummer: 25208443.9
(22) Anmeldetag: 14.10.2025
(51) Int. Cl.: C12N 9/54, C11D 3/386

(54) **LEISTUNGSVERBESSERTE PROTEASE-VARIANTEN**

(30) Priorität: 14.11.2024 DE 102024133317
(71) Anmelder: Henkel AG & Co. KGaA, 40589 Düsseldorf (DE)
(72) Erfinder: Degering, Christian, 40699 Erkrath (DE); Wieland, Susanne, 41541 Zons/Dormagen (DE); Prueser, Inken, 40215 Düsseldorf (DE); Durmaz, Vedat, 66123 Saarbrücken (DE); Gruber, Christian, 8160 Weiz (AT); Steinkellner, Georg, 8573 Kainach bei Voitsberg (AT)

(57) **Zusammenfassung**

Die Erfindung betrifft Proteasen, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5% und 98% identisch ist, wobei die Protease bezogen auf die Nummerierung gemäß SEQ ID NO:1 an mindestens einer der Positionen, die den Positionen 97,3 99, 127 und 211 entsprechen, mindestens eine Aminosäuresubstitution, die aus der aus N97E, R99E, R99I, R99N, R99V, R99T, D127E und M211C bestehenden Gruppe ausgewählt ist, aufweist. Solche Proteasen sind für den Einsatz in Wasch- und Reinigungsmitteln, insbesondere flüssigen Textilwaschmitteln, geeignet und weisen eine gegenüber einer Referenzprotease verbesserte Reinigungsleistung auf. Die Erfindung betrifft ferner die Verwendung dieser Proteasen und Verfahren, in denen sie eingesetzt werden, sowie diese enthaltende Wasch- und Reinigungsmittel, insbesondere flüssige Textilwaschmittel.

## Beschreibung

Die Erfindung liegt auf dem Gebiet der Enzymtechnologie. Die Erfindung betrifft Proteasen, deren Aminosäuresequenz insbesondere im Hinblick auf den Einsatz in Wasch- und Reinigungsmitteln, insbesondere im Hinblick auf flüssige Textilwaschmittel, verändert wurden, um ihre Reinigungsleistung zu verbessern, und die für sie codierende Nukleinsäuren sowie deren Herstellung. Die Erfindung betrifft ferner die Verwendung dieser Proteasen und Verfahren, in denen sie eingesetzt werden, sowie diese enthaltende Wasch- und Reinigungsmittel, bevorzugt flüssige Wasch- und Reinigungsmittel, besonders bevorzugt flüssige Textilwaschmittel.

Proteasen gehören zu den technisch bedeutendsten Enzymen überhaupt. Für Wasch- und Reinigungsmittel sind sie die am längsten etablierten und in praktisch allen modernen, leistungsfähigen Wasch- und Reinigungsmitteln enthaltenen Enzyme. Sie bewirken den Abbau proteinhaltiger Anschmutzungen auf dem Reinigungsgut. Hierunter sind wiederum Proteasen vom Subtilisin-Typ (Subtilasen, Subtilopeptidasen, EC 3.4.21.62) besonders wichtig, welche aufgrund der katalytisch wirksamen Aminosäuren Serin-Proteasen sind. Sie wirken als unspezifische Endopeptidasen und hydrolysieren beliebige Säureamidbindungen, die im Inneren von Peptiden oder Proteinen liegen. Ihr pH-Optimum liegt meist im deutlich alkalischen Bereich. Einen Überblick über diese Familie bietet z.B. der Artikel "Subtilases: Subtilisin-like Proteases" von R. Siezen, Seiten 75-95 in "Subtilisin enzymes", herausgegeben von R. Bott und C. Betzel, New York, 1996. Subtilasen werden natürlicherweise von Mikroorganismen gebildet. Hierunter sind insbesondere die von *Bacillus-*Spezies gebildeten und sezernierten Subtilisine als bedeutendste Gruppe innerhalb der Subtilasen zu erwähnen.

Beispiele für die in Wasch- und Reinigungsmitteln bevorzugt eingesetzten Proteasen vom Subtilisin-Typ sind die Subtilisine BPN' und Carlsberg, die Protease PB92, die Subtilisine 147 und 309, die alkalische Protease aus *Bacillus lentus,* insbesondere aus *Bacillus lentus* DSM 5483, Subtilisin DY und die den Subtilasen, nicht mehr jedoch den Subtilisinen im engeren Sinne zuzuordnenden Enzyme Thermitase, Proteinase K und die Proteasen TW3 und TW7, sowie Varianten der genannten Proteasen, die eine gegenüber der Ausgangsprotease veränderte Aminosäuresequenz aufweisen. Proteasen werden durch aus dem Stand der Technik bekannte Verfahren gezielt oder zufallsbasiert verändert und so z.B. für den Einsatz in Wasch- und Reinigungsmitteln optimiert. Dazu gehören Punkt-, Deletions- oder Insertionsmutagenese oder Fusion mit anderen Proteinen oder Proteinteilen. So sind für die meisten aus dem Stand der Technik bekannten Proteasen entsprechend optimierte Varianten bekannt. In WO 2017/215925 ist z.B. eine für Wasch- und Reinigungsmittel, insbesondere Maschinengeschirrspülmittel, vorgesehene Protease aus *Bacillus gibsonii* bzw. Varianten davon offenbart.

Generell sind nur ausgewählte Proteasen für den Einsatz in flüssigen tensidhaltigen Zubereitungen überhaupt geeignet. Viele Proteasen zeigen in derartigen Zubereitungen keine ausreichende katalytische Leistung oder aber sie sind nicht ausreichend stabil. Für die Anwendung von Proteasen in Wasch- und Reinigungsmitteln ist daher eine hohe katalytische Aktivität und Stabilität unter Bedingungen, wie sie sich während eines Waschvorgangs darstellen, besonders wünschenswert. Folglich haben Protease- und Tensid-haltige flüssige Formulierungen aus dem Stand der Technik den Nachteil, dass die enthaltenen Proteasen unter Standardreinigungsbedingungen keine zufriedenstellende proteolytische Aktivität aufweisen und/oder nicht ausreichend lagerstabil sind, und die Formulierungen daher keine optimale Reinigungsleistung an proteasesensitiven Anschmutzungen zeigen. Proteasesensitive Anschmutzungen sind vorzugsweise aus der aus blut-, ei(gelb)-, milch-, fleisch- und anderen proteinhaltigen Anschmutzungen bestehenden Gruppe ausgewählt.

Das wichtigste Kriterium beim Reinigen von Textilien und/oder harten Oberflächen ist die Reinigungsleistung an verschiedensten Anschmutzungen. Auch wenn die Reinigungsleistung der heutzutage eingesetzten Wasch- und Reinigungsmittel generell hoch ist, stellt sich, auch aufgrund des generellen Trends vermehrt Niedrigtemperatur-Programme zu verwenden, allerdings das Problem, dass viele der üblichen Wasch- und Reinigungsmittel bei hartnäckigen Anschmutzungen eine unzureichende Reinigungsleistung aufweisen. Insbesondere ei(gelb)-haltige Anschmutzungen stellen eine Herausforderung dar. Eine solche unzureichende Reinigungsleistung führen beim Verbraucher zu Unzufriedenheit und dazu, dass solche Anschmutzungen vom Verbraucher vorbehandelt werden, was wiederum den Wasser- und Energieverbrauch erhöht. Hartnäckige Verschmutzungen werden in gängigen Wasch- und Reinigungsmittel zudem üblicherweise mittels weiterer reinigungsaktiver Substanzen im Wasch- und Reinigungsmittel, wie z.B. Phosphonate und/oder Phosphate, adressiert. Aufgrund von Nachhaltigkeitsstreben und Bedenken hinsichtlich der Umweltverträglichkeit von Phosphaten und Phosphonaten in Wasch- und Reinigungsmittel werden zunehmend mehr Wasch- und Reinigungsmittel entwickelt, die wenig(er) bis keine phosphat- und/oder phosphonathaltigen Verbindungen enthalten.

Es besteht weiterhin Bedarf, die Reinigungsleistung von enzymhaltigen, insbesondere proteasehaltigen, Wasch- und Reinigungsmitteln, insbesondere flüssigen Textilwaschmitteln, zu verbessern, insbesondere hinsichtlich der Reinigungsleistung an proteasesensitiven Anschmutzungen. Besonderes Augenmerk der vorliegenden Erfindung liegt auf der Verbesserung der Reinigungsleistung von proteasehaltigen Wasch- und Reinigungsmitteln, insbesondere flüssigen Textilwaschmitteln an proteinhaltigen, insbesondere blut-, ei(gelb)-, milch-, und/oder fleischhaltigen, bevorzugt ei(gelb)- und/oder milchhaltigen, Anschmutzungen. Vorzugsweise wird die Verbesserung der Reinigungsleistung in einem Temperaturbereich von etwa 20°C bis etwa 60°C, bevorzugt von etwa 20°C bis etwa 40°C, weiter bevorzugt von etwa 20°C bis etwa 30°C, besonders bevorzugt etwa 20°C. Ferner besteht weiterhin Bedarf, die Lagerstabilität der Protease in einem proteasehaltigen Wasch- und Reinigungsmittel, insbesondere flüssigen Textilwaschmittel, zu verbessern.

Überraschenderweise wurde jetzt festgestellt, dass eine Protease aus *Bacillus gibsonii* oder eine hierzu hinreichend ähnliche Protease (bezogen auf die Sequenzidentität), die bezogen auf die Nummerierung gemäß SEQ ID NO:1 an mindestens einer der Positionen, die den Positionen 97, 99, 127 und 211 entsprechen, mindestens eine Aminosäuresubstitution, die aus der aus N97E, R99E, R99I, R99N, R99V, R99T, D127E und M211C bestehenden Gruppe ausgewählt ist, aufweist, hinsichtlich ihrer Reinigungsleistung gegenüber einer Referenzprotease (insbesondere Wildtyp-Protease gemäß SEQ ID NO:1) verbessert ist, und daher besonders für den Einsatz in Wasch- und Reinigungsmitteln, insbesondere flüssigen Textilwaschmitteln, geeignet ist.

Gegenstand der Erfindung ist daher eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease bezogen auf die Nummerierung gemäß SEQ ID NO:1 an mindestens einer der Positionen, die den Positionen 97, 99, 127 und 211 entsprechen, mindestens eine Aminosäuresubstitution, die aus der aus N97E, R99E, R99I, R99N, R99V, R99T, D127E und M211C bestehenden Gruppe ausgewählt ist, aufweist.

Ein bevorzugter Gegenstand der Erfindung ist eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease bezogen auf die Nummerierung gemäß SEQ ID NO:1 an mindestens zwei der Positionen, die den Positionen 97, 99, 127 und 211 entsprechen, mindestens zwei Aminosäuresubstitutionen, die aus der aus N97E, R99E, R99I, R99N, R99V, R99T, D127E und M211C bestehenden Gruppe ausgewählt ist, aufweist.

Ein bevorzugter Gegenstand der Erfindung ist eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease bezogen auf die Nummerierung gemäß SEQ ID NO:1 an mindestens drei der Positionen, die den Positionen 97, 99, 127 und 211 entsprechen, mindestens drei Aminosäuresubstitutionen, die aus der aus N97E, R99E, R99I, R99N, R99V, R99T, D127E und M211C bestehenden Gruppe ausgewählt ist, aufweist.

Ein bevorzugter Gegenstand der Erfindung ist eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease bezogen auf die Nummerierung gemäß SEQ ID NO:1 an mindestens vier der Positionen, die den Positionen 97, 99, 127 und 211 entsprechen, mindestens vier Aminosäuresubstitutionen, die aus der aus N97E, R99E, R99I, R99N, R99V, R99T, D127E und M211C bestehenden Gruppe ausgewählt ist, aufweist.

Ein weiterer Gegenstand der Erfindung ist ein Verfahren zur Herstellung einer Protease, umfassend das Einbringen mindestens einer Aminosäuresubstitution an mindestens einer der Positionen, die bezogen auf die Nummerierung gemäß SEQ ID NO:1 den Positionen 97, 99, 127 und 211 entsprechen, ausgewählt aus der aus N97E, R99E, R99I, R99N, R99V, R99T, D127E und M211C bestehenden Gruppe, in ein Ausgangsmolekül, das eine Aminosäuresequenz aufweist, die mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99%, 99,5% oder 100% Sequenzidentität mit der in SEQ ID NO:1 angegebenen Aminosäuresequenz über deren Gesamtlänge aufweist.

Eine Protease im Sinne der vorliegenden Patentanmeldung umfasst daher sowohl die Protease als solche als auch eine mit einem erfindungsgemäßen Verfahren hergestellte Protease. Alle Ausführungen zur Protease beziehen sich daher sowohl auf die Protease als solche wie auch auf die mittels entsprechender Verfahren hergestellten Proteasen, sowie auf die entsprechenden Verfahren, insbesondere Herstellungsverfahren der Protease.

Weitere Aspekte der Erfindung betreffen die für diese Proteasen codierenden Nukleinsäuren, erfindungsgemäße Proteasen oder Nukleinsäuren enthaltende nicht-menschliche Wirtszellen sowie erfindungsgemäße Proteasen umfassende Wasch- und Reinigungsmittel, bevorzugt flüssige Wasch- und Reinigungsmittel, besonders bevorzugt flüssige Textilwaschmittel, Wasch- und Reinigungsverfahren, und Verwendung erfindungsgemäßer Proteasen in Wasch- und/oder Reinigungsmitteln zur Entfernung von proteasesensitiven Anschmutzungen, insbesondere ei(gelb)-, milch-, blut- und/oder fleischhaltigen Anschmutzungen, bevorzugt ei(gelb)- und/oder milchhaltigen Anschmutzungen.

Diese und weitere Aspekte, Merkmale und Vorteile der Erfindung werden für den Fachmann aus dem Studium der folgenden detaillierten Beschreibung und Ansprüche ersichtlich. Dabei kann jedes Merkmal aus einem Aspekt der Erfindung in jedem anderen Aspekt der Erfindung eingesetzt werden. Ferner ist es selbstverständlich, dass die hierin enthaltenen Beispiele die Erfindung beschreiben und veranschaulichen sollen, diese aber nicht einschränken und insbesondere die Erfindung nicht auf diese Beispiele beschränkt ist.

Alle Prozentangaben sind, sofern nicht anders angegeben, Gewichtsprozent (Gew.-%).

Numerische Bereiche, die in dem Format "von x bis y" angegeben sind, schließen die genannten Werte ein. Wenn mehrere bevorzugte numerische Bereiche in diesem Format angegeben sind, ist es selbstverständlich, dass alle Bereiche, die durch die Kombination der verschiedenen Endpunkte entstehen, ebenfalls erfasst werden.

"Mindestens ein(e)", wie hierin verwendet, bedeutet ein(e) oder mehrere, d.h. 1, 2, 3, 4, 5, 6, 7, 8, 9, 10, 11, 12, 13, 14 oder mehr.

Der Begriff "Wasch- und Reinigungsmittel" bzw. "Wasch- oder Reinigungsmittel", wie hierin verwendet, ist gleichbedeutend mit dem Begriff "Mittel" und bezeichnet eine Zusammensetzung zum Reinigen von Textilien und/oder harten Oberflächen, insbesondere Geschirr, wie in der Beschreibung erläutert.

"Etwa", "ca." oder "ungefähr", wie hierin in Bezug auf einen Zahlenwert verwendet, beziehen sich auf den entsprechenden Zahlenwert ±10%, vorzugsweise ±5%.

"Im Wesentlichen frei von" bedeutet, dass die Zusammensetzung bzw. das Mittel weniger als 2 Gew.-%, vorzugsweise weniger als 1 Gew.-%, weiter bevorzugt weniger als 0,5 Gew.-% und besonders bevorzugt weniger als 0,1 Gew.-%, der entsprechenden Substanz, bezogen auf das Gesamtgewicht der Zusammensetzung/des Mittels, enthält.

"Flüssig", wie hierin verwendet, schließt Flüssigkeiten und Gele sowie auch pastöse Zusammensetzungen ein. Es ist bevorzugt, dass die flüssigen Zusammensetzungen bei Raumtemperatur fließfähig und gießbar sind, es ist aber auch möglich, dass sie eine Fließgrenze aufweisen.

Ein Stoff, z.B. eine Zusammensetzung oder ein Mittel ist gemäß Definition der Erfindung festförmig, wenn sie bei 25°C und 1.013 mbar im festen Aggregatzustand vorliegt.

Ein Stoff, z.B. eine Zusammensetzung oder ein Mittel ist gemäß Definition der Erfindung flüssig, wenn sie bei 25°C und 1.013 mbar im flüssigen Aggregatzustand vorliegt. Dabei umfasst flüssig auch gelförmig.

"Variante", wie hierin verwendet, bezieht sich auf natürliche oder artifiziell erzeugte Variationen einer nativen Protease, die eine gegenüber der Referenzform abgewandelte Aminosäuresequenz aufweist.

Der Begriff "Textil", wie hierin verwendet, bezeichnet jedes textile Material, einschließlich Garne, Garnvorprodukte, Fasern, Vliesstoffe, natürliche Materialien, synthetische Materialien und alle anderen textilen Materialien, Gewebe aus diesen Materialien und aus Geweben hergestellte Produkte (z.B. Kleidungsstücke und andere Artikel). Das Textil oder Gewebe kann in Form von Gewirken, Geweben, Denims, Vliesstoffen, Filzen, Garnen und Frottee vorliegen. Das Textil kann auf Cellulose basieren, wie z.B. natürliche Cellulosefasern wie Baumwolle, Flachs/Leinen, Jute, Ramie, Sisal oder Kokosfasern, oder künstlich hergestellte Zellulosefasern (z.B. aus Zellstoff) wie Viskose/Rayon, Celluloseacetatfasern (Tricell), Lyocell oder Mischungen davon. Das Textil oder Gewebe kann auch aus Nicht-Cellulosefasern bestehen, z.B. aus natürlichen Polyamiden wie Wolle, Kamel, Kaschmir, Mohair, Kaninchen und Seide oder aus synthetischen Polymeren wie Nylon, Aramid, Polyester, Acryl, Polypropylen und Spandex/Elasthan oder Mischungen daraus sowie aus Mischungen von Cellulosefasern und Nicht-Cellulosefasern. Beispiele für Mischungen sind Mischungen aus Baumwolle und/oder Rayon/Viskose mit einem oder mehreren Begleitmaterialien wie Wolle, synthetischen Fasern (z.B. Polyamidfasern, Acrylfasern, Polyesterfasern, Polyvinylchloridfasern, Polyurethanfasern, Polyharnstofffasern, Aramidfasern) und/oder cellulosehaltigen Fasern (z.B. Rayon/Viskose, Ramie, Flachs/Leinen, Jute, Celluloseacetatfasern, Lyocell). Bei dem Gewebe kann es sich um herkömmliche waschbare Wäsche handeln, z.B. um verschmutzte Haushaltswäsche. Wenn der Begriff "Gewebe" oder "Kleidungsstück" verwendet wird, soll er auch den weiter gefassten Begriff "Textilien" umfassen.

Die vorliegende Erfindung basiert auf der überraschenden Erkenntnis der Erfinder, dass Aminosäuresubstitutionen an den hierin beschriebenen Positionen eine verbesserte Reinigungsleistung dieser veränderten Protease in Wasch- und Reinigungsmitteln, insbesondere flüssigen Textilwaschmitteln, bewirkt, vorzugsweise bei niedrigen Temperaturen von etwa 20°C bis etwa 40°C, bevorzugt bei etwa 20°C.

In bevorzugten Ausführungsformen führen die erfindungsgemäßen Veränderungen an mindestens einer der Positionen, die den Positionen 97, 99, 127 und 211 entsprechen, mindestens eine Aminosäuresubstitution, die aus der aus N97E, R99E, R99I, R99N, R99V, R99T, D127E und M211C bestehenden Gruppe ausgewählt ist, zu einer verbesserten Reinigungsleistung dieser veränderten Protease in Wasch- und Reinigungsmitteln, insbesondere flüssigen Textilwaschmitteln, an mindestens einer proteasesensitiven Anschmutzung, unter Standardwaschbedingungen.

In bevorzugten Ausführungsformen führen die erfindungsgemäßen Veränderungen an mindestens einer der Positionen, die den Positionen 97, 99, 127 und 211 entsprechen, mindestens eine Aminosäuresubstitution, die aus der aus N97E, R99E, R99I, R99N, R99V, R99T, D127E und M211C bestehenden Gruppe ausgewählt ist, zu einer verbesserten Reinigungsleistung dieser veränderten Protease in Wasch- und Reinigungsmitteln, insbesondere flüssigen Textilwaschmitteln, an ei(gelb)-, milch-, blut- und/oder fleischhaltigen, insbesondere ei(gelb)- und/oder milchhaltigen, Anschmutzungen unter Standardwaschbedingungen.

Das ist insbesondere insoweit überraschend, als dass bisher keine Protease mit solchen Veränderungen für die Verwendung in Wasch- oder Reinigungsmitteln, insbesondere flüssigen Textilwaschmitteln, beschrieben wurden. Insbesondere wurden solche erfindungsgemäß veränderten Proteasen nicht im Zusammenhang mit einer verbesserten Reinigungsleistung an ei(gelb)-, milch-, blut- und/oder fleischhaltigen, insbesondere ei(gelb)- und/oder milchhaltigen, Anschmutzungen beschrieben.

In bevorzugten Ausführungsformen weist die erfindungsgemäße Protease an mindestens einer der Positionen, die den Positionen 97, 99, 127 und 211 entsprechen, mindestens eine Aminosäuresubstitution, die aus der N97E, R99E, R99I, R99N, R99V, R99T, D127E und M211C bestehenden Gruppe ausgewählt ist, auf, wobei die mindestens eine Aminosäuresubstitution zu einer verbesserten Reinigungsleistung dieser veränderten Protease in Wasch- und Reinigungsmitteln, insbesondere flüssigen Textilwaschmitteln, an mindestens einer proteasesensitiven Anschmutzung führt.

In bevorzugten Ausführungsformen weist die erfindungsgemäße Protease an mindestens einer der Positionen, die den Positionen 97, 99, 127 und 211 entsprechen, mindestens eine Aminosäuresubstitution, die aus der aus N97E, R99E, R99I, R99N, R99V, R99T, D127E und M211C bestehenden Gruppe ausgewählt ist, auf, wobei die mindestens eine Aminosäuresubstitution zu einer verbesserten Reinigungsleistung dieser veränderten Protease in Wasch- und Reinigungsmitteln, insbesondere flüssigen Textilwaschmitteln, an ei(gelb)-, milch-, blut- und/oder fleischhaltigen, insbesondere ei(gelb)- und/oder milchhaltigen, Anschmutzungen führt.

In bevorzugten Ausführungsformen führen die hierin beschriebenen erfindungsgemäßen Aminosäuresubstitutionen bzw. Aminosäuresubstitutionskombinationen zu einer verbesserten Reinigungsleistung dieser veränderten Protease in Wasch- und Reinigungsmitteln, insbesondere flüssigen Textilwaschmittel, an mindestens einer proteasesensitiven Anschmutzung, vorzugsweise ei(gelb)-, milch-, blut- und/oder fleischhaltigen, insbesondere ei(gelb)- und/oder milchhaltigen, Anschmutzungen. Erfindungsgemäße Proteasen ermöglichen folglich eine verbesserte Entfernung von mindestens einer, bevorzugt mehreren, proteasesensitiven Anschmutzungen auf Textilien und/oder harten Oberflächen, insbesondere Geschirr. Typische proteasesensitive Anschmutzungen umfassen z.B. ei(gelb)-, blut-, milch-, fleisch- und andere proteinhaltige Anschmutzungen. Besonders bevorzugt ermöglichen erfindungsgemäße Proteasen eine verbesserte Entfernung von ei(gelb)-, milch-, blut- und/oder fleischhaltigen, insbesondere ei(gelb) und/oder milchhaltigen, Anschmutzungen auf Textilien. Eine erfindungsgemäße Verbesserung der Reinigungsleistung, insbesondere der proteolytischen Reinigungsleistung, liegt dann vor, wenn die Protease an mindestens einer proteasesensitiven Anschmutzung, die vorzugsweise aus der aus blut-, ei(gelb)-, milch-, fleisch- und anderen proteinhaltigen Anschmutzungen bestehenden Gruppe ausgewählt ist, eine verbesserte Reinigungsleistung gegenüber einer Referenzprotease (insbesondere Wildtyp-Protease gemäß SEQ ID NO:1) zeigt, wie hierin beschrieben.

Unter Wasch- bzw. Reinigungsleistung wird im Rahmen der Erfindung das Vermögen eines Wasch- oder Reinigungsmittels, eine vorhandene Anschmutzung teilweise oder vollständig zu entfernen, d.h. die Aufhellungsleistung an einer oder mehreren Anschmutzung(en), insbesondere auf Textilien, Wäsche oder Geschirr, verstanden. Im Rahmen der Erfindung weist sowohl das Wasch- und/oder Reinigungsmittel, welches die Protease umfasst bzw. die durch dieses Mittel gebildete Wasch- oder Reinigungsflotte, als auch die Protease selbst eine jeweilige Reinigungsleistung auf. Die Reinigungsleistung des Enzyms trägt somit zur Reinigungsleistung des Mittels bzw. der durch das Mittel gebildeten Wasch- oder Reinigungsflotte bei. Die Reinigungsleistung wird bevorzugt ermittelt wie weiter unten angegeben.

Unter Wasch- bzw. Reinigungsflotte wird diejenige das Wasch- oder Reinigungsmittel enthaltende Gebrauchslösung verstanden, die auf Textilien oder Gewebe bzw. harte Oberflächen, insbesondere Geschirr, einwirkt und damit mit den auf Textilien bzw. Geweben oder harten Oberflächen, insbesondere Geschirr, vorhandenen Anschmutzungen in Kontakt kommt. Üblicherweise entsteht die Wasch- bzw. Reinigungsflotte, wenn der Wasch- oder Reinigungsvorgang beginnt und das Wasch- oder Reinigungsmittel z.B. in einer Geschirrspülmaschine, einer Waschmaschine oder in einem anderen geeigneten Behältnis mit Wasser verdünnt wird.

Die Reinigungsleistung an Textilien oder Geweben kann in einem Waschsystem bestimmt werden, das ein Waschmittel in einer Dosierung zwischen 2,0 und 8,0 Gramm pro Liter Waschflotte enthält. Die zu vergleichenden Proteasen werden konzentrationsgleich (bezogen auf aktives Protein) eingesetzt. Die Konzentration der Protease in dem für ein solches Waschsystem bestimmten Waschmittel beträgt 0,001 bis 0,1 Gew.-%, vorzugsweise 0,01 bis 0,06 Gew.-%, bezogen auf aktives Protein.

Ein flüssiges Referenzwaschmittel für ein solches Waschsystem kann z.B. wie folgt zusammengesetzt sein (alle Angaben in Gew.-%): 4,4% Alkylbenzolsulfonsäure, 5,6% weitere anionische Tenside, 2,4% C₁₂₋₁₈ Na-Salze von Fettsäuren (Seifen), 4,4% nicht-ionische Tenside, 0,2% Phosphonate, 1,4% Zitronensäure, 0,95% NaOH, 0,01% Entschäumer, 2% Glycerin, 0,08% Konservierungsstoffe, 1% Ethanol, Rest demineralisiertes Wasser. Bevorzugt beträgt die Dosierung des flüssigen Waschmittels zwischen 3,0 und 6,0 Gramm pro Liter Waschflotte, z.B. 3,0, 3,2, 3,5, 3,7, 4,0, 4,5, 4,7, 4,9 oder 5,9 Gramm pro Liter Waschflotte. Bevorzugt wird gewaschen in einem pH-Wertebereich zwischen pH 7 und pH 10,5, bevorzugt zwischen pH 8 und pH 9.

Alternativ kann ein flüssiges Referenzwaschmittel für ein solches Waschsystem wie folgt zusammengesetzt sein (alle Angaben in Gew.-%): 0,3-0,5% Xanthan, 0,2-0,4% Anti-Schaummittel, 6-7% Glycerin, 0,3-0,5% Ethanol, 4-7% FAEOS (Fettalkoholethersulfat), 5-15% nichtionische Tenside, 5-15% anionische Tenside (LAS), 1% Borsäure, 1-4% Natriumcitrat (Dihydrat), 2-4% Soda, 2-6% Kokosnuss-Fettsäuren, 0,5-2,5% HEDP (1-Hydroxyethan-(1,1-di-phosphonsäure)), 0-0,4% PVP (Polyvinylpyrrolidon), 0-0,15% optischer Aufheller, 0-0,001% Farbstoff, Rest demineralisiertes Wasser. Bevorzugt beträgt die Dosierung des flüssigen Waschmittels zwischen 3,5 und 6,0 Gramm pro Liter Waschflotte, z.B. 4,7, 4,9 oder 5,9 Gramm pro Liter Waschflotte. Bevorzugt wird gewaschen in einem pH-Wertebereich zwischen pH 8 und pH 10,5, bevorzugt zwischen pH 8 und pH 9.

Ein pulverförmiges Referenzwaschmittel für ein solches Waschsystem kann wie folgt zusammengesetzt sein (alle Angaben in Gew.-%): 10% lineares Alkylbenzolsulfonat (Natrium-Salz), 1,5% C₁₂₋₁₈-Fettalkoholsulfat (Natrium-Salz), 2,0% C₁₂₋₁₈-Fettalkohol mit 7 EO, 20% Natriumcarbonat, 6,5% Natriumhydrogencarbonat, 4,0% amorphes Natriumdisilikat, 17% Natriumcarbonatperoxohydrat, 4,0% TAED, 3,0% Polyacrylat, 1,0% Carboxymethylcellulose, 1,0% Phosphonat, 27% Natriumsulfat, Rest: Schauminhibitoren, optischer Aufheller, Duftstoffe. Bevorzugt beträgt die Dosierung des pulverförmigen Waschmittels zwischen 4,5 und 7,0 Gramm pro Liter Waschflotte, z.B. und besonders bevorzugt 4,7 Gramm pro Liter Waschflotte, oder 5,5, 5,9 oder 6,7 Gramm pro Liter Waschflotte. Bevorzugt wird gewaschen in einem pH-Wertebereich zwischen pH 9 und pH 11.

Die Reinigungsleistung wird gegenüber einer Anschmutzung auf Textil (z.B. Baumwoll- oder Baumwollmischgewebe) durch Messung des Reinigungsgrades der gewaschenen Textilien bestimmt. Beispielsweise kann der Waschvorgang für 60 Minuten bei einer Temperatur von etwa 20°C oder etwa 40°C erfolgen und das Wasser eine Wasserhärte zwischen 15,5°dH und 16,5°dH (deutsche Härte) aufweisen. Im Rahmen der Erfindung erfolgt die Bestimmung der Reinigungsleistung z.B. bei 20°C oder 40°C unter Verwendung eines flüssigen Waschmittels wie hierin angegebenen, wobei der Waschvorgang vorzugsweise für 60 Minuten bei 600 rpm erfolgt.

Der Weißegrad, d.h. die Aufhellung der Anschmutzungen, als Maß für die Reinigungsleistung wird mit optischen Messverfahren bestimmt, bevorzugt photometrisch. Ein hierfür geeignetes Gerät ist z.B. das Spektrometer Minolta CM508d. Üblicherweise werden die für die Messung eingesetzten Geräte zuvor mit einem Weißstandard, bevorzugt einem mitgelieferten Weißstandard, kalibriert.

Die Reinigungsleistung eines Geschirrspülmittels kann in einem System bestimmt werden, das ein Maschinengeschirrspülmittel in einer Dosierung wie hierin angegeben sowie die erfindungsgemäße Protease enthält, wobei die zu vergleichenden Proteasen konzentrationsgleich (bezogen auf aktives Protein) eingesetzt sind und die Reinigungsleistung gegenüber einer Anschmutzung von Tee, Fleisch, Spaghetti und/oder Creme Brûlée gemäß IKW Methode (Recommendations for the Quality Assessment of the Cleaning Performance of Dishwasher Detergents (Part B, Update 2015), sofwjournal, 142, 06/16, 34-48) in einer Miele GSL (Programm 45°C, 21°dH) bestimmt wird. Die Konzentration der Protease in dem für dieses Waschsystem bestimmten Mittels beträgt 0,001 bis 0,1 Gew.-%, vorzugsweise 0,01 bis 0,06 Gew.-%, bezogen auf aktives, gereinigtes Protein.

### Flüssiges Geschirrspülmittel (Zweikomponentenformulierung):

| **Enzymphase (EP) - Zubereitung A** | **Aktivstoffgehalt in Gew.-%** |
|---|---|
| Phosphonat (z.B. HEDP), sofern regulatorisch zulässig | 0,00-7,50 |
| CaCl₂ | 0,05-1,50 |
| Amylasehaltige Enzymzusammensetzung (t.q.) | 0,00-4,00 |
| Proteasehaltige Enzymzusammensetzung (t.q.) | 0,00001-10 |
| Sorbitol | 2,00-10,00 |
| Sulfonsäuregruppen-haltiges Polymer | 0,00-12,00 |
| Verdicker (auf Acrylatbasis oder Xanthan Gum) | 0,01-6,00 |
| GLDA oder MGDA | 3,00-25,00 |
| KOH | 0,50-4,00 |
| Nichtionische Tenside | 1,00-6,00 |
| Natriumcitrat | 2,00-20,00 |
| Zinksalz | 0,00-1,00 |
| Reste (Parfüm, Farbstoffe, Konservierungsstoffe, Wasser, Enzymstabilisator) (Gew.-%) | ad 100 |

| **Alkaliphase (AP) Zubereitung B** | |
|---|---|
| Phosphonat, sofern regulatorisch zulässig | 0,00-7,50 |
| Verdicker (Acrylat oder Xanthan) | 0,01-6,00 |
| GLDA oder MGDA | 3,00-25,00 |
| KOH | 0,50-4,00 |
| Soda | 5,00-20,00 |
| Monoethanolamin | 0,00-5,00 |
| Acrylatpolymer | 0,00-3,00 |
| Natriumcitrat | 2,00-20,00 |
| Reste (Parfüm, Farbstoffe, Konservierungsstoffe, Wasser, etc.) (Gew.-%) | ad 100 |

### Festes Geschirrspülmittel:

| | Gew.-% |
|---|---|
| Citrat, Na-Salz | 15-20 |
| Phosphonat (z.B. HEDP), sofern regulatorisch zulässig | 0-7,5 (2,5-7,5) |
| MGDA, Na-Salz | 0-25 |
| Disilicat, Na-Salz | 5-35 |
| Soda | 10-25 |
| Silberschutz | 0,0-1,0 |
| Percarbonat, Na-Salz | 10-15 |
| Bleichkatalysator (bevorzugt Mn-basiert) | 0,02-0,5 |
| Bleichaktivator (z.B. TAED) | 1-3 |
| Nichtionische(s) Tensid(e), z.B. Fettalkoholalkoxylat, bevorzugt 20-40 EO, ggf. endcapped | 2,5-10 |
| Polycarboxylat | 4-10 |
| Kationisches Copolymer | 0-0,75 |
| Disintegrant - (z.B. Crosslinked PVP) | 0-1,5 |
| Protease-Zubereitung (t.q.) | 0-5 |
| Amylase-Zubereitung (t.q.) | 0-3 |
| Parfüm | 0,05-0,25 |
| Farbstofflösung | 0,0-1 |
| Zink-Salz | 0,1-0,3 |
| Natriumsulfat | 0,0-10 |
| Wasser | 0,0-1,5 |
| pH-Stellmittel (z.B. Citronensäure) | 0-1,5 |
| Prozesshilfsmittel | 0-5 |

Durch den aktivitätsgleichen Einsatz der jeweiligen Enzyme wird sichergestellt, dass auch bei einem etwaigen Auseinanderklaffen des Verhältnisses von Aktivsubstanz zu Gesamtprotein (die Werte der spezifischen Aktivität) die jeweiligen enzymatischen Eigenschaften, also z.B. die Reinigungsleistung an bestimmten Anschmutzungen, verglichen werden. Generell gilt, dass eine niedrige spezifische Aktivität durch Zugabe einer größeren Proteinmenge ausgeglichen werden kann. Ferner können die zu untersuchenden Enzyme auch in gleicher Stoffmenge oder Gewichtsmenge eingesetzt werden, falls die zu untersuchenden Enzyme in einem Aktivitätstest eine unterschiedliche Affinität an das Testsubstrat aufweisen. Der Ausdruck "gleiche Stoffmenge" bezieht sich in diesem Zusammenhang auf eine molgleiche Verwendung der zu untersuchenden Enzyme. Der Ausdruck "gleiche Gewichtsmenge" bezieht sich auf einen gewichtsgleichen Einsatz der zu untersuchenden Enzyme.

Bevorzugte Ausführungsformen erfindungsgemäßer Proteasen erzielen solche vorteilhaften Reinigungsleistungen auch schon bei niedrigen Temperaturen, insbesondere in den Temperaturbereichen zwischen etwa 20°C und etwa 60°C, bevorzugt zwischen etwa 20°C und etwa 40°C, weiter bevorzugt zwischen etwa 20°C und etwa 30°C und besonders bevorzugt etwa 20°C.

Verfahren zur Bestimmung der Protease-Aktivität dem Fachmann auf dem Gebiet der Enzymtechnologie geläufig und werden von ihm routinemäßig angewendet. Beispielsweise sind solche Verfahren offenbart in Tenside, Band 7 (1970), S. 125-132. Alternativ kann die Protease-Aktivität über die Freisetzung des Chromophors para-Nitroanilin (pNA) aus dem Substrat suc-L-Ala-L-Ala-L-Pro-L-Phe-p-Nitroanilid (AAPF) bestimmt werden. Die Protease spaltet das Substrat und setzt pNA frei. Die Freisetzung des pNA verursacht eine Zunahme der Extinktion bei 410 nm, deren zeitlicher Verlauf ein Maß für die enzymatische Aktivität ist (vgl. Del Mar et al., 1979). Die Messung erfolgt bei einer Temperatur von 25°C, bei pH 8,6, und einer Wellenlänge von 410 nm. Die Messzeit beträgt 5 min und das Messintervall 20 s bis 60 s. Die Protease-Aktivität wird üblicherweise in Protease-Einheiten (PE) angegeben. Geeignete Protease-Aktivitäten betragen z.B. 2,25, 5 oder 10 PE pro ml Waschflotte. Die Protease-Aktivität ist jedoch nicht gleich Null.

Ein alternativer Test zur Feststellung der proteolytischen Aktivität der erfindungsgemäßen Proteasen ist ein optisches Messverfahren, bevorzugt ein photometrisches Verfahren. Der hierfür geeignete Test umfasst die Protease-abhängige Spaltung des Substratproteins Casein. Dieses wird durch die Protease in eine Vielzahl kleinerer Teilprodukte gespalten. Die Gesamtheit dieser Teilprodukte weist eine erhöhte Absorption bei 290 nm gegenüber nicht gespaltenem Casein auf, wobei diese erhöhte Absorption unter Verwendung eines Photometers ermittelt werden und somit ein Rückschluss auf die enzymatische Aktivität der Protease gezogen werden kann.

Die Proteinkonzentration kann mit Hilfe bekannter Methoden, z.B. dem BCA-Verfahren (Bicinchoninsäure; 2,2'-Bichinolyl-4,4'-dicarbonsäure) oder dem Biuret-Verfahren (Gornall et al., J. Biol. Chem. 177 (1948): 751-766), bestimmt werden. Die Bestimmung der Aktivproteinkonzentration kann diesbezüglich über eine Titration der aktiven Zentren unter Verwendung eines geeigneten irreversiblen Inhibitors und Bestimmung der Restaktivität (vgl. Bender et al., J. Am. Chem. Soc. 88, 24 (1966): 5890-5913) erfolgen.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease bezogen auf die Nummerierung gemäß SEQ ID NO:1 an mindestens einer der Positionen, die den Positionen 97, 99, 127 und 211 entsprechen, mindestens eine Aminosäuresubstitution, die aus der aus N97E, R99E, R99I, R99N, R99V, R99T, D127E und M211C bestehenden Gruppe ausgewählt ist, aufweist.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease bezogen auf die Nummerierung gemäß SEQ ID NO:1 an mindestens zwei der Positionen, die den Positionen 97, 99, 127 und 211 entsprechen, mindestens zwei Aminosäuresubstitutionen, die aus der aus N97E, R99E, R99I, R99N, R99V, R99T, D127E und M211C bestehenden Gruppe ausgewählt sind, aufweist.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease bezogen auf die Nummerierung gemäß SEQ ID NO:1 an mindestens drei der Positionen, die den Positionen 97, 99, 127 und 211 entsprechen, mindestens drei Aminosäuresubstitutionen, die aus der aus N97E, R99E, R99I, R99N, R99V, R99T, D127E und M211C bestehenden Gruppe ausgewählt sind, aufweist.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease bezogen auf die Nummerierung gemäß SEQ ID NO:1 an mindestens vier der Positionen, die den Positionen 97, 99, 127 und 211 entsprechen, mindestens vier Aminosäuresubstitutionen, die aus der aus N97E, R99E, R99I, R99N, R99V, R99T, D127E und M211C bestehenden Gruppe ausgewählt sind, aufweist.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease bezogen auf die Nummerierung gemäß SEQ ID NO:1 an mindestens zwei der Positionen, die den Positionen 97, 99, 127 und 211 entsprechen, mindestens zwei Aminosäuresubstitutionen, die aus der aus N97E, R99E, R99I, R99N, R99V, R99T, D127E und M211C bestehenden Gruppe ausgewählt sind, aufweist, und wobei die Aminosäuresubstitutionskombination aus der aus N97E-R99E, N97ER99I, N97E-R99N, N97E-R99V, N97E-R99T, N97E-D127E, N97E-M211C, R99E-D127E, R99E-M211C, R99I-D127E, R99I-M211C, R99N-D127E, R99N-M211C, R99V-D127E, R99V-M211C, R99T-D127E, R99T-M211C, D127E-M211C bestehenden Kombination ausgewählt ist.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease bezogen auf die Nummerierung gemäß SEQ ID NO:1 an mindestens drei der Positionen, die den Positionen 97, 99, 127 und 211 entsprechen, mindestens drei Aminosäuresubstitutionen, die aus der aus N97E, R99E, R99I, R99N, R99V, R99T, D127E und M211C bestehenden Gruppe ausgewählt sind, aufweist, und wobei die Aminosäuresubstitutionskombination aus der aus N97E-R99E-D127E, N97E-R99I-D127E, N97E-R99N-D127E, N97E-R99V-D127E, N97E-R99T-D127E, N97E-M211CD127E, N97E-R99E-M211C, N97E-R99I-M211C, N97E-R99N-M211C, N97E-R99V-M211C, N97ER99T-M211C, N97E-D127E-M211C, R99E-D127E-M211C, R99I-D127E-M211C, R99N-D127E-M211C, R99V-D127E-M211C, R99T-D127E-M211C bestehenden Kombination ausgewählt ist.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease bezogen auf die Nummerierung gemäß SEQ ID NO:1 an mindestens vier der Positionen, die den Positionen 97, 99, 127 und 211 entsprechen, mindestens vier Aminosäuresubstitutionen, die aus der aus N97E, R99E, R99I, R99N, R99V, R99T, D127E und M211C bestehenden Gruppe ausgewählt sind, aufweist, und wobei die Aminosäuresubstitutionskombination aus der aus N97E-R99E-D127E-M211C, N97E-R991-D127E-M211C, N97E-R99N-D127E-M211C, N97E-R99V-D127E-M211C, N97ER99T-D127E-M211C bestehenden Kombination ausgewählt ist.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease eine Aminosäuresubstitution bzw. Aminosäuresubstitutionskombination aufweist, die aus der aus N97E, R99E, R99I, R99N, R99V, R99T, D127E, M211C, N97E-R99E, N97E-R99I, N97E-R99N, N97E-R99V, N97E-R99T, N97E-D127E, N97E-M211C, R99E-D127E, R99E-M211C, R99I-D127E, R99I-M211C, R99N-D127E, R99N-M211C, R99V-D127E, R99V-M211C, R99T-D127E, R99T-M211C, D127E-M211C, N97E-R99E-D127E, N97E-R99I-D127E, N97E-R99N-D127E, N97E-R99V-D127E, N97ER99T-D127E, N97E-M211C-D127E, N97E-R99E-M211C, N97E-R99I-M211C, N97E-R99N-M211C, N97E-R99V-M211C, N97E-R99T-M211C, N97E-D127E-M211C, R99E-D127E-M211C, R99I-D127E-M211C, R99N-D127E-M211C, R99V-D127E-M211C, R99T-D127E-M211C, N97E-R99E-D127E-M211C, N97E-R99I-D127E-M211C, N97E-R99N-D127E-M211C, N97E-R99V-D127E-M211C und N97E-R99T-D127E-M211C, bevorzugt N97E, R99E, R99I, R99N, R99V, R99T, D127E, M211C, D127E-M211C und N97E-R99I bestehenden Kombination ausgewählt ist.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease eine Aminosäuresubstitution bzw. Aminosäuresubstitutionskombination aufweist, die aus der aus N97E, R99E, R99I, R99N, R99V, R99T, D127E, M211C, D127E-M211C und N97E-R99I bestehenden Kombination ausgewählt ist.

In besonders bevorzugten Ausführungsformen umfasst die erfindungsgemäße Protease eine Aminosäuresubstitutionskombination, die aus der aus N97E, R99E, R99I, R99N, R99V, R99T, D127E, M211C, N97E-R99E, N97E-R99I, N97E-R99N, N97E-R99V, N97E-R99T, N97E-D127E, N97E-M211C, R99E-D127E, R99E-M211C, R99I-D127E, R99I-M211C, R99N-D127E, R99N-M211C, R99V-D127E, R99V-M211C, R99T-D127E, R99T-M211C, D127E-M211C, N97E-R99E-D127E, N97E-R99I-D127E, N97E-R99N-D127E, N97E-R99V-D127E, N97E-R99T-D127E, N97E-M211C-D127E, N97ER99E-M211C, N97E-R99I-M211C, N97E-R99N-M211C, N97E-R99V-M211C, N97E-R99T-M211C, N97E-D127E-M211C, R99E-D127E-M211C, R99I-D127E-M211C, R99N-D127E-M211C, R99V-D127E-M211C, R99T-D127E-M211C, N97E-R99E-D127E-M211C, N97E-R99I-D127E-M211C, N97ER99N-D127E-M211C, N97E-R99V-D127E-M211C und N97E-R99T-D127E-M211C, bevorzugt N97E, R99E, R99I, R99N, R99V, R99T, D127E, M211C, D127E-M211C und N97E-R99I, bestehenden Gruppe ausgewählt ist, wobei die Nummerierung jeweils bezogen auf die Nummerierung gemäß SEQ ID NO:1 ist und wobei die Protease neben den genannten Aminosäuresubstitutionen keine weiteren Änderungen umfasst.

In besonders bevorzugten Ausführungsformen umfasst die erfindungsgemäße Protease eine Aminosäuresubstitutionskombination, die aus der aus N97E, R99E, R99I, R99N, R99V, R99T, D127E, M211C, D127E-M211C und N97E-R99I bestehenden Gruppe ausgewählt ist, wobei die Nummerierung jeweils bezogen auf die Nummerierung gemäß SEQ ID NO:1 ist und wobei die Protease neben den genannten Aminosäuresubstitutionen keine weiteren Änderungen umfasst.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease bezogen auf die Nummerierung gemäß SEQ ID NO:1 an mindestens einer der Positionen, die den Positionen 97, 99, 127 und 211 entsprechen, mindestens eine Aminosäuresubstitution, die aus der aus N97E, R99E, R99I, R99N, R99V, R99T, D127E und M211C bestehenden Gruppe ausgewählt ist, aufweist, wobei die Protease an mindestens einer proteasesensitiven Anschmutzung, die vorzugsweise aus der aus blut-, ei(gelb)-, milch-, fleisch- und anderen proteinhaltigen Anschmutzungen, bevorzugt ei(gelb)- und/oder milchhaltigen Anschmutzungen, bestehenden Gruppe ausgewählt ist, eine verbesserte Reinigungsleistung gegenüber einer Referenzprotease (insbesondere Wildtyp-Protease gemäß SEQ ID NO:1) zeigt, wobei die Reinigungsleistung, wie in Beispiel 2 beschrieben, bestimmt wird.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease bezogen auf die Nummerierung gemäß SEQ ID NO:1 an mindestens zwei der Positionen, die den Positionen 97, 99, 127 und 211 entsprechen, mindestens zwei Aminosäuresubstitutionen, die aus der aus N97E, R99E, R99I, R99N, R99V, R99T, D127E und M211C bestehenden Gruppe ausgewählt ist, aufweist, wobei die Protease an mindestens einer proteasesensitiven Anschmutzung, die vorzugsweise aus der aus blut-, ei(gelb)-, milch-, fleisch- und anderen proteinhaltigen Anschmutzungen, bevorzugt ei(gelb)- und/oder milchhaltigen Anschmutzungen, bestehenden Gruppe ausgewählt ist, eine verbesserte Reinigungsleistung gegenüber einer Referenzprotease (insbesondere Wildtyp-Protease gemäß SEQ ID NO:1) zeigt, wobei die Reinigungsleistung, wie in Beispiel 2 beschrieben, bestimmt wird.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease bezogen auf die Nummerierung gemäß SEQ ID NO:1 an mindestens drei der Positionen, die den Positionen 97, 99, 127 und 211 entsprechen, mindestens drei Aminosäuresubstitutionen, die aus der aus N97E, R99E, R99I, R99N, R99V, R99T, D127E und M211C bestehenden Gruppe ausgewählt ist, aufweist, wobei die Protease an mindestens einer proteasesensitiven Anschmutzung, die vorzugsweise aus der aus blut-, ei(gelb)-, milch-, fleisch- und anderen proteinhaltigen Anschmutzungen, bevorzugt ei(gelb)- und/oder milchhaltigen Anschmutzungen, bestehenden Gruppe ausgewählt ist, eine verbesserte Reinigungsleistung gegenüber einer Referenzprotease (insbesondere Wildtyp-Protease gemäß SEQ ID NO:1) zeigt, wobei die Reinigungsleistung, wie in Beispiel 2 beschrieben, bestimmt wird.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease bezogen auf die Nummerierung gemäß SEQ ID NO:1 an mindestens vier der Positionen, die den Positionen 97, 99, 127 und 211 entsprechen, mindestens vier Aminosäuresubstitutionen, die aus der aus N97E, R99E, R99I, R99N, R99V, R99T, D127E und M211C bestehenden Gruppe ausgewählt ist, aufweist, wobei die Protease an mindestens einer proteasesensitiven Anschmutzung, die vorzugsweise aus der aus blut-, ei(gelb)-, milch-, fleisch- und anderen proteinhaltigen Anschmutzungen, bevorzugt ei(gelb)- und/oder milchhaltigen Anschmutzungen, bestehenden Gruppe ausgewählt ist, eine verbesserte Reinigungsleistung gegenüber einer Referenzprotease (insbesondere Wildtyp-Protease gemäß SEQ ID NO:1) zeigt, wobei die Reinigungsleistung, wie in Beispiel 2 beschrieben, bestimmt wird.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease bezogen auf die Nummerierung gemäß SEQ ID NO:1 an mindestens zwei der Positionen, die den Positionen 97, 99, 127 und 211 entsprechen, mindestens zwei Aminosäuresubstitutionen, die aus der aus N97E, R99E, R99I, R99N, R99V, R99T, D127E und M211C bestehenden Gruppe ausgewählt sind, aufweist, und wobei die Aminosäuresubstitutionskombination aus der aus N97E-R99E, N97ER99I, N97E-R99N, N97E-R99V, N97E-R99T, N97E-D127E, N97E-M211C, R99E-D127E, R99E-M211C, R99I-D127E, R99I-M211C, R99N-D127E, R99N-M211C, R99V-D127E, R99V-M211C, R99T-D127E, R99T-M211C, D127E-M211C bestehenden Kombination ausgewählt ist, wobei die Protease an mindestens einer proteasesensitiven Anschmutzung, die vorzugsweise aus der aus blut-, ei(gelb)-, milch-, fleisch- und anderen proteinhaltigen Anschmutzungen, bevorzugt ei(gelb)- und/oder milchhaltigen Anschmutzungen, bestehenden Gruppe ausgewählt ist, eine verbesserte Reinigungsleistung gegenüber einer Referenzprotease (insbesondere Wildtyp-Protease gemäß SEQ ID NO:1) zeigt, wobei die Reinigungsleistung, wie in Beispiel 2 beschrieben, bestimmt wird.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease bezogen auf die Nummerierung gemäß SEQ ID NO:1 an mindestens drei der Positionen, die den Positionen 97, 99, 127 und 211 entsprechen, mindestens drei Aminosäuresubstitutionen, die aus der aus N97E, R99E, R99I, R99N, R99V, R99T, D127E und M211C bestehenden Gruppe ausgewählt sind, aufweist, und wobei die Aminosäuresubstitutionskombination aus der aus N97E-R99E-D127E, N97E-R99I-D127E, N97E-R99N-D127E, N97E-R99V-D127E, N97E-R99T-D127E, N97E-M211CD127E, N97E-R99E-M211C, N97E-R99I-M211C, N97E-R99N-M211C, N97E-R99V-M211C, N97ER99T-M211C, N97E-D127E-M211C, R99E-D127E-M211C, R991-D127E-M211C, R99N-D127E-M211C, R99V-D127E-M211C, R99T-D127E-M211C bestehenden Kombination ausgewählt ist, wobei die Protease an mindestens einer proteasesensitiven Anschmutzung, die vorzugsweise aus der aus blut-, ei(gelb)-, milch-, fleisch- und anderen proteinhaltigen Anschmutzungen, bevorzugt ei(gelb)- und/oder milchhaltigen Anschmutzungen, bestehenden Gruppe ausgewählt ist, eine verbesserte Reinigungsleistung gegenüber einer Referenzprotease (insbesondere Wildtyp-Protease gemäß SEQ ID NO:1) zeigt, wobei die Reinigungsleistung, wie in Beispiel 2 beschrieben, bestimmt wird.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease bezogen auf die Nummerierung gemäß SEQ ID NO:1 an mindestens vier der Positionen, die den Positionen 97, 99, 127 und 211 entsprechen, mindestens vier Aminosäuresubstitutionen, die aus der aus N97E, R99E, R99I, R99N, R99V, R99T, D127E und M211C bestehenden Gruppe ausgewählt sind, aufweist, und wobei die Aminosäuresubstitutionskombination aus der aus N97E-R99E-D127E-M211C, N97E-R99I-D127E-M211C, N97E-R99N-D127E-M211C, N97E-R99V-D127E-M211C, N97ER99T-D127E-M211C bestehenden Kombination ausgewählt ist, wobei die Protease an mindestens einer proteasesensitiven Anschmutzung, die vorzugsweise aus der aus blut-, ei(gelb)-, milch-, fleisch- und anderen proteinhaltigen Anschmutzungen, bevorzugt ei(gelb)- und/oder milchhaltigen Anschmutzungen, bestehenden Gruppe ausgewählt ist, eine verbesserte Reinigungsleistung gegenüber einer Referenzprotease (insbesondere Wildtyp-Protease gemäß SEQ ID NO:1) zeigt, wobei die Reinigungsleistung, wie in Beispiel 2 beschrieben, bestimmt wird.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease eine Aminosäuresubstitution bzw. Aminosäuresubstitutionskombination aufweist, die aus der aus N97E, R99E, R99I, R99N, R99V, R99T, D127E, M211C, N97E-R99E, N97E-R99I, N97E-R99N, N97E-R99V, N97E-R99T, N97E-D127E, N97E-M211C, R99E-D127E, R99E-M211C, R99I-D127E, R99I-M211C, R99N-D127E, R99N-M211C, R99V-D127E, R99V-M211C, R99T-D127E, R99T-M211C, D127E-M211C, N97E-R99E-D127E, N97E-R99I-D127E, N97E-R99N-D127E, N97E-R99V-D127E, N97ER99T-D127E, N97E-M211C-D127E, N97E-R99E-M211C, N97E-R99I-M211C, N97E-R99N-M211C, N97E-R99V-M211C, N97E-R99T-M211C, N97E-D127E-M211C, R99E-D127E-M211C, R99I-D127E-M211C, R99N-D127E-M211C, R99V-D127E-M211C, R99T-D127E-M211C, N97E-R99E-D127E-M211C, N97E-R99I-D127E-M211C, N97E-R99N-D127E-M211C, N97E-R99V-D127E-M211C und N97E-R99T-D127E-M211C, bevorzugt N97E, R99E, R99I, R99N, R99V, R99T, D127E, M211C, D127E-M211C und N97E-R99I bestehenden Kombination ausgewählt ist, wobei die Protease an mindestens einer proteasesensitiven Anschmutzung, die vorzugsweise aus der aus blut-, ei(gelb)-, milch-, fleisch- und anderen proteinhaltigen Anschmutzungen, bevorzugt ei(gelb)- und/oder milchhaltigen Anschmutzungen, bestehenden Gruppe ausgewählt ist, eine verbesserte Reinigungsleistung gegenüber einer Referenzprotease (insbesondere Wildtyp-Protease gemäß SEQ ID NO:1) zeigt, wobei die Reinigungsleistung, wie in Beispiel 2 beschrieben, bestimmt wird.

In bevorzugten Ausführungsformen ist die erfindungsgemäße Protease eine Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease eine Aminosäuresubstitution bzw. Aminosäuresubstitutionskombination aufweist, die aus der aus N97E, R99E, R99I, R99N, R99V, R99T, D127E, M211C, D127E-M211C und N97E-R99I bestehenden Kombination ausgewählt ist, wobei die Protease an mindestens einer proteasesensitiven Anschmutzung, die vorzugsweise aus der aus blut-, ei(gelb)-, milch-, fleisch- und anderen proteinhaltigen Anschmutzungen, bevorzugt ei(gelb)- und/oder milchhaltigen Anschmutzungen, bestehenden Gruppe ausgewählt ist, eine verbesserte Reinigungsleistung gegenüber einer Referenzprotease (insbesondere Wildtyp-Protease gemäß SEQ ID NO:1) zeigt, wobei die Reinigungsleistung, wie in Beispiel 2 beschrieben, bestimmt wird.

In besonders bevorzugten Ausführungsformen umfasst die erfindungsgemäße Protease eine Aminosäuresubstitutionskombination, die aus der aus N97E, R99E, R99I, R99N, R99V, R99T, D127E, M211C, N97E-R99E, N97E-R99I, N97E-R99N, N97E-R99V, N97E-R99T, N97E-D127E, N97E-M211C, R99E-D127E, R99E-M211C, R99I-D127E, R99I-M211C, R99N-D127E, R99N-M211C, R99V-D127E, R99V-M211C, R99T-D127E, R99T-M211C, D127E-M211C, N97E-R99E-D127E, N97E-R99I-D127E, N97E-R99N-D127E, N97E-R99V-D127E, N97E-R99T-D127E, N97E-M211C-D127E, N97ER99E-M211C, N97E-R99I-M211C, N97E-R99N-M211C, N97E-R99V-M211C, N97E-R99T-M211C, N97E-D127E-M211C, R99E-D127E-M211C, R99I-D127E-M211C, R99N-D127E-M211C, R99V-D127E-M211C, R99T-D127E-M211C, N97E-R99E-D127E-M211C, N97E-R991-D127E-M211C, N97ER99N-D127E-M211C, N97E-R99V-D127E-M211C und N97E-R99T-D127E-M211C, bevorzugt N97E, R99E, R99I, R99N, R99V, R99T, D127E, M211C, D127E-M211C und N97E-R99I, bestehenden Gruppe ausgewählt ist, wobei die Nummerierung jeweils bezogen auf die Nummerierung gemäß SEQ ID NO:1 ist und wobei die Protease neben den genannten Aminosäuresubstitutionen keine weiteren Änderungen umfasst, wobei die Protease an mindestens einer proteasesensitiven Anschmutzung, die vorzugsweise aus der aus blut-, ei(gelb)-, milch-, fleisch- und anderen proteinhaltigen Anschmutzungen, bevorzugt ei(gelb)- und/oder milchhaltigen Anschmutzungen, bestehenden Gruppe ausgewählt ist, eine verbesserte Reinigungsleistung gegenüber einer Referenzprotease (insbesondere Wildtyp-Protease gemäß SEQ ID NO:1) zeigt, wobei die Reinigungsleistung, wie in Beispiel 2 beschrieben, bestimmt wird.

In besonders bevorzugten Ausführungsformen umfasst die erfindungsgemäße Protease eine Aminosäuresubstitutionskombination, die aus der aus N97E, R99E, R99I, R99N, R99V, R99T, D127E, M211C, D127E-M211C und N97E-R99I bestehenden Gruppe ausgewählt ist, wobei die Nummerierung jeweils bezogen auf die Nummerierung gemäß SEQ ID NO:1 ist und wobei die Protease neben den genannten Aminosäuresubstitutionen keine weiteren Änderungen umfasst, wobei die Protease an mindestens einer proteasesensitiven Anschmutzung, die vorzugsweise aus der aus blut-, ei(gelb)-, milch-, fleisch- und anderen proteinhaltigen Anschmutzungen, bevorzugt ei(gelb)- und/oder milchhaltigen Anschmutzungen, bestehenden Gruppe ausgewählt ist, eine verbesserte Reinigungsleistung gegenüber einer Referenzprotease (insbesondere Wildtyp-Protease gemäß SEQ ID NO:1) zeigt, wobei die Reinigungsleistung, wie in Beispiel 2 beschrieben, bestimmt wird.

Erfindungsgemäße Proteasen verfügen über eine erhöhte katalytische Aktivität in Wasch- und/oder Reinigungsmitteln, insbesondere flüssigen Textilwaschmitteln. In vielfältigen Ausführungsformen können die erfindungsgemäßen Proteasen eine proteolytische Aktivität besitzen, die bezogen auf den Wildtyp (SEQ ID NO:1) mindestens 101%, 102%, 103%, 104%, 105%, 106%, 107%, 108%, 109%, 110%, 111%, 112%, 113%, 114%, 115%, 116%, 117%, 118%, 119%, 120%, 121%, 122%, 123%, 124%, 125%, 126%, 127%, 128%, 129%, 130% oder mehr beträgt. Solche leistungsverbesserten Proteasen ermöglichen verbesserte Reinigungsergebnisse an proteasesensitiven Anschmutzungen. Solche leistungsverbesserten Reinigungsergebnisse an proteasesensitiven Anschmutzungen können in verschiedenen Temperaturbereichen erzielt werden, z.B. in einem Bereich von etwa 0°C bis etwa 100°C, bevorzugt etwa 20°C bis etwa 60°C, weiter bevorzugt etwa 20°C bis etwa 40°C, besonders bevorzugt etwa 20°C bis etwa 30°C, ganz besonders bevorzugt etwa 20°C.

Die erfindungsgemäßen Proteasen verfügen über eine hohe Stabilität in Wasch- oder Reinigungsmitteln, insbesondere flüssigen Textilwaschmitteln, z.B. gegenüber Tensiden und/oder Bleichmitteln und/oder Chelatoren, und/oder gegenüber Temperatureinflüssen, insbesondere gegenüber hohen Temperaturen, z.B. zwischen etwa 40°C und etwa 60°C, und/oder gegenüber sauren oder alkalischen Bedingungen und/oder gegenüber pH-Wert-Änderungen und/oder gegenüber denaturierenden oder oxidierenden Agentien und/oder gegenüber (auto)proteolytischem Abbau und/oder gegenüber einer Veränderung der Redox-Verhältnisse. Mit besonders bevorzugten Ausführungsformen der Erfindung werden folglich leistungsverbesserte und/oder stabilere Protease-Varianten bereitgestellt. Mit weiteren ganz besonders bevorzugten Ausführungsformen der Erfindung werden leistungsverbesserte und stabilere Protease-Varianten bereitgestellt. Solche vorteilhaften Ausführungsformen erfindungsgemäßer Proteasen ermöglichen folglich verbesserte Reinigungsergebnisse an proteasesensitiven Anschmutzungen, die vorzugsweise aus der aus blut-, ei(gelb)-, milch-, fleisch- und anderen proteinhaltigen Anschmutzungen, bevorzugt ei(gelb)- und/oder milchhaltigen Anschmutzungen, bestehenden Gruppe ausgewählt sind.

Die erfindungsgemäßen Proteasen weisen enzymatische Aktivität auf, d.h. sie sind zur Hydrolyse von Peptiden und Proteinen befähigt, insbesondere in einem Wasch- oder Reinigungsmittel, bevorzugt flüssigen Textilwaschmittel. Eine erfindungsgemäße Protease ist daher ein Enzym, welches die Hydrolyse von Amid/Peptidbindungen in Protein/Peptid-Substraten katalysiert und dadurch in der Lage ist, Proteine oder Peptide zu spalten. Vorteilhafterweise weist das erfindungsgemäße Wasch- und Reinigungsmittel, insbesondere flüssige Textilwaschmittel, eine verbesserte Reinigungsleistung, insbesondere bei der Entfernung von proteasesensitiven Anschmutzungen, die vorzugsweise aus der aus blut-, ei(gelb)-, milch-, fleisch- und anderen proteinhaltigen Anschmutzungen, bevorzugt ei(gelb)- und/oder milchhaltigen Anschmutzungen, bestehenden Gruppe ausgewählt sind, auf. Besonders bevorzugt eignet sich das erfindungsgemäße Wasch- und Reinigungsmittel, insbesondere flüssige Textilwaschmittel, zur Entfernung von proteasesensitiven Anschmutzungen, die aus der aus blut-, ei(gelb)-, milch-, fleisch- und anderen proteinhaltigen Anschmutzungen bestehenden Gruppe ausgewählt sind. Ganz besonders bevorzugt eignet sich das erfindungsgemäße Wasch- und Reinigungsmittel, insbesondere flüssige Textilwaschmittel, zur Entfernung von ei(gelb)- und/oder milchhaltigen Anschmutzungen.

Ferner handelt es sich bei einer erfindungsgemäßen Protease vorzugsweise um eine reife (mature) Protease, d.h. um das katalytisch aktive Molekül ohne Signal- und/oder Propeptid(e). Soweit nicht anders angegeben beziehen sich auch die angegebenen Sequenzen auf jeweils reife (prozessierte) Enzyme.

In verschiedenen Ausführungsformen der Erfindung ist die Protease ein frei vorliegendes Enzym. Dies bedeutet, dass die Protease mit allen Komponenten eines Mittels direkt agieren kann und, falls es sich bei dem Mittel um ein Flüssigmittel handelt, dass die Protease direkt mit dem Lösungsmittel des Mittels (z.B. Wasser) in Kontakt steht. In anderen Ausführungsformen kann ein Mittel Proteasen enthalten, die einen Interaktionskomplex mit anderen Molekülen bilden oder die eine "Umhüllung" enthalten. Hierbei kann ein einzelnes oder mehrere Protease-Molekül(e) durch eine sie umgebende Struktur von den anderen Bestandteilen des Mittels getrennt sein. Eine solche trennende Struktur kann entstehen durch, ist allerdings nicht beschränkt auf, Vesikel, wie etwa eine Micelle oder ein Liposom. Die umgebende Struktur kann aber auch ein Viruspartikel, eine bakterielle Zelle oder eine eukaryotische Zelle sein. In verschiedenen Ausführungsformen kann ein Mittel Zellen von z.B. *Bacillus pumilus* oder *Bacillus subtilis* oder anderen Expressionsstämmen, die die erfindungsgemäßen Proteasen exprimieren, oder Zellkulturüberstände solcher Zellen enthalten.

Die Bestimmung der Identität von Nukleinsäure- oder Aminosäuresequenzen erfolgt durch einen Sequenzvergleich. Dieser Sequenzvergleich basiert auf dem im Stand der Technik etablierten und üblicherweise genutzten BLAST-Algorithmus (vgl. z.B. Altschul et al. (1990) Basic local alignment search tool, J. Mol. Biol., 215:403-410, und Altschul et al. (1997) Gapped BLAST and PSI-BLAST: a new generation of protein database search programs, Nucleic Acids Res., 25:3389-3402) und geschieht prinzipiell dadurch, dass ähnliche Abfolgen von Nukleotiden oder Aminosäuren in den Nukleinsäure- oder Aminosäuresequenzen einander zugeordnet werden. Eine tabellarische Zuordnung der betreffenden Positionen wird als Alignment bezeichnet. Ein weiterer im Stand der Technik verfügbarer Algorithmus ist der FASTA-Algorithmus. Sequenzvergleiche (Alignments), insbesondere multiple Sequenzvergleiche, werden mit Computerprogrammen erstellt. Häufig genutzt werden z.B. die Clustal-Serie (vgl. z.B. Chenna et al. (2003) Multiple sequence alignment with the Clustal series of programs, Nucleic Acid Res., 31:3497-3500), T-Coffee (vgl. z.B. Notredame et al. (2000) T-Coffee: A novel method for multiple sequence alignments, J. Mol. Biol., 302:205-217) oder Programme, die auf diesen Programmen bzw. Algorithmen basieren. Ferner möglich sind Sequenzvergleiche (Alignments) mit dem Computer-Programm Vector NTI^{®} Suite 10.3 (Invitrogen Corporation, 1600 Faraday Avenue, Carlsbad, Kalifornien, USA) mit den vorgegebenen Standardparametern, dessen AlignX-Modul für die Sequenzvergleiche auf ClustalW basiert, oder Clone Manager 10 (Verwendung der Scoring Matrix BLOSUM 62 für Sequenz-Alignment auf Aminosäureebene). Soweit nicht anders angegeben, wird die hierin angegebene Sequenzidentität mit dem BLAST-Algorithmus bestimmt.

Solch ein Vergleich erlaubt auch eine Aussage über die Ähnlichkeit der verglichenen Sequenzen zueinander. Sie wird üblicherweise in Prozent Identität, d.h. dem Anteil der identischen Nukleotide oder Aminosäurereste an denselben oder in einem Alignment einander entsprechenden Positionen angegeben. Der weiter gefasste Begriff der Homologie bezieht bei Aminosäuresequenzen konservierte Aminosäure-Austausche in die Betrachtung mit ein, also Aminosäuren mit ähnlicher chemischer Aktivität, da diese innerhalb des Proteins meist ähnliche chemische Aktivitäten ausüben. Daher kann die Ähnlichkeit der verglichenen Sequenzen auch Prozent Homologie oder Prozent Ähnlichkeit angegeben sein. Identitäts- und/oder Homologieangaben können über ganze Polypeptide oder Gene oder nur über einzelne Bereiche getroffen werden. Homologe oder identische Bereiche von verschiedenen Nukleinsäure- oder Aminosäuresequenzen sind daher durch Übereinstimmungen in den Sequenzen definiert. Solche Bereiche weisen oftmals identische Funktionen auf. Sie können klein sein und nur wenige Nukleotide oder Aminosäuren umfassen. Oftmals üben solche kleinen Bereiche für die Gesamtaktivität des Proteins essenzielle Funktionen aus. Es kann daher sinnvoll sein, Sequenzübereinstimmungen nur auf einzelne, ggf. kleine Bereiche zu beziehen. Soweit nicht anders angegeben beziehen sich Identitäts- oder Homologieangaben in der vorliegenden Anmeldung aber auf die Gesamtlänge der jeweils angegebenen Nukleinsäure- oder Aminosäuresäuresequenz.

Im Zusammenhang mit der vorliegenden Erfindung bedeutet die Angabe, dass eine Aminosäureposition einer numerisch bezeichneten Position in SEQ ID NO:1 entspricht daher, dass die entsprechende Position der numerisch bezeichneten Position in SEQ ID NO:1 in einem wie oben definierten Alignment zugeordnet ist. Weiterhin richtet sich die Zuordnung der Positionen nach dem reifen (maturen) Protein. Diese Zuordnung ist insbesondere auch anzuwenden, wenn die Aminosäuresequenz einer erfindungsgemäßen Protease eine höhere oder niedrigere Zahl von Aminosäureresten umfasst als die Protease gemäß SEQ ID NO:1. Ausgehend von den genannten Positionen in der Aminosäuresequenz der Protease gemäß SEQ ID NO:1 sind die Veränderungspositionen in einer erfindungsgemäßen Protease diejenigen, die eben diesen Positionen in einem Alignment zugeordnet sind.

Zusätzlich zu den vorstehend erläuterten Aminosäureveränderungen können erfindungsgemäße Proteasen weitere Aminosäureveränderungen, insbesondere Aminosäure-Substitutionen, -Insertionen oder -Deletionen, aufweisen. Solche Proteasen sind z.B. durch gezielte genetische Veränderung, d.h. durch Mutageneseverfahren, weiterentwickelt und für bestimmte Einsatzzwecke oder hinsichtlich spezieller Eigenschaften (z.B. hinsichtlich ihrer katalytischen Aktivität, Stabilität, usw.) optimiert. Ferner können erfindungsgemäße Nukleinsäuren in Rekombinationsansätze eingebracht und damit zur Erzeugung völlig neuartiger Proteasen oder anderer Polypeptide genutzt werden. Das Ziel ist es, in die bekannten Moleküle gezielte Mutationen wie Substitutionen, Insertionen oder Deletionen einzuführen, um z.B. die Reinigungsleistung von erfindungsgemäßen Enzymen zu verbessern. Hierzu können insbesondere die Oberflächenladungen und/oder der isoelektrische Punkt der Moleküle und dadurch ihre Wechselwirkungen mit dem Substrat verändert werden. So kann z.B. die Nettoladung der Enzyme verändert werden, um darüber die Substratbindung insbesondere für den Einsatz in Wasch- und Reinigungsmitteln zu beeinflussen. Alternativ oder ergänzend kann durch eine oder mehrere entsprechende Mutationen die Stabilität oder katalytische Aktivität der Protease erhöht und dadurch ihre Reinigungsleistung verbessert werden. Vorteilhafte Eigenschaften einzelner Mutationen, z.B. einzelner Substitutionen, können sich ergänzen. Eine hinsichtlich bestimmter Eigenschaften bereits optimierte Protease, z.B. hinsichtlich ihrer Stabilität bei Lagerung und/oder Aktivität und/oder ihrer Toleranz in Bezug auf das Substratspektrum, kann daher im Rahmen der Erfindung zusätzlich weiterentwickelt sein.

Für die Beschreibung von Substitutionen, die genau eine Aminosäureposition betreffen (Aminosäureaustausche), wird hierin folgende Konvention angewendet: zunächst wird die natürlicherweise vorhandene Aminosäure in Form des international gebräuchlichen Einbuchstaben-Codes bezeichnet, dann folgt die zugehörige Sequenzposition und schließlich die eingefügte Aminosäure. Mehrere oder alternative Austausche innerhalb derselben Polypeptidkette werden durch Schrägstriche voneinander getrennt. "130D/V" bedeutet somit, dass die Position 130 zu D oder V mutiert ist. Bei Insertionen sind nach der Sequenzposition zusätzliche Aminosäuren benannt. Bei Deletionen ist die fehlende Aminosäure durch ein Symbol, z.B. einen Stern oder einen Strich, ersetzt oder vor der entsprechenden Position ein Δ angegeben. Beispielsweise beschreibt P9T die Substitution von Prolin an Position 9 durch Threonin, P9TH die Insertion von Histidin nach der Aminosäure Threonin an Position 9 und P9* oder ΔP9 die Deletion von Prolin an Position 9. Diese Nomenklatur ist dem Fachmann auf dem Gebiet der Enzymtechnologie bekannt.

Ein weiterer Gegenstand der Erfindung ist daher eine Protease, die dadurch gekennzeichnet ist, dass sie aus einer Protease wie hierin beschrieben als Ausgangsmolekül erhältlich ist durch ein- oder mehrfache konservative Aminosäuresubstitution, wobei die Protease in der Zählung gemäß SEQ ID NO:1 mindestens eine der vorstehend beschriebenen Aminosäuresubstitutionen aufweist. Der Begriff "konservative Aminosäuresubstitution" bedeutet den Austausch (Substitution) eines Aminosäurerestes gegen einen anderen Aminosäurerest, wobei dieser Austausch nicht zu einer Änderung der Polarität oder Ladung an der Position der ausgetauschten Aminosäure führt, z.B. der Austausch eines unpolaren Aminosäurerestes gegen einen anderen unpolaren Aminosäurerest. Konservative Aminosäuresubstitutionen im Rahmen der Erfindung umfassen z.B.: G=A=S, I=V=L=M, D=E, N=Q, K=R, Y=F, S=T, G=A=I=V=L=M=Y=F=W=P=S=T.

Alternativ oder ergänzend ist die Protease dadurch gekennzeichnet, dass sie aus einer erfindungsgemäßen Protease als Ausgangsmolekül erhältlich ist durch Fragmentierung, Deletions-, Insertions- oder Substitutionsmutagenese und eine Aminosäuresequenz umfasst, die über eine Länge von mindestens 190, 200, 210, 220, 230, 240, 250, 255, 256, 257, 258, 259, 260, 261, 262, 263, 264, 265, 266, 267, 268 oder 269 zusammenhängenden Aminosäuren mit dem Ausgangsmolekül übereinstimmt, wobei die Protease, jeweils bezogen auf die Nummerierung gemäß SEQ ID NO:1 an mindestens einer der Positionen, die den Positionen 97, 99, 127 und 211 entsprechen, mindestens eine Aminosäuresubstitution, die aus der aus N97E, R99E, R99I, R99N, R99V, R99T, D127E und M211C bestehenden Gruppe ausgewählt ist, aufweist.

So ist es z.B. möglich, an den Termini oder in den Loops des Enzyms einzelne Aminosäuren zu deletieren, ohne dass dadurch die proteolytische Aktivität verloren oder vermindert wird. Ferner kann durch derartige Fragmentierung, Deletions-, Insertions- oder Substitutionsmutagenese z.B. auch die Allergenizität betreffender Enzyme gesenkt und somit insgesamt ihre Einsetzbarkeit verbessert werden. Vorteilhafterweise behalten die Enzyme auch nach der Mutagenese ihre proteolytische Aktivität, d.h. ihre proteolytische Aktivität entspricht mindestens derjenigen des Ausgangsenzyms, d.h. in einer bevorzugten Ausführungsform beträgt die proteolytische Aktivität mindestens 100%, vorzugsweise mindestens 105%, weiter bevorzugt mindestens 110%, noch weiter bevorzugt mindestens 120% oder mehr, der Aktivität des Ausgangsenzyms. Auch weitere Substitutionen können vorteilhafte Wirkungen zeigen. Sowohl einzelne wie auch mehrere zusammenhängende Aminosäuren können gegen andere Aminosäuren ausgetauscht werden.

Vorteilhafte Positionen für Sequenzveränderungen, insbesondere Substitutionen, der Protease gemäß SEQ ID NO:1, die übertragen auf homologe Positionen der erfindungsgemäßen Proteasen bevorzugt von Bedeutung sind und der Protease vorteilhafte funktionelle Eigenschaften verleihen, sind demnach die Positionen, die in einem Alignment den hierin beschriebenen Positionen entsprechen, d.h. in der Zählung gemäß SEQ ID NO:1. An den genannten Positionen liegen in dem Wildtypmolekül der Protease folgende Aminosäurereste: 97N, 99R, 127D, 211M.

Eine weitere Bestätigung der korrekten Zuordnung der zu verändernden Aminosäuren, d.h. insbesondere deren funktionelle Entsprechung, können Vergleichsversuche liefern, wonach die beiden auf der Basis eines Alignments einander zugeordneten Positionen in beiden miteinander verglichenen Proteasen auf die gleiche Weise verändert werden und beobachtet wird, ob bei beiden die enzymatische Aktivität auf gleiche Weise verändert wird. Geht z.B. ein Aminosäureaustausch in einer bestimmten Position der Protease gemäß SEQ ID NO:1 mit einer Veränderung eines enzymatischen Parameters einher, z.B. mit der Erhöhung des K_{M}-Wertes, und wird eine entsprechende Veränderung des enzymatischen Parameters, z.B. also ebenfalls eine Erhöhung des K_{M}-Wertes, in einer erfindungsgemäßen Protease-Variante beobachtet, deren Aminosäureaustausch durch dieselbe eingeführte Aminosäure erreicht wurde, so ist hierin eine Bestätigung der korrekten Zuordnung zu sehen.

Alle genannten Sachverhalte sind auch auf die erfindungsgemäßen Verfahren zur Herstellung einer Protease anwendbar.

Ein erfindungsgemäßes Verfahren zur Herstellung einer Protease, umfasst das Einbringen mindestens einer Aminosäuresubstitution an mindestens einer der Positionen, die bezogen auf die Nummerierung gemäß SEQ ID NO:1 den Positionen 97, 99, 127 und 211 entsprechen, ausgewählt aus der aus N97E, R99E, R99I, R99N, R99V, R99T, D127E und M211C bestehenden Gruppe, in ein Ausgangsmolekül, das eine Aminosäuresequenz aufweist, die mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99%, 99,5% oder 100% Sequenzidentität mit der in SEQ ID NO:1 angegebenen Aminosäuresequenz über deren Gesamtlänge aufweist.

Ein erfindungsgemäßes Verfahren kann ferner einen oder mehrere der folgenden Verfahrensschritte umfassen:
(a) Einbringen einer ein- oder mehrfachen konservativen Aminosäuresubstitution, wobei die Protease, jeweils bezogen auf die Nummerierung gemäß SEQ ID NO:1 an mindestens einer der Positionen, die den Positionen 97, 99, 127 und 211 entsprechen, mindestens eine Aminosäuresubstitution, die aus der aus N97E, R99E, R99I, R99N, R99V, R99T, D127E und M211C bestehenden Gruppe ausgewählt ist, aufweist;
(b) Veränderung der Aminosäuresequenz durch Fragmentierung, Deletions-, Insertions- oder Substitutionsmutagenese derart, dass die Protease eine Aminosäuresequenz umfasst, die über eine Länge von mindestens 190, 200, 210, 220, 230, 240, 250, 255, 256, 257, 258, 259, 260, 261, 262, 263, 264, 265, 266, 267, 268 oder 269 zusammenhängenden Aminosäuren mit dem Ausgangsmolekül übereinstimmt, wobei die Protease, jeweils bezogen auf die Nummerierung gemäß SEQ ID NO:1 an mindestens einer der Positionen, die den Positionen 97, 99, 127 und 211 entsprechen, mindestens eine Aminosäuresubstitution, die aus der aus N97E, R99E, R99I, R99N, R99V, R99T, D127E und M211C bestehenden Gruppe ausgewählt ist, aufweist.

Sämtliche Ausführungsformen gelten auch für die erfindungsgemäßen Verfahren.

In einer weiteren Ausführungsform der Erfindung ist eine zuvor beschriebene Protease stabilisiert, insbesondere durch eine oder mehrere Mutationen, z.B. Substitutionen, oder durch Kopplung an ein Polymer. Eine Erhöhung der Stabilität bei der Lagerung und/oder während des Einsatzes, z.B. beim Waschprozess, führt dazu, dass die enzymatische Aktivität länger anhält und damit die Reinigungsleistung verbessert wird. Grundsätzlich kommen alle im Stand der Technik beschriebenen und/oder zweckmäßigen Stabilisierungsmöglichkeiten in Betracht. Bevorzugt sind solche Stabilisierungen, die über Mutationen des Enzyms selbst erreicht werden, da solche Stabilisierungen im Anschluss an die Gewinnung des Enzyms keine weiteren Arbeitsschritte erfordern. Beispiele für hierfür geeignete Sequenzveränderungen sind vorstehend genannt. Weitere geeignete Sequenzveränderungen sind aus dem Stand der Technik bekannt.

Weitere Möglichkeiten der Stabilisierung sind z.B.:
- Veränderung der Bindung von Metallionen, insbesondere der Calcium-Bindungsstellen, z.B. durch Austauschen von einer oder mehreren der an der Calcium-Bindung beteiligten Aminosäure(n) gegen eine oder mehrere negativ geladene Aminosäuren und/oder durch Einführen von Sequenzveränderungen in mindestens einer der Folgen der beiden Aminosäuren Arginin/Glycin;
- Schutz gegen den Einfluss von denaturierenden Agentien wie Tensiden durch Mutationen, die eine Veränderung der Aminosäuresequenz auf oder an der Oberfläche des Proteins bewirken;
- Austausch von Aminosäuren, die nahe dem N-Terminus liegen, gegen solche, die vermutlich über nicht-kovalente Wechselwirkungen mit dem Rest des Moleküls in Kontakt treten und somit einen Beitrag zur Aufrechterhaltung der globulären Struktur leisten.

Bevorzugte Ausführungsformen sind solche, bei denen das Enzym auf mehrere Arten stabilisiert wird, da mehrere stabilisierende Mutationen additiv oder synergistisch wirken.

Alternativ oder ergänzend kann die erfindungsgemäße Protease mit mindestens einer reversiblen Inhibitor-/Stabilisatorverbindung, die aus der aus Peptidinhibitoren, insbesondere Peptidaldehyde, Polyolen, insbesondere Glycerin und 1,2-Propylenglykol, Benzamidinhydrochlorid, Borax, Borsäuren, Boronsäuren oder deren Salze oder Ester oder Derivate, insbesondere Phenylboronsäurederivate oder 4-Formylphenylboronsäure (4-FPBA), Antioxidantien, und Kombinationen davon bestehenden Gruppe ausgewählt ist, kombiniert werden, um die Stabilität der Protease in Wasch- und Reinigungsmitteln weiter zu erhöhen. Besonders bevorzugte reversible Proteaseinibitoren umfassen Borsäure, 4-FPBA und Peptidinhibitoren.

Im Rahmen der vorliegenden Erfindung wird unter "Phenylboronsäurederivat" eine Verbindung mit der folgenden Formel verstanden: 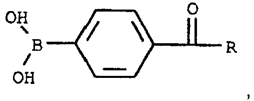 wobei R Wasserstoff, eine Hydroxyl-, eine C₁₋₆ Alkyl-, eine substituierte C₁₋₆ Alkyl-, eine C₁₋₆ Alkenyl oder eine substituierte C₁₋₆ Alkenyl-Gruppe ist. Bevorzugt ist der Rest R in dem Phenylboronsäurederivat eine C₁₋₆ Alkyl-Gruppe und hierunter weiter bevorzugt -CH₃, -CH₃CH₂ oder-CH₃CH₂CH₂ sein. Weiter bevorzugt ist der Rest R in dem Phenylboronsäurederivat Wasserstoff. Besonders bevorzugt ist das Phenylboronsäurederivat 4-Formylphenylboronsäure (4-FPBA).

Die eingesetzte Inhibitor/Stabilisatorverbindung kann Borsäure sein.

In bevorzugten Ausführungsformen ist das erfindungsgemäße Mittel im Wesentlichen frei von borhaltigen Verbindungen. "Im Wesentlichen frei von borhaltigen Verbindungen" bedeutet in diesem Zusammenhang, dass die erfindungsgemäßen Mittel weniger als 2 Gew.-%, vorzugsweise weniger als 1 Gew.-%, weiter bevorzugt weniger als 0,5 Gew.-% und besonders bevorzugt weniger als 0,1 Gew.-%, borhaltige Verbindungen, bezogen auf das Gesamtgewicht des Mittels, enthalten. In besonders bevorzugten Ausführungsformen sind die erfindungsgemäßen Mittel frei von borhaltigen Verbindungen, d.h. sie enthalten insbesondere keine Borsäure und/oder Phenylboronsäurederivate.

In verschiedenen Ausführungsformen können das Enzym und die Inhibitor/Stabilisatorverbindung in einer Enzymzusammensetzung vorformuliert vorliegen. Wie aus den vorherigen Ausführungen ersichtlich, bildet das Enzym-Protein nur einen Bruchteil des Gesamtgewichts üblicher Enzymzubereitungen. Bevorzugt eingesetzte Enzymzubereitungen enthalten zwischen 0,1 und 40 Gew.-%, bevorzugt zwischen 0,2 und 30 Gew.-%, besonders bevorzugt zwischen 0,4 und 20 Gew.-% und insbesondere zwischen 0,8 und 10 Gew.-% des Enzymproteins. In solchen Zusammensetzungen kann die Inhibitor/Stabilisatorverbindung in einer Menge von 0,05 bis 35 Gew.-%, vorzugsweise von 0,05 bis 10 Gew.-%, bezogen auf das Gesamtgewicht in der Enzymzusammensetzung, enthalten sein. Diese Enzymzusammensetzung kann in erfindungsgemäßen Mitteln eingesetzt werden, und zwar in Mengen, die zu den oben angegeben Endkonzentrationen im Mittel führen.

Ein weiterer Gegenstand der Erfindung ist eine Protease wie hierin beschrieben, die dadurch gekennzeichnet ist, dass sie mindestens eine chemische Modifikation aufweist. Eine Protease mit einer solchen Veränderung wird als Derivat bezeichnet, d.h. die Protease ist derivatisiert. Unter Derivaten werden im Sinne der vorliegenden Anmeldung demnach solche Proteine verstanden, deren reine Aminosäurekette chemisch modifiziert worden ist. Solche Derivatisierungen können z.B. *in vivo* durch die Wirtszelle erfolgen, die das Protein exprimiert. Diesbezüglich sind Kopplungen niedrigmolekularer Verbindungen wie von Lipiden oder Oligosacchariden besonders hervorzuheben. Derivatisierungen können aber auch *in vitro* durchgeführt werden, etwa durch die chemische Umwandlung einer Seitenkette einer Aminosäure oder durch kovalente Bindung einer anderen Verbindung an das Protein. Beispielsweise ist die Kopplung von Aminen an Carboxylgruppen eines Enzyms zur Veränderung des isoelektrischen Punkts möglich. Eine solche andere Verbindung kann auch ein weiteres Protein sein, das z.B. über bifunktionelle chemische Verbindungen an ein erfindungsgemäßes Protein gebunden wird. Ebenso ist unter Derivatisierung die kovalente Bindung an einen makromolekularen Träger zu verstehen, oder auch ein nichtkovalenter Einschluss in geeignete makromolekulare Käfigstrukturen. Derivatisierungen können z.B. die Substratspezifität oder die Bindungsstärke an das Substrat beeinflussen oder eine vorübergehende Blockierung der enzymatischen Aktivität herbeiführen, wenn es sich bei der angekoppelten Substanz um einen Inhibitor handelt. Dies kann z.B. für den Zeitraum der Lagerung sinnvoll sein. Derartige Modifikationen können ferner die Stabilität oder die enzymatische Aktivität beeinflussen. Sie können ferner auch dazu dienen, die Allergenizität und/oder Immunogenizität des Proteins herabzusetzen und damit z.B. dessen Hautverträglichkeit zu erhöhen. Beispielsweise können Kopplungen mit makromolekularen Verbindungen, z.B. Polyethylenglykol, das Protein hinsichtlich der Stabilität und/oder Hautverträglichkeit verbessern. Unter Derivaten eines erfindungsgemäßen Proteins können im weitesten Sinne auch Präparationen dieser Proteine verstanden werden. Je nach Gewinnung, Aufarbeitung oder Präparation kann ein Protein mit diversen anderen Stoffen kombiniert sein, z.B. aus der Kultur der produzierenden Mikroorganismen. Ein Protein kann auch, z.B. zur Erhöhung seiner Lagerstabilität, mit anderen Stoffen gezielt versetzt worden sein. Erfindungsgemäß sind deshalb auch alle Präparationen eines erfindungsgemäßen Proteins. Das ist auch unabhängig davon, ob es in einer bestimmten Präparation tatsächlich diese enzymatische Aktivität entfaltet oder nicht. Denn es kann gewünscht sein, dass es bei der Lagerung keine oder nur geringe Aktivität besitzt, und erst zum Zeitpunkt der Verwendung seine enzymatische Funktion entfaltet. Dies kann z.B. über entsprechende Begleitstoffe gesteuert werden. Insbesondere die gemeinsame Präparation von Proteasen mit spezifischen Inhibitoren ist diesbezüglich möglich. Unter allen hierin beschriebenen Proteasen bzw. Protease-Varianten und/oder -Derivaten sind im Rahmen der vorliegenden Erfindung diejenigen besonders bevorzugt, deren Lagerstabilität und/oder katalytische Aktivität und/oder Substrattoleranz und/oder Reinigungsleistung gegenüber dem Wildtyp bzw. einer Ausgangsvariante verbessert ist, wobei die katalytische Aktivität und/oder Reinigungsleistung wie hierin beschrieben bestimmt wird.

Ein weiterer Gegenstand der Erfindung ist eine Nukleinsäure, die für eine erfindungsgemäße Protease codiert, sowie ein Vektor enthaltend eine solche Nukleinsäure, insbesondere ein Klonierungsvektor oder ein Expressionsvektor. Hierbei kann es sich um DNA- oder RNA-Moleküle handeln. Sie können als Einzelstrang, als ein zu diesem Einzelstrang komplementärer Einzelstrang oder als Doppelstrang vorliegen. Insbesondere bei DNA-Molekülen sind die Sequenzen beider komplementärer Stränge in jeweils allen drei möglichen Leserastern zu berücksichtigen. Ferner ist zu berücksichtigen, dass verschiedene Codons, also Basentriplets, für die gleichen Aminosäuren codieren können, so dass eine bestimmte Aminosäuresequenz von mehreren unterschiedlichen Nukleinsäuren codiert werden kann. Auf Grund dieser Degeneriertheit des genetischen Codes sind sämtliche Nukleinsäuresequenzen in diesem Erfindungsgegenstand eingeschlossen, die eine der vorstehend beschriebenen Proteasen codieren können. Der Fachmann ist in der Lage, diese Nukleinsäuresequenzen zweifelsfrei zu bestimmen, da trotz der Degeneriertheit des genetischen Codes einzelnen Codons definierte Aminosäuren zuzuordnen sind. Daher kann der Fachmann ausgehend von einer Aminosäuresequenz für diese Aminosäuresequenz codierende Nukleinsäuren problemlos ermitteln. Weiterhin können bei erfindungsgemäßen Nukleinsäuren ein oder mehrere Codons durch synonyme Codons ersetzt sein. Dieser Aspekt bezieht sich insbesondere auf die heterologe Expression der erfindungsgemäßen Enzyme. So besitzt jeder Organismus, z.B. eine Wirtszelle eines Produktionsstammes, eine bestimmte Codon-Verwendung. Unter Codon-Verwendung wird die Übersetzung des genetischen Codes in Aminosäuren durch den jeweiligen Organismus verstanden. Es kann zu Engpässen in der Proteinbiosynthese kommen, wenn die auf der Nukleinsäure liegenden Codons in dem Organismus einer vergleichsweise geringen Zahl von beladenen tRNA-Molekülen gegenüberstehen. Obwohl für die gleiche Aminosäure codierend führt das dazu, dass in dem Organismus ein Codon weniger effizient translatiert wird als ein synonymes Codon, das für dieselbe Aminosäure codiert. Auf Grund des Vorliegens einer höheren Anzahl von tRNA-Molekülen für das synonyme Codon kann dieses in dem Organismus effizienter translatiert werden.

Einem Fachmann ist es über heutzutage allgemein bekannte Methoden, wie z.B. die chemische Synthese oder die Polymerase-Kettenreaktion (PCR) in Verbindung mit molekularbiologischen und/oder proteinchemischen Standardmethoden möglich, anhand bekannter DNA- und/oder Aminosäuresequenzen die entsprechenden Nukleinsäuren bis hin zu vollständigen Genen herzustellen. Derartige Methoden sind z.B. aus Sambrook, J., Fritsch, E.F. and Maniatis, T. 2001. Molecular cloning: a laboratory manual, 3. Edition Cold Spring Laboratory Press bekannt.

Unter Vektoren werden im Sinne der vorliegenden Erfindung aus Nukleinsäuren bestehende Elemente verstanden, die als kennzeichnenden Nukleinsäurebereich eine erfindungsgemäße Nukleinsäure enthalten. Sie vermögen diese in einer Spezies oder einer Zelllinie über mehrere Generationen oder Zellteilungen hinweg als stabiles genetisches Element zu etablieren. Vektoren sind insbesondere bei der Verwendung in Bakterien spezielle Plasmide, also zirkulare genetische Elemente. Im Rahmen der vorliegenden Erfindung wird eine erfindungsgemäße Nukleinsäure in einen Vektor kloniert. Zu den Vektoren zählen z.B. solche, deren Ursprung bakterielle Plasmide, Viren oder Bakteriophagen sind, oder überwiegend synthetische Vektoren oder Plasmide mit Elementen verschiedenster Herkunft. Mit den weiteren jeweils vorhandenen genetischen Elementen vermögen Vektoren sich in den betreffenden Wirtszellen über mehrere Generationen hinweg als stabile Einheiten zu etablieren. Sie können extrachromosomal als eigene Einheiten vorliegen oder in ein Chromosom oder chromosomale DNA integrieren. Expressionsvektoren umfassen Nukleinsäuresequenzen, die sie dazu befähigen, in den sie enthaltenden Wirtszellen, vorzugsweise Mikroorganismen, besonders bevorzugt Bakterien, zu replizieren und dort eine enthaltene Nukleinsäure zur Expression zu bringen. Die Expression wird insbesondere von dem oder den Promotoren beeinflusst, welche die Transkription regulieren. Prinzipiell kann die Expression durch den natürlichen, ursprünglich vor der zu exprimierenden Nukleinsäure lokalisierten Promotor erfolgen, aber auch durch einen auf dem Expressionsvektor bereitgestellten Promotor der Wirtszelle oder auch durch einen modifizierten oder einen völlig anderen Promotor eines anderen Organismus oder einer anderen Wirtszelle. Im vorliegenden Fall wird zumindest ein Promotor für die Expression einer erfindungsgemäßen Nukleinsäure zur Verfügung gestellt und für deren Expression genutzt. Expressionsvektoren können ferner regulierbar sein, z.B. durch Änderung der Kultivierungsbedingungen oder bei Erreichen einer bestimmten Zelldichte der sie enthaltenen Wirtszellen oder durch Zugabe von bestimmten Substanzen, insbesondere Aktivatoren der Genexpression. Ein Beispiel für eine solche Substanz ist das Galactose-Derivat Isopropyl-β-D-thiogalactopyranosid (IPTG), welches als Aktivator des bakteriellen Lactose-Operons (lac-Operons) verwendet wird. Im Gegensatz zu Expressionsvektoren wird die enthaltene Nukleinsäure in Klonierungsvektoren nicht exprimiert.

Ein weiterer Gegenstand der Erfindung ist eine nicht-menschliche Wirtszelle, die eine erfindungsgemäße Nukleinsäure oder einen erfindungsgemäßen Vektor beinhaltet, oder die eine erfindungsgemäße Protease beinhaltet, insbesondere eine, die die Protease in das die Wirtszelle umgebende Medium sezerniert. Bevorzugt wird eine erfindungsgemäße Nukleinsäure oder ein erfindungsgemäßer Vektor in einen Mikroorganismus transformiert, der dann eine erfindungsgemäße Wirtszelle darstellt. Alternativ können auch einzelne Komponenten, d.h. Nukleinsäure-Teile oder -Fragmente einer erfindungsgemäßen Nukleinsäure derart in eine Wirtszelle eingebracht werden, dass die dann resultierende Wirtszelle eine erfindungsgemäße Nukleinsäure oder einen erfindungsgemäßen Vektor enthält. Dieses Vorgehen eignet sich besonders dann, wenn die Wirtszelle bereits einen oder mehrere Bestandteile einer erfindungsgemäßen Nukleinsäure oder eines erfindungsgemäßen Vektors enthält und die weiteren Bestandteile dann entsprechend ergänzt werden. Verfahren zur Transformation von Zellen sind im Stand der Technik etabliert und dem Fachmann hinlänglich bekannt. Als Wirtszellen eignen sich prinzipiell alle Zellen, d.h. prokaryotische oder eukaryotische Zellen. Bevorzugt sind solche Wirtszellen, die sich genetisch vorteilhaft handhaben lassen, was z.B. die Transformation mit der Nukleinsäure oder dem Vektor und dessen stabile Etablierung angeht, z.B. einzellige Pilze oder Bakterien. Ferner zeichnen sich bevorzugte Wirtszellen durch eine gute mikrobiologische und biotechnologische Handhabbarkeit aus. Das betrifft z.B. leichte Kultivierbarkeit, hohe Wachstumsraten, geringe Anforderungen an Fermentationsmedien und gute Produktions- und Sekretionsraten für Fremdproteine. Bevorzugte erfindungsgemäße Wirtszellen sezernieren das (transgen) exprimierte Protein in das die Wirtszellen umgebende Medium. Ferner können die Proteasen von den sie produzierenden Zellen nach deren Herstellung modifiziert werden, z.B. durch Anknüpfung von Zuckermolekülen, Formylierungen, Aminierungen, usw. Solche posttranslationalen Modifikationen können die Protease funktionell beeinflussen.

Weitere bevorzugte Ausführungsformen stellen solche Wirtszellen dar, die aufgrund genetischer Regulationselemente, die z.B. auf dem Vektor zur Verfügung gestellt werden, aber auch von vornherein in diesen Zellen vorhanden sein können, in ihrer Aktivität regulierbar sind. Beispielsweise durch kontrollierte Zugabe von chemischen Verbindungen, die als Aktivatoren dienen, durch Änderung der Kultivierungsbedingungen oder bei Erreichen einer bestimmten Zelldichte können diese zur Expression angeregt werden. Dies ermöglicht eine wirtschaftliche Produktion der erfindungsgemäßen Proteine. Ein Beispiel für eine solche Verbindung ist IPTG wie vorstehend beschrieben.

Bevorzugte Wirtszellen sind prokaryotische oder bakterielle Zellen. Bakterien zeichnen sich durch kurze Generationszeiten und geringe Ansprüche an die Kultivierungsbedingungen aus. Dadurch können kostengünstige Kultivierungsverfahren oder Herstellungsverfahren etabliert werden. Zudem verfügt der Fachmann bei Bakterien in der Fermentationstechnik über einen reichhaltigen Erfahrungsschatz. Für eine spezielle Produktion können aus verschiedensten, im Einzelfall experimentell zu ermittelnden Gründen wie Nährstoffquellen, Produktbildungsrate, Zeitbedarf usw., gramnegative oder grampositive Bakterien geeignet sein. Bei gramnegativen Bakterien wie z.B. *Escherichia coli* wird eine Vielzahl von Proteinen in den periplasmatischen Raum sezerniert, also in das Kompartiment zwischen den beiden die Zellen einschließenden Membranen. Dies kann für spezielle Anwendungen vorteilhaft sein. Ferner können auch gramnegative Bakterien so ausgestaltet werden, dass sie die exprimierten Proteine nicht nur in den periplasmatischen Raum, sondern in das das Bakterium umgebende Medium ausschleusen. Grampositive Bakterien wie z.B. Bacilli oder Actinomyceten oder andere Vertreter der *Actinomycetales* besitzen demgegenüber keine äußere Membran, so dass sezernierte Proteine sogleich in das die Bakterien umgebende Medium, in der Regel das Nährmedium, abgegeben werden, aus welchem sich die exprimierten Proteine aufreinigen lassen. Sie können aus dem Medium direkt isoliert oder weiter prozessiert werden. Zudem sind grampositive Bakterien mit den meisten Herkunftsorganismen für technisch wichtige Enzyme verwandt oder identisch und bilden meist selbst vergleichbare Enzyme, so dass sie über eine ähnliche Codon-Verwendung verfügen und ihr Protein-Syntheseapparat naturgemäß entsprechend ausgerichtet ist. Erfindungsgemäße Wirtszellen können hinsichtlich ihrer Anforderungen an die Kulturbedingungen verändert sein, andere oder zusätzliche Selektionsmarker aufweisen oder noch andere oder zusätzliche Proteine exprimieren. Es kann sich insbesondere auch um solche Wirtszellen handeln, die mehrere Proteine oder Enzyme transgen exprimieren. Die vorliegende Erfindung ist prinzipiell auf alle Mikroorganismen, insbesondere auf alle fermentierbaren Mikroorganismen, besonders bevorzugt auf solche der Gattung *Bacillus,* anwendbar und führt dazu, dass sich durch den Einsatz solcher Mikroorganismen erfindungsgemäße Proteine herstellen lassen. Solche Mikroorganismen stellen dann Wirtszellen im Sinne der Erfindung dar. In einer weiteren Ausführungsform der Erfindung ist die Wirtszelle dadurch gekennzeichnet, dass sie ein Bakterium ist, bevorzugt eines, das ausgewählt ist aus der Gruppe der Gattungen von *Escherichia, Klebsiella, Bacillus, Staphylococcus, Corynebacterium, Arthrobacter, Streptomyces, Stenotrophomonas* und *Pseudomonas,* weiter bevorzugt eines, das ausgewählt ist aus der Gruppe von *Escherichia coli, Klebsiella planticola, Bacillus licheniformis, Bacillus lentus, Bacillus amyloliquefaciens, Bacillus subtilis, Bacillus alcalophilus, Bacillus globigii, Bacillus gibsonii, Bacillus clausii, Bacillus halodurans, Bacillus pumilus, Staphylococcus carnosus, Corynebacterium glutamicum, Arthrobacter oxidans, Streptomyces lividans, Streptomyces coelicolor* und *Stenotrophomonas maltophilia.*

Die Wirtszelle kann aber auch eine eukaryotische Zelle sein, die dadurch gekennzeichnet ist, dass sie einen Zellkern besitzt. Einen weiteren Gegenstand der Erfindung stellt daher eine Wirtszelle dar, die dadurch gekennzeichnet ist, dass sie einen Zellkern besitzt. Im Gegensatz zu prokaryotischen Zellen sind eukaryotische Zellen in der Lage, das gebildete Protein posttranslational zu modifizieren. Beispiele dafür sind Pilze wie Actinomyceten oder Hefen wie *Saccharomyces* oder *Kluyveromyces.* Dies kann z.B. dann besonders vorteilhaft sein, wenn die Proteine im Zusammenhang mit ihrer Synthese spezifische Modifikationen erfahren sollen, die derartige Systeme ermöglichen. Zu den Modifikationen, die eukaryotische Systeme besonders im Zusammenhang mit der Proteinsynthese durchführen, gehören z.B. die Bindung niedermolekularer Verbindungen wie Membrananker oder Oligosaccharide. Derartige Oligosaccharid-Modifikationen können z.B. zur Senkung der Allergenizität eines exprimierten Proteins wünschenswert sein. Auch eine Co-Expression mit den natürlicherweise von derartigen Zellen gebildeten Enzymen, wie z.B. Cellulasen, kann vorteilhaft sein. Ferner können sich z.B. thermophile pilzliche Expressionssysteme besonders zur Expression temperaturbeständiger Proteine oder Varianten eignen.

Die erfindungsgemäßen Wirtszellen werden in üblicher Weise kultiviert und fermentiert, z.B. in diskontinuierlichen oder kontinuierlichen Systemen. Im ersten Fall wird ein geeignetes Nährmedium mit den Wirtszellen beimpft und das Produkt nach einem experimentell zu ermittelnden Zeitraum aus dem Medium geerntet. Kontinuierliche Fermentationen zeichnen sich durch Erreichen eines Fließgleichgewichts aus, in dem über einen vergleichsweise langen Zeitraum Zellen teilweise absterben aber auch nachwachsen und gleichzeitig aus dem Medium das gebildete Protein entnommen werden kann.

Erfindungsgemäße Wirtszellen werden bevorzugt verwendet, um erfindungsgemäße Proteasen herzustellen. Ein weiterer Gegenstand der Erfindung ist daher ein Verfahren zur Herstellung einer Protease umfassend
a) Kultivieren einer erfindungsgemäßen Wirtszelle, und
b) Isolieren der Protease aus dem Kulturmedium oder aus der Wirtszelle.

Dieser Erfindungsgegenstand umfasst bevorzugt Fermentationsverfahren.

Fermentationsverfahren sind an sich aus dem Stand der Technik bekannt und stellen den eigentlichen großtechnischen Produktionsschritt dar, in der Regel gefolgt von einer geeigneten Aufreinigungsmethode des hergestellten Produktes, z.B. der erfindungsgemäßen Proteasen. Alle Fermentationsverfahren, die auf einem entsprechenden Verfahren zur Herstellung einer erfindungsgemäßen Protease beruhen, stellen Ausführungsformen dieses Erfindungsgegenstandes dar. Fermentationsverfahren, die dadurch gekennzeichnet sind, dass die Fermentation über eine Zulaufstrategie durchgeführt wird, kommen insbesondere in Betracht. Hierbei werden die Medienbestandteile, die durch die fortlaufende Kultivierung verbraucht werden, zugefüttert. Hierdurch können beträchtliche Steigerungen sowohl in der Zelldichte als auch in der Zellmasse bzw. Trockenmasse und/oder insbesondere in der Aktivität der interessierenden Protease erreicht werden. Ferner kann die Fermentation auch so gestaltet werden, dass unerwünschte Stoffwechselprodukte herausgefiltert oder durch Zugabe von Puffer oder jeweils passende Gegenionen neutralisiert werden. Die hergestellte Protease kann aus dem Fermentationsmedium geerntet werden. Ein solches Fermentationsverfahren ist gegenüber einer Isolation der Protease aus der Wirtszelle, d.h. einer Produktaufbereitung aus der Zellmasse (Trockenmasse) bevorzugt, erfordert jedoch die Zurverfügungstellung von geeigneten Wirtszellen oder von einem oder mehreren geeigneten Sekretionsmarkern oder -mechanismen und/oder Transportsystemen, damit die Wirtszellen die Protease in das Fermentationsmedium sezernieren. Ohne Sekretion kann alternativ die Isolation der Protease aus der Wirtszelle, d.h. eine Aufreinigung derselben aus der Zellmasse, erfolgen, z.B. durch Fällung mit Ammoniumsulfat oder Ethanol, oder durch chromatographische Reinigung.

Alle vorstehend ausgeführten Sachverhalte können zu Verfahren kombiniert werden, um erfindungsgemäße Protease herzustellen.

Ein weiterer Gegenstand der Erfindung ist ein Wasch- und/oder Reinigungsmittel, das dadurch gekennzeichnet ist, dass es eine erfindungsgemäße Protease wie hierin beschrieben enthält.

Unter einem Wasch- oder Reinigungsmittel sind erfindungsgemäß alle denkbaren Wasch- oder Reinigungsmittelarten zu verstehen, sowohl Konzentrate als auch unverdünnt anzuwendende Mittel, zum Einsatz im kommerziellen Maßstab, in der Waschmaschine oder bei der Handwäsche bzw. -reinigung. Dazu gehören z.B. Waschmittel für Textilien, Teppiche, oder Naturfasern, für die die Bezeichnung Waschmittel verwendet wird. Dazu gehören z.B. auch Geschirrspülmittel für Geschirrspülmaschinen (maschinelle Geschirrspülmittel) oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder, für die die Bezeichnung Reinigungsmittel verwendet wird, also neben manuellen und maschinellen Geschirrspülmitteln z.B. auch Scheuermittel, Glasreiniger, WC-Duftspüler, usw. Zu den Wasch- und Reinigungsmittel im Rahmen der Erfindung zählen ferner Waschhilfsmittel, die bei der manuellen oder maschinellen Textilwäsche zum eigentlichen Waschmittel hinzudosiert werden, um eine weitere Wirkung zu erzielen. Ferner zählen zu Wasch- und Reinigungsmittel im Rahmen der Erfindung auch Textilvor- und Nachbehandlungsmittel, also solche Mittel, mit denen das Wäschestück vor der eigentlichen Wäsche in Kontakt gebracht wird, z.B. zum Anlösen hartnäckiger Verschmutzungen, und auch solche Mittel, die in einem der eigentlichen Textilwäsche nachgeschalteten Schritt dem Waschgut weitere wünschenswerte Eigenschaften wie angenehmen Griff, Knitterfreiheit oder geringe statische Aufladung verleihen. Zu letztgenannten Mittel werden u.a. die Weichspüler gerechnet. Mit umfasst sind dabei auch Mittel zur Verwendung in (halb-)automatisierten Wasch- oder Reinigungssystemen wie z.B. Wischrobotor oder Nassstaubsauger.

Erfindungsgemäße Mittel, die als pulverförmige oder granulare Feststoffe, in verdichteter oder nachverdichteter Teilchenform, als homogene Lösungen oder Suspensionen vorliegen können, können neben einer erfindungsgemäßen Protease alle bekannten und in derartigen Mitteln üblichen Inhaltsstoffe enthalten, wobei bevorzugt mindestens ein weiterer Inhaltsstoff in dem Mittel vorhanden ist. Erfindungsgemäße Mittel können insbesondere Tenside, Builder (Gerüststoffe), Polymere, Glaskorrosionsinhibitoren, Korrosionsinhibitoren, Bleichmittel wie Persauerstoffverbindungen, Bleichaktivatoren oder Bleichkatalysatoren enthalten. Ferner können sie wassermischbare organische Lösungsmittel, weitere Enzyme, Enzymstabilisatoren, Sequestrierungsmittel, Elektrolyte, pH-Regulatoren und/oder weitere Hilfsstoffe wie optische Aufheller, Vergrauungsinhibitoren, Farbübertragungsinhibitoren, Schaumregulatoren sowie Farb- und Duftstoffe sowie Kombinationen hiervon enthalten. Vorteilhafte Inhaltsstoffe erfindungsgemäßer Mittel sind offenbart in WO 2009/121725, dort beginnend auf Seite 5, vorletzter Absatz, und endend auf Seite 13 nach dem zweiten Absatz. Auf diese Offenbarung wird ausdrücklich Bezug genommen und der dortige Offenbarungsgehalt in die vorliegende Patentanmeldung einbezogen.

Erfindungsgemäße Mittel enthalten die erfindungsgemäße Protease vorteilhafterweise in einer Menge von 2 µg bis 20 mg, vorzugsweise von 5 µg bis 17,5 mg, besonders bevorzugt von 20 µg bis 15 mg und ganz besonders bevorzugt von 50 µg bis 10 mg pro g des Mittels. In verschiedenen Ausführungsformen beträgt die Konzentration der hierin beschriebenen Protease (aktives Enzym) in dem Mittel >0 bis 1 Gew.-%, vorzugsweise 0,0001 oder 0,001 bis 0,1 Gew.-% bezogen auf das Gesamtgewicht des Mittels oder der Zusammensetzung.

Erfindungsgemäße Mittel enthalten die erfindungsgemäße Protease zunehmend bevorzugt in einer Menge von 1 x 10⁻⁸ bis 5 Gew.-%, von 0,0001 bis 1 Gew.-%, von 0,0005 bis 0,5 Gew.-%, von 0,001 bis 0,1 Gew.-%, jeweils bezogen auf aktives Protein und bezogen auf das Gesamtgewicht des Mittels.

Weitere Ausführungsformen umfassen alle festen, pulverförmigen, flüssigen, gelförmigen oder pastösen Darreichungsformen erfindungsgemäßer Mittel, die ggf. auch aus mehreren Phasen bestehen können sowie in komprimierter oder nicht komprimierter Form vorliegen können. Erfindungsgemäße Mittel können als rieselfähiges Pulver vorliegen, insbesondere mit einem Schüttgewicht von 300 g/l bis 1200 g/l, insbesondere 500 g/l bis 900 g/l oder 600 g/l bis 850 g/l. Zu den festen Darreichungsformen erfindungsgemäßer Mittel zählen ferner Extrudate, Granulate, Tabletten oder Pouches. Alternativ kann erfindungsgemäße Mittel auch flüssig, gelförmig oder pastös sein, z.B. in Form eines nicht-wässrigen Flüssigwaschmittels oder einer nicht-wässrigen Paste oder in Form eines wässrigen Flüssigwaschmittels oder einer wasserhaltigen Paste. Flüssige Mittel sind generell bevorzugt. Weiterhin können erfindungsgemäße Mittel als Einkomponentensystem vorliegen. Solche Mittel bestehen aus einer Phase. Alternativ können erfindungsgemäße Mittel auch aus mehreren Phasen bestehen. Ein solches Mittel ist demnach in mehrere Komponenten aufgeteilt.

In bevorzugten Ausführungsformen ist das erfindungsgemäße Mittel ein Textilwaschmittel.

In bevorzugten Ausführungsformen ist das erfindungsgemäße Mittel ein flüssiges Textilwaschmittel.

In bevorzugten Ausführungsformen ist das erfindungsgemäße Mittel ein vorportioniertes Waschmittel, insbesondere eine Waschmittelportionseinheit umfassend eine erfindungsgemäße Waschmittelzubereitung und einen wasserlöslichen Film, welcher die Waschmittelzubereitung vollständig umschließt.

Der wasserlösliche Film, in welche die Waschmittelzubereitung verpackt ist, kann ein oder mehrere strukturell verschiedene wasserlösliche(s) Polymer(e) umfassen. Als wasserlösliche(s) Polymer(e) eignen sich insbesondere Polymere aus der Gruppe (ggf. acetalisierter) Polyvinylalkohole (PVAL) sowie deren Copolymere. Geeignete wasserlösliche Filme zum Einsatz werden u.a. von der Firma MonoSol LLC z.B. unter der Bezeichnung M8630, M8720, M8310, C8400 oder M8900 vertrieben. Geeignet sind z.B. auch Filme mit der Bezeichnung Solublon^{®} PT, Solublon^{®} GA, Solublon^{®} KC oder Solublon^{®} KL von der Aicello Chemical Europe GmbH oder die Folien VF-HP von Kuraray.

Wenn erfindungsgemäße Mittel in flüssiger Form vorliegen, enthalten sie vorzugsweise bezogen auf ihr Gesamtgewicht mehr als 40 Gew.-%, vorzugsweise 50 bis 90 Gew.-% und besonders bevorzugt 60 bis 80 Gew.-% Wasser.

Erfindungsgemäße Mittel können ein oder mehrere Tenside enthalten, wobei insbesondere anionische Tenside, nichtionische Tenside und deren Gemische in Frage kommen, aber auch kationische, zwitterionische und/oder amphotere Tenside können enthalten sein. Die Mittel enthalten vorzugsweise 5 bis 70 Gew.-% Tensid, bevorzugt 35 bis 60 Gew.-% und weiter bevorzugt 40 bis 55 Gew.-%, Tensid, bezogen auf das Gesamtgewicht des Mittels. In bevorzugten Ausführungsformen enthalten die Mittel vorzugsweise 3 bis 35 Gew.-%, bevorzugt 5 bis 30 Gew.-%, Tensid, bezogen auf das Gesamtgewicht des Mittels.

Geeignete anionische Tenside sind insbesondere Seifen und solche, die Sulfat- oder SulfonatGruppen mit bevorzugt Alkaliionen als Kationen enthalten. Verwendbare Seifen sind bevorzugt die Alkalisalze der gesättigten oder ungesättigten C₁₂₋₁₈-Fettsäuren. Derartige Fettsäuren können auch in nicht vollständig neutralisierter Form eingesetzt werden. Zu den brauchbaren Tensiden des Sulfat-Typs gehören die Salze der Schwefelsäurehalbester von C₁₂₋₁₈-Fettalkoholen und die Sulfatierungsprodukte der genannten nichtionischen Tenside mit niedrigem Ethoxylierungsgrad. Zu den verwendbaren Tensiden vom Sulfonat-Typ gehören z.B. C₉₋₁₄-Alkylbenzolsulfonate, Alkansulfonate, die aus C₁₂₋₁₈-Alkanen z.B. durch Sulfochlorierung oder Sulfoxidation mit anschließender Hydrolyse bzw. Neutralisation gewonnen werden, C₁₂₋₁₈-Olefinsulfonate, die bei der Umsetzung entsprechender Monoolefine mit Schwefeltrioxid entstehen, Gemische aus Alken- und Hydroxyalkansulfonaten, Disulfonaten, wie man sie z.B. aus C₁₂₋₁₈-Monoolefinen mit end- oder innenständigen Doppelbindung durch Sulfonieren mit gasförmigem Schwefeltrioxid und anschließende alkalische oder saure Hydrolyse der Sulfonierungsprodukte erhält, sowie α-Sulfofettsäureester (Estersulfonate), die bei der Sulfonierung von Fettsäuremethyl- oder -ethylestern entstehen, z.B. α-sulfonierte Methylester der hydrierten Kokos-, Palmkern- oder Talgfettsäuren.

Vorzugsweise weist das Mittel, bezogen auf das Gesamtgewicht des Mittels, 2 bis 55 Gew.-%, vorzugsweise 3 bis 35 Gew.-%, Aniontensid auf. Ganz besonders bevorzugt weist das Mittel 3 bis 25 Gew.-% Alkylbenzolsulfonat auf. Darüber hinaus kann das Mittel vorzugsweise noch weitere anionische Tenside, insbesondere Alkylethersulfate, sowie nichtionische Tenside, insbesondere Fettalkoholalkoxylate, enthalten. Diese können dann den Rest der Tenside ausmachen.

Geeignete Alkylbenzolsulfonate sind vorzugsweise ausgewählt aus linearen oder verzweigten Alkylbenzolsulfonaten der Formel 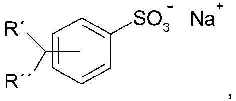 in der R' und R" unabhängig H oder Alkyl sind und zusammen 6 bis 19, vorzugsweise 7 bis 15 und insbesondere 9 bis 13 C-Atome enthalten. Ein ganz besonders bevorzugter Vertreter ist Natriumdodecylbenzylsulfonat.

Als Alk(en)ylsulfate werden die Alkali- und insbesondere die Natriumsalze der Schwefelsäurehalbester der C₁₂₋₁₈-Fettalkohole, z.B. aus Kokosfettalkohol, Talgfettalkohol, Lauryl-, Myristyl-, Cetyl- oder Stearylalkohol oder der C₁₀₋₂₀-Oxoalkohole und diejenigen Halbester sekundärer Alkohole dieser Kettenlängen bevorzugt. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Aus waschtechnischem Interesse sind die C₁₂₋₁₆-Alkylsulfate und C₁₂₋₁₅-Alkylsulfate sowie C₁₄₋₁₅-Alkylsulfate bevorzugt.

Auch die Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO, sind geeignet.

Geeignete Alkylethersulfate sind z.B. Verbindungen der Formel

R¹-O-(AO)ₙ-SO₃⁻ X⁺.

In dieser Formel steht R¹ für einen linearen oder verzweigten, substituierten oder unsubstituierten Alkylrest, vorzugsweise für einen linearen, unsubstituierten Alkylrest, besonders bevorzugt für einen Fettalkoholrest. Bevorzugte Reste R¹ sind ausgewählt aus Decyl-, Undecyl-, Dodecyl-, Tridecyl-, Tetradecyl-, Pentadecyl-, Hexadecyl-, Heptadecyl-, Octadecyl-, Nonadecyl-, Eicosylresten und deren Mischungen, wobei die Vertreter mit gerader Anzahl an C-Atomen bevorzugt sind. Besonders bevorzugte Reste R¹ sind abgeleitet von C₁₂₋₁₈-Fettalkoholen, z.B. von Kokosfettalkohol, Talgfettalkohol, Lauryl-, Myristyl-, Cetyl- oder Stearylalkohol oder von C₁₀₋₂₀-Oxoalkoholen. AO steht für eine Ethylenoxid-(EO) oder Propylenoxid-(PO)-Gruppierung, vorzugsweise für eine Ethylenoxidgruppierung. Der Index n steht für eine ganze Zahl von 1 bis 50, vorzugsweise von 1 bis 20 und insbesondere von 2 bis 10. Ganz besonders bevorzugt steht n für die Zahlen 2, 3, 4, 5, 6, 7 oder 8. X⁺ steht für ein einwertiges Kation oder den n-ten Teil eines n-wertigen Kations, bevorzugt sind dabei die Alkalimetallionen und darunter Na⁺ oder K⁺, wobei Na⁺ äußerst bevorzugt ist. Weitere Kationen X⁺ können ausgewählt sein aus NH₄⁺, ½ Zn²⁺, ½ Mg²⁺, ½ Ca²⁺, ½ Mn²⁺ und deren Mischungen.

In verschiedenen Ausführungsformen kann das Alkylethersulfat ausgewählt sein aus Fettalkoholethersulfaten der Formel 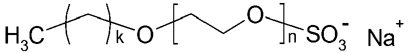 mit k = 11 bis 19, n = 2, 3, 4, 5, 6, 7 oder 8. Ganz besonders bevorzugte Vertreter sind Na-C₁₂₋₁₄-Fettalkoholethersulfate mit 2 EO (k = 11-13, n = 2). Der angegebenen Ethoxylierungsgrad stellt einen statistischen Mittelwert dar, der für ein spezielles Produkt eine ganze oder eine gebrochene Zahl sein kann. Die angegebenen Alkoxylierungsgrade stellen statistische Mittelwerte dar, die für ein spezielles Produkt eine ganze oder eine gebrochene Zahl sein können. Bevorzugte Alkoxylate/Ethoxylate weisen eine eingeengte Homologenverteilung auf (narrow range ethoxylates, NRE).

Es hat sich für die Kaltwaschleistung als vorteilhaft erwiesen, wenn die Mittel zusätzlich Seife(n) enthalten. Bevorzugte Mittel sind daher dadurch gekennzeichnet, dass sie Seife(n) enthalten. Geeignet sind gesättigte Fettsäureseifen, wie die Salze der Laurinsäure, Myristinsäure, Palmitinsäure, Stearinsäure, hydrierte Erucasäure und Behensäure sowie insbesondere aus natürlichen Fettsäuren, z.B. Kokos-, Palmkern- oder Talgfettsäuren, abgeleitete Seifengemische.

Geeignete nichtionische Tenside sind insbesondere Alkylglykoside und Ethoxylierungs- und/oder Propoxylierungsprodukte von Alkylglykosiden oder linearen oder verzweigten Alkoholen mit jeweils 8 bis etwa 18 C-Atomen im Alkylteil und 3 bis 20, vorzugsweise 4 bis 10 Alkylethergruppen. Weiterhin sind entsprechende Ethoxylierungs- und/oder Propoxylierungsprodukte von N-Alkylaminen, vicinalen Diolen, Fettsäureestern und Fettsäureamiden, die hinsichtlich des Alkylteils den genannten langkettigen Alkoholderivaten entsprechen, sowie von Alkylphenolen mit 5 bis 12 C-Atomen im Alkylrest brauchbar.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören z.B. C₁₂₋₁₄-Alkohole mit 3 EO oder 4 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 5 EO. Die angegebenen Ethoxylierungsgrade stellen statistische Mittelwerte dar, die für ein spezielles Produkt eine ganze oder eine gebrochene Zahl sein können. Bevorzugte Alkoholethoxylate weisen eine eingeengte Homologenverteilung auf (narrow range ethoxylates, NRE). Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.

Eine weitere Klasse bevorzugt eingesetzter nichtionischer Tenside, die entweder als alleiniges nichtionisches Tensid oder in Kombination mit anderen nichtionischen Tensiden eingesetzt werden, sind alkoxylierte, vorzugsweise ethoxylierte oder ethoxylierte und propoxylierte Fettsäurealkylester, vorzugsweise mit 1 bis 4 Kohlenstoffatomen in der Alkylkette, insbesondere Fettsäuremethylester.

Eine weitere Klasse von nichtionischen Tensiden, die vorteilhaft eingesetzt werden kann, sind die Alkylpolyglycoside (APG). Einsetzbare Alkylpolyglycoside genügen der allgemeinen Formel

RO(G)_{z},

in der R für einen linearen oder verzweigten, insbesondere in 2-Stellung methylverzweigten, gesättigten oder ungesättigten, aliphatischen Rest mit 8 bis 22, vorzugsweise 12 bis 18 C-Atomen bedeutet und G das Symbol ist, das für eine Glykoseeinheit mit 5 oder 6 C-Atomen, vorzugsweise für Glucose, steht. Der Glycosidierungsgrad z liegt dabei zwischen 1 und 4, vorzugsweise zwischen 1 und 2 und insbesondere zwischen 1,1 und 1,4. Bevorzugt eingesetzt werden lineare Alkylpolyglycoside, also Alkylpolyglycoside, in denen der Polyglycosylrest ein Glucoserest und der Alkylrest ein n-Alkylrest ist.

Auch nichtionische Tenside vom Typ der Aminoxide, z.B. N-Kokosalkyl-N,N-dimethylaminoxid und N-Talgalkyl-N,N-dihydroxyethylaminoxid, und der Fettsäurealkanolamide können geeignet sein. Die Menge dieser nichtionischen Tenside beträgt vorzugsweise nicht mehr als die der ethoxylierten Fettalkohole, insbesondere nicht mehr als die Hälfte davon.

Geeignete Amphotenside sind z.B. Betaine der Formel

(Rⁱⁱⁱ)(R^{iv})(R^{v})N⁺CH₂COO⁻,

in der Rⁱⁱⁱ einen ggf. durch Heteroatome oder Heteroatomgruppen unterbrochenen Alkylrest mit 8 bis 25, vorzugsweise 10 bis 21 Kohlenstoffatomen und R^{iv} sowie R^{v} gleichartige oder verschiedene Alkylreste mit 1 bis 3 Kohlenstoffatomen bedeuten, insbesondere C₁₀₋₁₈-Alkyldimethylcarboxymethylbetain und C₁₁₋₁₇-Alkylamidopropyldimethylcarboxymethylbetain.

Geeignete kationische Tenside sind u.a. die quartären Ammoniumverbindungen der Formel

(R^{vi})(R^{vii})(R^{viii})(R^{ix})N⁺X⁻,

in der R^{vi} bis R^{ix} für vier gleich- oder verschiedenartige, insbesondere zwei lang- und zwei kurzkettige, Alkylreste und X⁻ für ein Anion, insbesondere ein Halogenidion, stehen, z.B. Didecyldimethylammoniumchlorid, Alkylbenzyldidecylammoniumchlorid und deren Mischungen. Weitere geeignete kationische Tenside sind die quaternären oberflächenaktiven Verbindungen, insbesondere mit einer Sulfonium-, Phosphonium-, Jodonium- oder Arsoniumgruppe, die auch als antimikrobielle Wirkstoffe bekannt sind. Durch den Einsatz von quaternären oberflächenaktiven Verbindungen mit antimikrobieller Wirkung kann das Mittel mit einer antimikrobiellen Wirkung ausgestaltet werden bzw. dessen ggf. aufgrund anderer Inhaltsstoffe bereits vorhandene antimikrobielle Wirkung verbessert werden.

In bevorzugten Ausführungsformen umfasst das erfindungsgemäße Wasch- und Reinigungsmittel, jeweils bezogen auf das Gesamtgewicht des Mittels,
(i) 2 bis 20 Gew.-%, vorzugsweise 3 bis 17 Gew.-% anionische Tenside,
(ii) 1 bis 10 Gew.-%, vorzugsweise 3 bis 8 Gew.-%, nichtionische Tenside,
(iii) 0 bis 1 Gew.-%, vorzugsweise 0 bis 0,5 Gew.-%, Seife, und
(iv) 0 bis 5 Gew.-%, vorzugsweise 0 bis 3 Gew.-% Fettsäuren.

Ein weiterer bevorzugter Bestandteil erfindungsgemäßer Mittel sind Komplexbildner. Besonders bevorzugte Komplexbildner sind die Phosphonate, sofern ihr Einsatz regulatorisch zulässig ist. Die komplexbildenden Phosphonate umfassen neben der 1-Hydroxyethan-1,1-diphosphonsäure eine Reihe unterschiedlicher Verbindungen, wie z.B. Diethylentriaminpenta(methylenphosphonsäure) (DTPMP). In dieser Anmeldung bevorzugt sind insbesondere Hydroxyalkan- bzw. Aminoalkanphosphonate. Unter den Hydroxyalkanphosphonaten ist das 1-Hydroxyethan-1,1-diphosphonat (HEDP) von besonderer Bedeutung als Cobuilder. Es wird vorzugsweise als Natriumsalz eingesetzt, wobei das Dinatriumsalz neutral und das Tetranatriumsalz alkalisch (pH 9) reagiert. Als Aminoalkanphosphonate kommen vorzugsweise Ethylendiamintetramethylenphosphonat (EDTMP), Diethylentriaminpentamethylenphosphonat (DTPMP) sowie deren höhere Homologe in Frage. Sie werden vorzugsweise in Form der neutral reagierenden Natriumsalze, z.B. als Hexanatriumsalz der EDTMP bzw. als Hepta- und Octa-Natriumsalz der DTPMP, eingesetzt. Als Builder wird dabei aus der Klasse der Phosphonate bevorzugt HEDP verwendet. Die Aminoalkanphosphonate besitzen zudem ein ausgeprägtes Schwermetallbindevermögen. Dementsprechend kann es, insbesondere wenn die Mittel auch Bleiche enthalten, bevorzugt sein, Aminoalkanphosphonate, insbesondere DTPMP, einzusetzen, oder Mischungen aus den genannten Phosphonaten zu verwenden. Ein im Rahmen dieser Anmeldung bevorzugtes Mittel enthält ein oder mehrere Phosphonat(e) aus der Gruppe Aminotrimethylenphosphonsäure (ATMP) und/oder deren Salze; Ethylendiamintetra(methylenphosphonsäure) (EDTMP) und/oder deren Salze; Diethylentriaminpenta(methylenphosphonsäure) (DTPMP) und/oder deren Salze; 1-Hydroxyethan-1,1-diphosphonsäure (HEDP) und/oder deren Salze; 2-Phosphonobutan-1,2,4-tricarbonsäure (PBTC) und/oder deren Salze; Hexamethylendiamintetra(methylenphosphonsäure) (HDTMP) und/oder deren Salze; Nitrilotri(methylenphosphonsäure) (NTMP) und/oder deren Salze. Besonders bevorzugt sind Mittel, welche als Phosphonate 1-Hydroxyethan-1,1-diphosphonsäure (HEDP) oder Diethylentriaminpenta(methylenphosphonsäure) (DTPMP) enthalten. Selbstverständlich können erfindungsgemäße Mittel zwei oder mehr unterschiedliche Phosphonate enthalten.

Bevorzugte erfindungsgemäße Mittel sind dadurch gekennzeichnet, dass das Mittel mindestens einen Komplexbildner aus der Gruppe der Phosphonate, vorzugsweise 1-Hydroxyethan-1,1-diphosphonat, enthält, wobei der Gewichtsanteil des Phosphonat am Gesamtgewicht des Mittels vorzugsweise 0,1 und 8,0 Gew.-%, bevorzugt 0,2 und 5,0 Gew.-%, weiter bevorzugt 0,3 und 3,0 Gew.-% und besonders bevorzugt 0,5-2,0 Gew.-% beträgt.

In weiter bevorzugten Ausführungsformen sind erfindungsgemäße Mittel im Wesentlichen frei von phosphonathaltigen Verbindungen. "Im Wesentlichen frei von phosphonathaltigen Verbindungen" bedeutet in diesem Zusammenhang, dass die entsprechende Mittel oder Zusammensetzungen, bezogen auf das Gesamtgewicht des Mittels, weniger als 2 Gew.-%, vorzugsweise weniger als 1 Gew.-%, weiter bevorzugt weniger als 0,5 Gew.-% und besonders bevorzugt weniger als 0,1 Gew.-%, phosphonathaltige Verbindungen enthalten. In besonders bevorzugten Ausführungsformen sind diese Mittel/Zusammensetzungen frei von phosphonathaltigen Verbindungen.

Erfindungsgemäße Mittel enthalten weiterhin vorzugsweise Gerüststoff (Builder), vorzugsweise mindestens einen wasserlöslichen und/oder wasserunlöslichen, organischen und/oder anorganischen Builder. Zu den Gerüststoffe zählen dabei insbesondere die Silikate, Carbonate und organische Cobuilder.

Als organische Cobuilder sind insbesondere Polycarboxylate/Polycarbonsäuren, polymere Polycarboxylate, Asparaginsäure, Polyacetale, Dextrine, weitere organische Cobuilder sowie Phosphonate zu nennen. Diese Stoffklassen werden nachfolgend beschrieben. Organische Cobuildersubstanzen können gewünschtenfalls in Mengen bis zu 40 Gew.-%, insbesondere bis zu 25 Gew.-% und vorzugsweise von 1 bis 8 Gew.-% enthalten sein, bezogen auf das Gesamtgewicht des Mittels. Brauchbare organische Gerüstsubstanzen sind z.B. die in Form der freien Säure und/oder ihrer Natriumsalze einsetzbaren Polycarbonsäuren, wobei unter Polycarbonsäuren solche Carbonsäuren verstanden werden, die mehr als eine Säurefunktion tragen. Beispielsweise sind dies Citronensäure, Adipinsäure, Bernsteinsäure, Glutarsäure, Äpfelsäure, Weinsäure, Maleinsäure, Fumarsäure, Zuckersäuren und Carboxymethylinuline, monomere und polymere Aminopolycarbonsäuren, insbesondere Glycindiessigsäure, Methylglycindiessigsäure, Glutamindiessigsäure, Nitrilotriessigsäure (NTA), Iminodisuccinat wie Ethylendiamin-N,N'-dibernsteinsäure und Hydroxyiminodisuccinate, Ethylendiamintetraessigsäure sowie Polyasparaginsäure, Polyphosphonsäuren, insbesondere Aminotris(methylenphosphonsäure), Ethylendiamintetrakis(methylenphosphonsäure), Lysintetra(methylenphosphosäure) und 1-Hydroxyethan-1,1-diphosphonsäure, polymere Hydroxyverbindungen wie Dextrin sowie polymere (Poly)carbonsäuren, insbesondere durch Oxidation von Polysacchariden bzw. Dextrinen zugängliche Polycarboxylate, und/oder polymere Acrylsäuren, Methacrylsäuren, Maleinsäuren und Mischpolymere aus diesen, die auch geringe Anteile polymerisierbarer Substanzen ohne Carbonsäurefunktionalität einpolymerisiert enthalten können. Derartige organische Buildersubstanzen können gewünschtenfalls in Mengen bis zu 50 Gew.-%, insbesondere bis zu 25 Gew.-%, vorzugsweise von 10 bis 20 Gew.-% und besonders bevorzugt von 1 bis 5 Gew.-% enthalten sein, bezogen auf das Gesamtgewicht des Mittels. Die freien Säuren besitzen neben ihrer Builderwirkung typischerweise auch die Eigenschaft einer Säuerungskomponente und dienen somit auch zur Einstellung eines niedrigeren und milderen pH-Wertes von Mitteln. Insbesondere sind hierbei Citronensäure, Bernsteinsäure, Glutarsäure, Adipinsäure, Gluconsäure und beliebige Mischungen aus diesen zu nennen. Mit besonderem Vorzug wird als Gerüstsubstanz die Citronensäure oder Salze der Citronensäure eingesetzt. Weitere besonders bevorzugte Gerüstsubstanzen sind ausgewählt unter Methylglycindiessidsäure (MGDA), Glutaminsäurediacetat (GLDA), Asparaginsäurediacetat (ASDA), Hydroxyethyliminodiacetat (HEIDA), Iminodisuccinat (IDS) und Ethylendiamindisuccinat (EDDS), Carboxymethylinulin und Polyaspartat.

In bevorzugten Ausführungsformen wird als wasserlöslicher, organischer Builder Citronensäure und/oder Citrat eingesetzt. Besonders bevorzugt ist der Einsatz, bezogen auf das Gesamtgewicht des Mittels, von 0,5 bis 25 Gew.-%, vorzugsweise 0,75 bis 12,5 Gew.-%, weiter bevorzugt 1 bis 4 Gew.-% Citronensäure und/oder 0,5 bis 25 Gew.-%, vorzugsweise 0,75 bis 12,5 Gew.-%, weiter bevorzugt 1 bis 4 Gew.-% Citrat, vorzugsweise Alkalicitrat, noch bevorzugter Natriumcitrat. Citronensäure/Citrat können jeweils in Form ihrer Hydrate eingesetzt werden, so kann z.B. Citronensäure in Form des Monohydrats, Citrat in Form des Trinatriumcitratdihydrats eingesetzt werden.

In bevorzugteren Ausführungsformen sind die Builder-Substanzen ausgewählt aus MGDA und GLDA. Wie hier verwendet, umfasst der Begriff "MGDA" unter anderem Methylglycindiessigsäure, α-Alanindiessigsäure, N-(1-Carboxyethyl)-iminodiessigsäure und N,N-Bis(carboxymethyl)-DL-Alanin, wobei die freien Säureformen und die entsprechenden Salze, vorzugsweise Alkalisalze, insbesondere Trinatriumsalze, eingeschlossen sind. Wie hier verwendet, umfasst der Begriff "GLDA" unter anderem Glutaminsäurediessigsäure, L-Glutaminsäure-N,N-diessigsäure und N,N-Bis(carboxylatomethyl)-L-Glutamat, wobei freie Säureformen und entsprechende Salze, vorzugsweise Alkalisalze, insbesondere Tetranatriumsalze, eingeschlossen sind. Obwohl, bezogen auf das Gesamtgewicht des Mittels, höhere MGDA- oder GLDA-Konzentrationen möglich sind, insbesondere bis zu 25 Gew.-%, wird besonders bevorzugt 0,2 bis 5 Gew.-%, vorzugsweise 0,25 bis 3 Gew.-%, noch bevorzugter 0,5 bis 2 Gew.-% MGDA, vorzugsweise MGDA-Trinatriumsalz (MGDA-Na₃) verwendet. Noch bevorzugter ist die Verwendung, bezogen auf das Gesamtgewicht des Mittels, von 0,2 bis 5 Gew.-%, vorzugsweise 0,25 bis 3 Gew.-%, noch bevorzugter von 0,5 bis 2 Gew.-% GLDA, vorzugsweise GLDA-Tetranatriumsalz (GLDA-Na₄).

Als Gerüststoffe sind weiter polymere Polycarboxylate geeignet, dies sind z.B. die Alkalimetallsalze der Polyacrylsäure oder der Polymethacrylsäure, z.B. solche mit einer relativen Molekülmasse von 500 bis 70.000 g/mol. Bei den für polymere Polycarboxylate angegebenen Molmassen handelt es sich im Sinne dieser Anmeldung um gewichtsmittlere Molmassen Mw der jeweiligen Säureform, die grundsätzlich mittels Gelpermeationschromatographie (GPC) bestimmt wurden, wobei ein UV-Detektor eingesetzt wurde. Die Messung erfolgte dabei gegen einen externen Polyacrylsäure-Standard, der aufgrund seiner strukturellen Verwandtschaft mit den untersuchten Polymeren realistische Molgewichtswerte liefert. Diese Angaben weichen deutlich von den Molgewichtsangaben ab, bei denen Polystyrolsulfonsäuren als Standard eingesetzt werden. Die gegen Polystyrolsulfonsäuren gemessenen Molmassen sind in der Regel deutlich höher als die in dieser Anmeldung angegebenen Molmassen. Geeignete Polymere sind insbesondere Polyacrylate, die bevorzugt eine Molekülmasse von 2.000 bis 20.000 g/mol aufweisen. Aufgrund ihrer überlegenen Löslichkeit können aus dieser Gruppe wiederum die kurzkettigen Polyacrylate, die Molmassen von 2.000 bis 10.000 g/mol, und besonders bevorzugt von 3.000 bis 5.000 g/mol, aufweisen, bevorzugt sein. Geeignet sind weiterhin copolymere Polycarboxylate, insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im Allgemeinen 2.000 bis 70.000 g/mol, vorzugsweise 20.000 bis 50.000 g/mol und insbesondere 30.000 bis 40.000 g/mol.

Ein erfindungsgemäßes festes Mittel enthält vorzugsweise mindestens einen wasserlöslichen und/oder wasserunlöslichen, organischen und/oder anorganischen Builder. Zu den wasserlöslichen organischen Buildersubstanzen gehören die oben genannten organischen Gerüstsubstanzen.

Zusätzlich zu den vorstehend genannten wasserlöslichen organischen Buildern, können die Mittel der Erfindung weiterhin auch anorganische wasserlösliche Builder enthalten. Als wasserlösliche anorganische Buildermaterialien kommen insbesondere Alkalisilikate, Alkalicarbonate, Alkalihydrogenbarbonate, Alkaliphosphate und/oder Sesquicarbonate, die in Form ihrer alkalischen, neutralen oder sauren Natrium- oder Kaliumsalze vorliegen können, in Betracht. Ggf. können auch geringe Mengen an Calciumcarbonate in festen Textilwaschmitteln enthalten sein. Geeignet sind z.B. wasserlöslichen kristalline und/oder amorphe Alkalisilikate. Die in den erfindungsgemäßen Mitteln als Gerüststoffe brauchbaren Alkalisilikate weisen vorzugsweise ein molares Verhältnis von Alkalioxid zu SiO₂ unter 0,95, insbesondere von 1:1,1 bis 1:12 auf und können amorph oder kristallin vorliegen. Bevorzugte Alkalisilikate sind die Natriumsilikate, insbesondere die amorphen Natriumsilikate, mit einem molaren Verhältnis Na₂O:SiO₂ von 1:2 bis 1:2,8. Als kristalline Silikate, die allein oder im Gemisch mit amorphen Silikaten vorliegen können, werden vorzugsweise kristalline Schichtsilikate der allgemeinen Formel Na₂SiₓO₂ₓ₊₁ · y H₂O eingesetzt, in der x, das sogenannte Modul, eine Zahl von 1,9 bis 22, insbesondere 1,9 bis 4 und y eine Zahl von 0 bis 33 ist und bevorzugte Werte für x 2, 3 oder 4 sind. Bevorzugte kristalline Schichtsilikate sind solche, bei denen x in der genannten allgemeinen Formel die Werte 2 oder 3 annimmt. Insbesondere sind sowohl β- als auch δ-Natriumdisilikate (Na₂Si₂O₅ · y H₂O) bevorzugt. Auch aus amorphen Alkalisilikaten hergestellte, praktisch wasserfreie kristalline Alkalisilikate der oben genannten allgemeinen Formel, in der x eine Zahl von 1,9 bis 2,1 bedeutet, können in erfindungsgemäßen Mitteln eingesetzt werden. In einer weiteren Ausführungsform erfindungsgemäßer Mittel wird ein kristallines Natriumschichtsilikat mit einem Modul von 2 bis 3 eingesetzt, wie es aus Sand und Soda hergestellt werden kann. Kristalline Natriumsilikate mit einem Modul im Bereich von 1,9 bis 3,5 werden in einer weiteren Ausführungsform erfindungsgemäßer Mittel eingesetzt. In Mitteln, die sowohl amorphe als auch kristalline Alkalisilikate enthalten, beträgt das Gewichtsverhältnis von amorphem Alkalisilikat zu kristallinem Alkalisilikat vorzugsweise 1:2 bis 2:1 und insbesondere 1:1 bis 2:1. Kristalline schichtförmige Silikate der oben angegebenen Formel werden von der Fa. Clariant GmbH unter dem Handelsnamen Na-SKS vertrieben, z.B. Na-SKS-1 (Na₂Si₂₂O₄₅ · x H₂O, Kenyait), Na-SKS-2 (Na₂Si₁₄O₂₉ · x H₂O, Magadiit), Na-SKS-3 (Na₂Si₈O₁₇ · x H₂O) oder Na-SKS-4 (Na₂Si₄O₉ · x H₂O, Makatit). Von diesen eignen sich vor allem Na-SKS-5 (α-Na₂Si₂O₅), Na-SKS-7 (β-Na₂Si₂O₅, Natrosilit), Na-SKS-9 (NaHSi₂O₅ · 3 H₂O), Na-SKS-10 (NaHSi₂O₅ · 3 H₂O, Kanemit), Na-SKS-11 (t-Na₂Si₂O₅) und Na-SKS-13 (NaHSi₂O₅), insbesondere aber Na-SKS-6 (δ-Na₂Si₂O₅). In einer Ausgestaltung erfindungsgemäßer Mittel setzt man ein granulares Compound aus kristallinem Schichtsilikat und Citrat, aus kristallinem Schichtsilikat und oben genannter (co-)polymerer Polycarbonsäure, oder aus Alkalisilikat und Alkalicarbonat ein, wie es z.B. unter dem Namen Nabion^{®} 15 im Handel erhältlich ist. Derartige wasserlösliche anorganischen Buildermaterialien sind in den erfindungsgemäßen Mitteln vorzugsweise in Mengen von 1 bis 20 Gew.-%, insbesondere 5 bis 15 Gew.-%, enthalten, bezogen auf das Gesamtgewicht des Mittels. Des Weiteren sind als wasserlösliche anorganische Buildersubstanzen noch die Carbonate (und Hyodrogencarbonate), insbesondere Natriumcarbonat, und die Phosphonsäuren/Phosphonate von Bedeutung.

In bevorzugten Ausführungsformen sind erfindungsgemäße Mittel im Wesentlichen frei von phosphathaltigen Verbindungen sind. "Im Wesentlichen frei von phosphathaltigen Verbindungen" bedeutet in diesem Zusammenhang, dass die entsprechende Mittel weniger als 2 Gew.-%, vorzugsweise weniger als 1 Gew.-%, weiter bevorzugt weniger als 0,5 Gew.-% und besonders bevorzugt weniger als 0,1 Gew.-%, phosphathaltige Verbindungen, bezogen auf das Gesamtgewicht des Mittels enthalten. In besonders bevorzugten Ausführungsformen sind diese Mittel frei von phosphathaltigen Verbindungen. D.h. erfindungsgemäße Mittel enthalten keinen bewusst zugegebenen Phosphat-Builder.

Erfindungsgemäße Mittel können auch wasserunlösliche Buildersubstanzen enthalten. Als wasserunlösliche anorganische Buildermaterialien werden insbesondere kristalline oder amorphe wasserdispergierbare Alkalialumosilikate, in Mengen von bis zu 50 Gew.-%, vorzugsweise nicht über 40 Gew.-%, insbesondere von 3 bis 20 Gew.-% und besonders bevorzugt von 1 bis 15 Gew.-%, bezogen auf das Gesamtgewicht des Mittels, eingesetzt. Unter diesen sind die kristallinen Natriumalumosilikate in Waschmittelqualität, insbesondere Zeolith A, Zeolith P, Zeolith MAP und ggf. Zeolith X, allein oder in Mischungen, z.B. in Form eines Co-Kristallisats aus den Zeolithen A und X (Vegobond^{®} AX, ein Handelsprodukt der Condea Augusta S.p.A.), bevorzugt. Mengen nahe der genannten Obergrenze werden vorzugsweise in festen, teilchenförmigen Mitteln eingesetzt. Geeignete Alumosilikate weisen insbesondere keine Teilchen mit einer Korngröße über 30 µm auf und bestehen vorzugsweise zu wenigstens 80 Gew.-% aus Teilchen mit einer Größe unter 10 µm. Ihr Calciumbindevermögen, das gemäß DE 2412837 A1 bestimmt werden kann, liegt in der Regel im Bereich von 100 bis 200 mg CaO pro Gramm.

In bevorzugten Ausführungsformen umfassen erfindungsgemäße Mittel ein Buildersystem, umfassend mindestens einen Builder, vorzugsweise in einer Menge von 0,5 bis 50 Gew.-%, bevorzugt 0,5 bis 20 Gew.-%, besonders bevorzugt 0,5 bis 10 Gew.-%, bezogen auf das Gesamtgewicht des Mittels, wobei der Builder aus der aus Polycarbonsäuren wie Zitronensäure, Adipinsäure, Bernsteinsäure, Glutarsäure, Äpfelsäure, Weinsäure, Maleinsäure Fumarsäure, Zuckersäuren und Carboxymethylinuline oder deren Salzen, monomeren und polymeren Aminopolycarbonsäuren wie Glycindiessigsäure, Methylglycindiessigsäure (MGDA), Glutaminsäurediessigsäure (GLDA), Nitriltriessigsäure, Iminodisuccinat wie Ethylendiamin-N,N'-Disuccinsäure und Hydroxyiminodisuccinate, Ethylendiamintetraessigsäure und Polyasparaginsäure oder deren Salze, Polyphosphonsäuren wie Aminotris(methylenphosphonsäure), Ethylendiamintetrakis(methylenphosphonsäure), Lysintetra(methylenphosphonsäure), Diethylentriaminpenta(methylenphosphonsäure) (DTPMP) und 1-Hydroxyethan-1,1-diphosphonsäure (HEDP) oder deren Salze, polymeren Hydroxyverbindungen wie Dextrin, und Mischungen davon bestehenden Gruppe ausgewählt ist.

In bevorzugten Ausführungsformen umfassen erfindungsgemäße Mittel, jeweils bezogen auf das Gesamtgewicht des Mittels,
(i) 0 bis 10 Gew.-%, vorzugsweise 1 bis 4 Gew.-% Zitronensäure und/oder Citrat, vorzugsweise Alkalicitrat,
(ii) 0 bis 40 Gew.-%, vorzugsweise 0 bis 15 Gew.-%, stärker bevorzugt 1 bis 3 Gew.-%, Alkalicarbonat, vorzugsweise Natriumcarbonat,
(iii) 0 bis 20 Gew.-%, vorzugsweise 3 bis 10 Gew.-% Alkalisilikat,
(iv) 0 bis 10 Gew.-%, vorzugsweise 0,5 bis 2 Gew.-%, Phosphonsäure und/oder Alkaliphosphonat, besonders bevorzugt HEDP und/oder DTPMP, und/oder
(v) 0 bis 10 Gew.-%, vorzugsweise 0,5 bis 3 Gew.-%, Aminopolycarbonsäuren, vorzugsweise MGDA und/oder GLDA.

In Ergänzung zu den zuvor beschriebenen Gerüststoffen können in dem Mittel reinigungsaktive Polymere enthalten sein. Der Gewichtsanteil der reinigungsaktiven Polymere am Gesamtgewicht erfindungsgemäßer Mittel beträgt vorzugsweise 0,1 bis 20 Gew.-%, vorzugsweise 1,0 bis 15 Gew.-% und weiter bevorzugt 2,0 bis 12 Gew.-%.

Als für den Einsatz in erfindungsgemäßen Mitteln geeignete Persauerstoffverbindungen kommen insbesondere organische Persäuren bzw. persaure Salze organischer Säuren, wie Phthalimidopercapronsäure, Perbenzoesäure oder Salze der Diperdodecandisäure, Wasserstoffperoxid und unter den Waschbedingungen Wasserstoffperoxid abgebende anorganische Salze, zu denen Perborat, Percarbonat, Persilikat und/oder Persulfat wie Caroat gehören, sowie Wasserstoffperoxid-Einschlussverbindungen, wie H₂O₂-Harnstoffaddukte, in Betracht. Wasserstoffperoxid kann dabei auch mit Hilfe eines enzymatischen Systems, d.h. einer Oxidase und ihres Substrates, erzeugt werden. Sofern feste Persauerstoffverbindungen eingesetzt werden sollen, können diese in Form von Pulvern oder Granulaten verwendet werden, die auch in im Prinzip bekannter Weise umhüllt sein können. Die Persauerstoffverbindungen können als solche oder in Form diese enthaltender Mittel, die prinzipiell alle üblichen Wasch-, Reinigungs- oder Desinfektionsmittelbestandteile enthalten können, zu der Waschlauge zugegeben werden. Besonders bevorzugt wird Alkalipercarbonat oder Alkaliperborat-Monohydrat eingesetzt. Falls ein erfindungsgemäßes Mittel Persauerstoffverbindungen enthält, sind diese in Mengen von vorzugsweise bis zu 50 Gew.-%, insbesondere von 5 bis 30 Gew.-%, weiter bevorzugt von 0,1 bis 20 Gew.-% vorhanden, bezogen auf das Gesamtgewicht des Mittels.

In bevorzugten Ausführungsformen sind erfindungsgemäße Mittel im Wesentlichen frei von Persauerstoffverbindungen. "Im Wesentlichen frei von persauerstoffhaltigen Verbindungen" bedeutet in diesem Zusammenhang, dass die entsprechende Mittel oder Zusammensetzungen weniger als 2 Gew.-%, vorzugsweise weniger als 1 Gew.-%, weiter bevorzugt weniger als 0,5 Gew.-% und besonders bevorzugt weniger als 0,1 Gew.-%, persauerstoffhaltigen Verbindungen, bezogen auf das Gesamtgewicht des Mittels/der Zusammensetzung, enthalten. In besonders bevorzugten Ausführungsformen sind diese Mittel/Zusammensetzungen frei von persauerstoffhaltigen Verbindungen.

Als Bleichaktivatoren können in den Mitteln Verbindungen, die unter Perhydrolysebedingungen aliphatische Peroxocarbonsäuren mit vorzugsweise 1 bis 10 C-Atomen, insbesondere 2 bis 4 C-Atomen, und/oder ggf. substituierte Perbenzoesäure ergeben, eingesetzt werden. Geeignet sind Substanzen, die O- und/oder N-Acylgruppen der genannten C-Atomzahl und/oder ggf. substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate oder - carboxylate bzw. die Sulfon- oder Carbonsäuren von diesen, insbesondere Nonanoyl- oder Isononanoyloxybenzolsulfonat oder Laroyloxybenzolsulfonat (NOBS bzw. iso-NOBS bzw. LOBS), 4-(2-Decanoyloxyethoxycarbonyloxy)-benzolsulfonat (DECOBS) oder Decanoyloxybenzoat (DOBA), Carbonsäureanhydride, insbesondere Phthalsäureanhydrid, acylierte mehrwertige Alkohole, insbesondere Triacetin, Ethylenglykoldiacetat, 2,5-Diacetoxy-2,5-dihydrofuran und Enolester sowie acetyliertes Sorbitol und Mannitol bzw. deren beschriebene Mischungen (SORMAN), acylierte Zuckerderivate, insbesondere Pentaacetylglukose (PAG), Pentaacetylfruktose, Tetraacetylxylose und Octaacetyllactose, acetyliertes, ggf. N-alkyliertes Glucamin und Gluconolacton, N-acylierte Lactame, z.B. N-Benzoylcaprolactam, Nitrile, aus denen sich Perimidsäuren bilden, insbesondere Aminoacetonitrilderivate mit quaterniertem Stickstoffatom, und/oder sauerstoffübertragende Sulfonimine und/oder Acylhydrazone. Die hydrophil substituierten Acylacetale und die Acyllactame werden ebenfalls bevorzugt eingesetzt. Auch Kombinationen konventioneller Bleichaktivatoren können eingesetzt werden. Derartige Bleichaktivatoren können, insbesondere bei Anwesenheit obengenannter Wasserstoffperoxid-liefernder Bleichmittel, im üblichen Mengenbereich, vorzugsweise in Mengen von 0,5 bis 10 Gew.-%, insbesondere 1 bis 8 Gew.-%, bezogen auf das Gesamtgewicht des Mittels, enthalten sein, fehlen bei Einsatz von Percarbonsäure als alleinigem Bleichmittel jedoch vorzugsweise ganz.

Zusätzlich zu den konventionellen Bleichaktivatoren oder an deren Stelle können in festen Mitteln auch Sulfonimine und/oder bleichverstärkende Übergangsmetallsalze bzw. Übergangsmetallkomplexe als sogenannte Bleichkatalysatoren enthalten sein.

In bevorzugten Ausführungsformen sind erfindungsgemäße Mittel im Wesentlichen frei von Bleichaktivatoren und/oder frei von Bleichkatalysatoren. "Im Wesentlichen frei" bedeutet in diesem Zusammenhang, dass die entsprechende Mittel oder Zusammensetzungen, bezogen auf das Gesamtgewicht des Mittels, weniger als 2 Gew.-%, vorzugsweise weniger als 1 Gew.-%, weiter bevorzugt weniger als 0,5 Gew.-% und besonders bevorzugt weniger als 0,1 Gew.-%, Bleichaktivatoren und/oder Bleichkatalysatoren enthalten. In besonders bevorzugten Ausführungsformen sind diese Mittel/Zusammensetzungen frei von Bleichaktivatoren und/oder Bleichkatalysatoren.

In bevorzugten Ausführungsformen sind erfindungsgemäße Mittel im Wesentlichen frei von Bleiche, d.h. im Wesentlichen frei von jeglichen bleichenden Substanzen, insbesondere frei von den zuvor stehend beschriebenen Persauerstoffverbindungen, Bleichaktivatoren und Bleichkatalysatoren. "Im Wesentlichen frei" bedeutet in diesem Zusammenhang, dass die entsprechende Mittel oder Zusammensetzungen, bezogen auf das Gesamtgewicht des Mittels, weniger als 2 Gew.-%, vorzugsweise weniger als 1 Gew.-%, weiter bevorzugt weniger als 0,5 Gew.-% und besonders bevorzugt weniger als 0,1 Gew.-%, Bleiche enthalten, d.h. jeglichen bleichenden Substanzen, insbesondere den zuvor stehend beschriebenen Persauerstoffverbindungen, Bleichaktivatoren und Bleichkatalysatoren. In besonders bevorzugten Ausführungsformen sind diese Mittel/Zusammensetzungen frei von Bleiche, d.h. frei von jeglichen bleichenden Substanzen, insbesondere frei von den zuvor stehend beschriebenen Persauerstoffverbindungen, Bleichaktivatoren und Bleichkatalysatoren.

Geeignete Vergrauungsinhibitoren bzw. soil-release-Wirkstoffe (soil release polymer) sind Celluloseether, wie Carboxymethylcellulose, Methylcellulose, Hydroxyalkylcellulosen und Cellulosemischether, wie Methylhydroxyethylcellulose, Methylhydroxypropylcellulose und Methylcarboxymethylcellulose. Vorzugsweise werden Natriumcarboxymethylcellulose, Hydroxypropylmethylcellulose und deren Gemische und ggf. deren Gemische mit Methylcellulose eingesetzt. Zu den üblicherweise eingesetzten Soil-release-Wirkstoffen gehören Copolyester, die Dicarbonsäureeinheiten, Alkylenglykoleinheiten und Polyalkylenglykoleinheiten enthalten. Der Anteil an Vergrauungsinhibitoren und/oder soil-release-Wirkstoffen in erfindungsgemäßen Mitteln liegt im Allgemeinen nicht über 2 Gew.-% und beträgt vorzugsweise 0,5 bis 1,5 Gew.-%, besonders bevorzugt 0,5 bis 2 Gew.-%, bezogen auf das Gesamtgewicht des Mittels.

Als optische Aufheller für insbesondere Textilien aus Cellulosefasern (z.B. Baumwolle) können z.B. Derivate der Diaminostilbendisulfonsäure bzw. deren Alkalimetallsalze enthalten sein. Geeignet sind z.B. Salze der 4,4'-Bis(2-anilino-4-morpholino-1,3,5-triazin-6-yl-amino)-stilben-2,2'-disulfonsäure oder gleichartig aufgebaute Verbindungen, die anstelle der Morpholinogruppe eine Diethanolaminogruppe, eine Methylaminogruppe oder eine 2-Methoxyethylaminogruppe tragen. Weiterhin können Aufheller vom Typ des substituierten 4,4'-Distyryl-diphenyl anwesend sein, z.B. 4,4'-Bis-(4-chlor-3-sulfostyryl)-diphenyl. Auch Gemische von Aufhellern können verwendet werden. Für Polyamidfasern eignen sich besonders gut Aufheller vom Typ der 1,3-Diaryl-2-pyrazoline, z.B. 1-(p-Sulfoamoylphenyl)-3-(p-chlorphenyl)-2-pyrazolin sowie gleichartig aufgebaute Verbindungen. Der Gehalt des Mittels an optischen Aufhellern bzw. Aufhellergemischen liegt im Allgemeinen nicht über 1 Gew.-%, vorzugsweise 0,05 bis 0,5 Gew.-%, bezogen auf das Gesamtgewicht des Mittels. In einer bevorzugten Ausführungsform der Erfindung ist das Mittel frei von derartigen Wirkstoffen.

Zu den in den erfindungsgemäßen Mitteln einsetzbaren üblichen Schaumregulatoren gehören z.B. Polysiloxan-Kieselsäure-Gemische, wobei die darin enthaltene feinteilige Kieselsäure vorzugsweise silaniert oder anderweitig hydrophobiert ist. Die Polysiloxane können sowohl aus linearen Verbindungen wie auch aus vernetzten Polysiloxan-Harzen sowie aus deren Gemischen bestehen. Weitere Entschäumer sind Paraffinkohlenwasserstoffe, insbesondere Mikroparaffine und Paraffinwachse, deren Schmelzpunkt oberhalb 40°C liegt, gesättigte Fettsäuren bzw. Seifen mit insbesondere 20 bis 22 C-Atomen, z.B. Natriumbehenat, und Alkalisalze von Phosphorsäuremono- und/oder -dialkylestern, in denen die Alkylketten jeweils 12 bis 22 C-Atome aufweisen. Unter diesen wird bevorzugt Natriummonoalkylphosphat und/oder -dialkylphosphat mit C₁₆₋₁₈-Alkylgruppen eingesetzt. Der Anteil der Schaumregulatoren kann vorzugsweise 0,2 bis 2 Gew.-%, besonders bevorzugt nicht mehr als 1 Gew.-%, bezogen auf das Gesamtgewicht des Mittels, betragen.

Zur Einstellung des gewünschten pH-Werts können erfindungsgemäße Mittel system- und umweltverträgliche Säuren, insbesondere Citronensäure, Essigsäure, Weinsäure, Äpfelsäure, Milchsäure, Glykolsäure, Bernsteinsäure, Glutarsäure und/oder Adipinsäure, aber auch Mineralsäuren, insbesondere Schwefelsäure oder Alkalihydrogensulfate, oder Basen, insbesondere Ammonium- oder Alkalihydroxide, vorzugsweise Natriumhydroxid, enthalten. Derartige pH-Regulatoren sind in den erfindungsgemäßen Mitteln vorzugsweise nicht über 10 Gew.-%, insbesondere von 0,5 bis 6 Gew.-%, besonders bevorzugt von 0,3 bis 2 Gew.-%, bezogen auf das Gesamtgewicht des Mittels, enthalten.

Als einen weiteren Bestandteil können erfindungsgemäße Mittel ein organisches Lösungsmittel enthalten. Der Zusatz organischer Lösungsmittel wirkt sich vorteilhaft auf die Enzymstabilität und die Reinigungsleistung dieser Mittel aus. Bevorzugte organische Lösungsmittel stammen aus der Gruppe ein- oder mehrwertigen Alkohole, Alkanolamine oder Glykolether. Vorzugsweise werden die Lösungsmittel ausgewählt aus Ethanol, n- oder i-Propanol, Butanol, Glykol, Propandiol, Butandiol, Glycerin, Diglykol, Propyldiglykol, Butyldiglykol, Hexylenglycol, Ethylenglykolmethylether, Ethylenglykolethylether, Ethylenglykolpropylether, Etheylenglykolmono-n-butylether, Diethylenglykolmethylether, Di-ethylenglykolethylether, Propylenglykolmethylether, Propylenglykolethylether, Propylenglykolpropylether, Dipropylenglykolmethylether, Dipropylenglykolethylether, Methoxytriglykol, Ethoxytriglykol, Butoxytriglykol, 1-Butoxyethoxy-2-propanol, 3-Methyl-3-methoxybutanol, Propylenglykol-t-butylether sowie Mischungen dieser Lösungsmittel. Der Gewichtsanteil dieser organischen Lösungsmittel am Gesamtgewicht erfindungsgemäßer Mittel beträgt vorzugsweise 0,1 bis 10 Gew.-%, bevorzugt 0,2 bis 8,0 Gew.-% und weiter bevorzugt 0,5 bis 5,0 Gew.-%. Ein besonders bevorzugtes und in Bezug auf die Stabilisierung der Mittel besonders wirksames organisches Lösungsmittel ist das Glycerin sowie das 1,2-Propylenglykol. Flüssige Mittel umfassen vorzugsweise mindestens ein Polyol, vorzugsweise aus der Gruppe Glycerin und 1,2-Propylenglycol, bezogen auf das Gesamtgewicht des Mittels, vorzugsweise zu 0,1 bis 10 Gew.-%, bevorzugt 0,2 bis 8,0 Gew.-% und weiter bevorzugt 0,5 bis 5,0 Gew.-%. Weitere bevorzugte organische Lösungsmittel sind die organischen Amine und Alkanolamine. Erfindungsgemäße Mittel enthalten diese Amine vorzugsweise in Mengen von 0,1 bis 10 Gew.-%, bevorzugt von 0,2 bis 8,0 Gew.-% und weiter bevorzugt von 0,5 bis 5,0 Gew.-%, bezogen auf das Gesamtgewicht des Mittels. Ein besonders bevorzugtes Alkanolamin ist das Ethanolamin.

Erfindungsgemäße Mittel können ausschließlich eine erfindungsgemäße Protease enthalten. Alternativ können sie auch weitere hydrolytische Enzyme oder andere Enzyme in einer für die Wirksamkeit des Mittels zweckmäßigen Konzentration enthalten. Eine weitere Ausführungsform der Erfindung stellen somit Mittel dar, die ferner eines oder mehrere weitere Enzyme umfassen. Als weitere Enzyme bevorzugt einsetzbar sind alle Enzyme, die in dem erfindungsgemäßen Mittel eine katalytische Aktivität entfalten können, insbesondere eine Lipase, Amylase, Cellulase, Hemicellulase, Mannanase, Tannase, Xylanase, Xanthanase, Xyloglucanase, ß-Glucosidase, Pektinase, Carrageenase, Perhydrolase, Oxidase, Oxidoreduktase oder andere - von den erfindungsgemäßen Proteasen unterscheidbare - Protease, sowie deren Gemische. Weitere Enzyme sind in dem Mittel vorteilhafterweise jeweils in einer Menge von 1 x 10⁻⁸ bis 5 Gew.-% bezogen auf aktives Protein enthalten. Zunehmend bevorzugt ist jedes weitere Enzym in einer Menge von 1 x 10⁻⁷ bis 3 Gew.-%, von 0,00001 bis 1 Gew.-%, von 0,00005 bis 0,5 Gew.-%, von 0,0001 bis 0,1 Gew.-% und besonders bevorzugt von 0,0001 bis 0,05 Gew.-% in erfindungsgemäßen Mitteln enthalten, bezogen auf aktives Protein. Dem Fachmann sind Methoden zur Bestimmung der Enzymkonzentration bzw. des Aktivenzymproteingehaltes bekannt. Besonders bevorzugt zeigen die Enzyme synergistische Reinigungsleistungen gegenüber bestimmten Anschmutzungen oder Flecken, d.h. die in dem Mittel enthaltenen Enzyme unterstützen sich in ihrer Reinigungsleistung gegenseitig. Ganz besonders bevorzugt liegt ein solcher Synergismus vor zwischen der erfindungsgemäß enthaltenen Protease und einem weiteren Enzym eines erfindungsgemäßen Mittels. Synergistische Effekte können nicht nur zwischen verschiedenen Enzymen, sondern auch zwischen einem oder mehreren Enzymen und weiteren Inhaltsstoffen des erfindungsgemäßen Mittels auftreten.

Erfindungsgemäß bevorzugte Maschinengeschirrspülmittel weisen mindestens eine Protease und mindestens eine Amylase auf.

Erfindungsgemäß bevorzugte Textilwaschmittel, insbesondere flüssige Textilwaschmittel, weisen mindestens eine Protease und mindestens eine Amylase auf. In einer weiteren bevorzugten Ausführungsform der Erfindung weisen Textilwaschmittel, insbesondere flüssige Textilwaschmittel, mindestens eine Protease und mindestens eine Cellulase auf. In einer weiteren bevorzugten Ausführungsform weisen Textilwaschmittel, insbesondere flüssige Textilwaschmittel, mindestens eine Protease und mindestens eine Lipase auf. In einer weiteren bevorzugten Ausführungsform weisen Textilwaschmittel, insbesondere flüssige Textilwaschmittel, mindestens eine Protease, mindestens eine Amylase und mindestens eine Lipase auf. In einer weiteren bevorzugten Ausführungsform weisen Textilwaschmittel, insbesondere flüssige Textilwaschmittel, mindestens eine Protease, mindestens eine Amylase und mindestens eine Cellulase auf. In einer weiteren bevorzugten Ausführungsform weisen Textilwaschmittel, insbesondere flüssige Textilwaschmittel, mindestens eine Protease, mindestens eine Amylase, mindestens eine Cellulase und mindestens eine Lipase auf. Besonders bevorzugt sind Textilwaschmittel, insbesondere flüssige Textilwaschmittel, welche 3 bis 10 verschiedene Enzyme aufweisen, wobei Textilwaschmittel, welche 3 bis 10 unterschiedliche Enzymarten aufweisen, im Hinblick auf die Reinigungsleistung gegenüber einem sehr großen Fleckenspektrum von besonderer Bevorzugung sein können.

In bevorzugten Ausführungsformen enthält ein erfindungsgemäßes Mittel mindestens ein Enzym und zunehmend bevorzugt mindestens zwei, drei, vier oder fünf Enzyme, die vorzugsweise aus der aus Amylasen, Proteasen, Lipasen, Cellulasen, Mannanasen und Mischungen davon ausgewählt sind, insgesamt in einer Menge von 0,01 bis 10 Gew.-%, bevorzugt von 0,1 bis 8 Gew.-%, besonders bevorzugt von 0,2 bis 6 Gew.-%, bezogen auf aktives Protein und das Gesamtgewicht des Mittels.

Beispiele für Proteasen sind die Subtilisine BPN' aus *Bacillus amyloliquefaciens* und Carlsberg aus *Bacillus licheniformis,* die Protease PB92, die Subtilisine 147 und 309, die Protease aus *Bacillus lentus*, Subtilisin DY und die den Subtilasen, nicht mehr jedoch den Subtilisinen im engeren Sinne zuzuordnenden Enzyme Thermitase, Proteinase K und die Proteasen TW3 und TW7. Subtilisin Carlsberg ist in weiterentwickelter Form unter dem Handelsnamen Alcalase^{®} von dem Unternehmen Novozymes erhältlich. Die Subtilisine 147 und 309 werden unter den Handelsnamen Esperase^{®} bzw. Savinase^{®} von dem Unternehmen Novozymes vertrieben. Von der Protease aus *Bacillus lentus* DSM 5483 leiten sich Protease-Varianten ab, beschrieben in z.B. WO 95/23221, WO 92/21760 WO 2013/060621 und EP 3660151. Weitere brauchbare Proteasen sind z.B. die unter den Handelsnamen Durazym^{®}, Relase^{®}, Everlase^{®}, Nafizym^{®}, Natalase^{®}, Kannase^{®}, Progress Uno 101L^{®} und Ovozyme^{®} von dem Unternehmen Novozymes, die unter den Handelsnamen, Purafect^{®}, Purafect^{®} OxP, Purafect^{®} Prime, Excellase^{®}, Properase^{®}, Preferenz P100^{®} und Preferenz P300^{®} von dem Unternehmen Danisco/DuPont, das unter dem Handelsnamen Lavergy pro 104 LS^{®} von dem Unternehmen BASF, das unter dem Handelsnamen Protosol^{®} von dem Unternehmen Advanced Biochemicals Ltd., das unter dem Handelsnamen Wuxi^{®} von dem Unternehmen Wuxi Snyder Bioproducts Ltd., die unter den Handelsnamen Proleather^{®} und Protease P^{®} von dem Unternehmen Amano Pharmaceuticals Ltd., und das unter der Bezeichnung Proteinase K-16 von dem Unternehmen Kao Corp. erhältlichen Enzyme. Besonders bevorzugt eingesetzt werden auch die Proteasen aus *Bacillus gibsonii* und *Bacillus pumilus,* die offenbart sind in WO 2008/086916, WO 2007/131656, WO 2017/215925, WO 2021/175696 und WO 2021/175697. Weitere verwendbare Proteasen sind diejenigen, die in den Mikroorganismen *Stenotrophomonas maltophilia,* insbesondere *Stenotrophomonas maltophilia* K279a, *Bacillus intermedius* sowie *Bacillus sphaericus* natürlicherweise vorhanden sind.

Beispiele für Amylasen sind die α-Amylasen aus *Bacillus licheniformis, Bacillus amyloliquefaciens* oder *Bacillus stearothermophilus* sowie insbesondere auch deren für den Einsatz in Wasch- oder Reinigungsmitteln verbesserte Weiterentwicklungen. Das Enzym aus *Bacillus licheniformis* ist von dem Unternehmen Novozymes unter dem Namen Termamyl^{®} und von dem Unternehmen Danisco/DuPont unter dem Namen Purastar^{®} ST erhältlich. Weiterentwicklungsprodukte dieser α-Amylase sind unter den Handelsnamen Duramyl^{®} und Termamyl^{®} ultra (beide von Novozymes), Purastar^{®} OxAm (Danisco/DuPont) und Keistase^{®} (Daiwa Seiko Inc.) erhältlich. Die α-Amylase von *Bacillus amyloliquefaciens* wird von dem Unternehmen Novozymes unter dem Namen BAN^{®} vertrieben, und abgeleitete Varianten von der α-Amylase aus *Bacillus stearothermophilus* unter den Namen BSG^{®} und Novamyl^{®}, ebenfalls von dem Unternehmen Novozymes. Des Weiteren sind für diesen Zweck die α-Amylase aus *Bacillus sp.* A 7-7 (DSM 12368) und die Cyclodextrin-Glucanotransferase (CGTase) aus *Bacillus agaradherens* (DSM 9948) hervorzuheben. Ebenso sind Fusionsprodukte aller genannten Moleküle einsetzbar. Darüber hinaus sind die unter den Handelsnamen Fungamyl^{®} von dem Unternehmen Novozymes erhältlichen Weiterentwicklungen der α-Amylase aus *Aspergillus niger* und A. *oryzae* geeignet. Weitere vorteilhaft einsetzbare Handelsprodukte sind z.B. die Amylase-LT^{®} und Stainzyme^{®} oder Stainzyme^{®} ultra bzw. Stainzyme^{®} plus sowie Amplify^{™} 12L, Amplify Prime^{™} 100L oder Amplify Prime^{™} 120L, letztere ebenfalls von dem Unternehmen Novozymes, sowie die PREFERENZ S^{®} Serie von dem Unternehmen Danisco/DuPont, umfassend z.B. PREFERENZ S100^{®}, PREFERENZ S1000^{®} oder PREFERENZ S210^{®}. Auch durch Punktmutationen erhältliche Varianten dieser Enzyme können erfindungsgemäß eingesetzt werden.

Geeignete Cellulasen umfassen solche bakterieller oder pilzlicher Herkunft. Chemisch modifizierte oder proteintechnisch veränderte Mutanten sind eingeschlossen. Geeignete Cellulasen sind Cellulasen aus den Gattungen *Bacillus, Pseudomonas, Humicola, Fusarium, Thielavia, Acremonium,* z. B. die Pilzcellulasen aus *Humicola insolens, Myceliophthora thermophila* und *Fusarium oxysporum,* die in US 4435307, US 5648263, US 5691178, US 5776757 und WO 89/09259 offenbart sind. Besonders geeignete Cellulasen sind die alkalischen oder neutralen Cellulasen mit farbpflegenden Eigenschaften. Beispiele für solche Cellulasen sind Cellulasen, die in EP 0495257, EP 0531372, WO 96/11262, WO 96/29397 und WO 98/08940 beschrieben sind. Beispiele für Cellulasen mit Endo-1,4-Glucanase-Aktivität (EC 3.2.1.4) sind in WO 2002/099091 beschrieben, z.B. solche mit einer Sequenz von mindestens 97% Identität zu der Aminosäuresequenz der Positionen 1 bis 773 von SEQ ID NO:2 der WO 2002/099091. Ein weiteres Beispiel kann eine GH44-Xyloglucanase umfassen, z.B. ein Xyloglucanase-Enzym mit einer Sequenz von mindestens 60% Identität zu den Positionen 40 bis 559 der SEQ ID NO:2 der WO 2001/062903. Zu den kommerziell verfügbaren Cellulasen gehören Celluzyme'", Carezyme^{™}, Carezyme Premium^{™}, Celluclean^{™} (z.B. Celluclean^{™} 5000L und Celluclean^{™} 4000T), Celluclean Classic^{™}, Cellusoft^{™}, Endolase^{®}, Renozyme^{®} und Whitezyme^{™} (Novozymes A/S), Clazinase^{™} und Puradax HA^{™} (Genencor International Inc.), KAC-500(B)^{™} (Kao Corporation), Revitalenz^{™} 1000, Revitalenz^{™} 2000 und Revitalenz^{™} 3000 (DuPont), sowie die Ecostone^{®} und Biotouch^{®} Serie (AB Enzymes).

Geeignete Lipasen und Cutinasen sind solche bakteriellen oder pilzlichen Ursprungs. Chemisch modifizierte oder durch Proteinengineering erzeugte mutierte Enzyme sind eingeschlossen. Beispiele sind Lipase aus *Thermomyces,* z.B. aus *T. lanuginosus* (früher *Humicola lanuginosa* genannt), wie in EP 0258068 und EP 0305216 beschrieben, Cutinase aus *Humicola,* z.B. *H. insolens* (WO 96/13580), Lipase aus Stämmen von *Pseudomonas* (einige davon jetzt umbenannt in *Burkholderia*), z.B. *P*. *alcaligenes* oder *P. pseudoalcaligenes, P. cepacia, P. sp.* Stamm SD705, *P. wisconsinensis,* Streptomyces-Lipasen vom GDSL-Typ, Cutinase aus *Magnaporthe grisea*, Cutinase aus *Pseudomonas mendocina*, Lipase aus *Thermobifida fusca*, Lipase aus *Geobacillus stearothermophilus,* Lipase aus *Bacillus subtilis* und Lipase aus *Streptomyces griseus* und S. *pristinaespiralis.* Zu bevorzugten Lipasen gehören z.B. die ursprünglich aus *Humicola lanuginosa* (*Thermomyces lanuginosus*) erhältlichen bzw. daraus weiterentwickelten Lipasen, insbesondere solche mit einem oder mehreren der folgenden Aminosäureaustausche ausgehend von der genannten Lipase in den Positionen D96L, T213R und/oder N233R, besonders bevorzugt T213R und N233R. Zu den bevorzugten kommerziellen Lipaseprodukten gehören Lipolase^{™}, Lipex^{™}, Lipolex^{™} und Lipoclean^{™} (Novozymes A/S), Lumafast (Genencor/DuPont), Lipomax (Gist-Brocades) und Biotouch LL100 (AB Enzymes).

Geeignete Mannanasen sind z.B. die *Bacillus subtilis* Endo-β-Mannanase, *Bacillus sp.* I633 Endo-β-Mannanase, *Bacillus sp.* AAI12 Endo-β-Mannanase, *Bacillus sp.* AA349 Endo-β-Mannanase, *Bacillus agaradhaerens* NCIMB 40482 Endo-β-Mannanase, *Bacillus halodurans* Endo-β-Mannanase, *Bacillus clausii* Endo-β-Mannanase, *Bacillus licheniformis* Endo-β-Mannanase, *Humicola insolens* Endo-β-Mannanase und *Caldocellulosiruptor sp.* Endo-β-Mannanase (z.B. US 6060299, WO 99/64573, US 6566114 und WO 99/64619).

Für Wasch- und Reinigungsmittel geeignete Pektatlyasen sind z.B. in WO 2003/095638 oder WO 2015/121133 beschrieben. Beispiele für geeignete pektinolytische Enzyme sind zudem die unter den Handelsbezeichnungen Gamanase^{®}, Pektinex AR^{®}, X-Pect^{®} oder Pectaway^{®} von dem Unternehmen Novozymes, unter den Handelsbezeichnungen Rohapect UF^{®}, Rohapect TPL^{®}, Rohapect PTE100^{®}, Rohapect MPE^{®}, Rohapect MA plus HC, Rohapect DA12L^{®}, Rohapect 10L^{®}, Rohapect B1L^{®} von dem Unternehmen AB Enzymes und unter der Handelsbezeichnung Pyrolase^{®} von dem Unternehmen Diversa Corp. erhältlichen Enzyme und Enzym-Zubereitungen.

Zur Erhöhung der bleichenden Wirkung können erfindungsgemäß Oxidoreduktasen, z.B. Oxidasen, Oxygenasen, Katalasen, Peroxidasen, wie Halo-, Chloro-, Bromo-, Lignin-, Glucose- oder Manganperoxidasen, Dioxygenasen oder Laccasen (Phenoloxidasen, Polyphenoloxidasen) eingesetzt werden. Vorteilhafterweise werden zusätzlich vorzugsweise organische, besonders bevorzugt aromatische, mit den Enzymen wechselwirkende Verbindungen zugegeben, um die Aktivität der betreffenden Oxidoreduktasen zu verstärken (Enhancer) oder um bei stark unterschiedlichen Redoxpotentialen zwischen den oxidierenden Enzymen und den Anschmutzungen den Elektronenfluss zu gewährleisten (Mediatoren).

In den hierin beschriebenen Reinigungsmitteln können die einzusetzenden Enzyme ferner zusammen mit Begleitstoffen, etwa aus der Fermentation, konfektioniert sein. In flüssigen Formulierungen werden die Enzyme bevorzugt als Enzymflüssigformulierung(en) eingesetzt.

Die Enzyme werden in der Regel nicht in Form des reinen Proteins, sondern vielmehr in Form stabilisierter, lager- und transportfähiger Zubereitungen bereitgestellt. Zu diesen vorkonfektionierten Zubereitungen zählen z.B. die durch Granulation, Extrusion oder Lyophilisierung erhaltenen festen Präparationen oder, insbesondere bei flüssigen oder gelförmigen Mitteln, Lösungen der Enzyme, vorteilhafterweise möglichst konzentriert, wasserarm und/oder mit Stabilisatoren oder weiteren Hilfsmitteln versetzt.

Alternativ können die Enzyme sowohl für die feste als auch für die flüssige Darreichungsform verkapselt werden, z.B. durch Sprühtrocknung oder Extrusion der Enzymlösung zusammen mit einem vorzugsweise natürlichen Polymer oder in Form von Kapseln, z.B. solchen, bei denen die Enzyme wie in einem erstarrten Gel eingeschlossen sind oder in solchen vom Kern-Schale-Typ, bei dem ein enzymhaltiger Kern mit einer Wasser-, Luft- und/oder Chemikalien-undurchlässigen Schutzschicht überzogen ist. In aufgelagerten Schichten können zusätzlich weitere Wirkstoffe, z.B. Stabilisatoren, Emulgatoren, Pigmente, Bleich- oder Farbstoffe aufgebracht werden. Derartige Kapseln werden nach an sich bekannten Methoden, z.B. durch Schüttel- oder Rollgranulation oder in Fluid-bed-Prozessen aufgebracht. Vorteilhafterweise sind derartige Granulate, z.B. durch Aufbringen polymerer Filmbildner, staubarm und aufgrund der Beschichtung lagerstabil.

Weiterhin ist es möglich, zwei oder mehrere Enzyme zusammen zu konfektionieren, so dass ein einzelnes Granulat mehrere Enzymaktivitäten aufweist.

Die Enzyme können auch in wasserlösliche Filme, wie sie z.B. bei der Konfektionierung von Wasch- und Reinigungsmitteln in Einheitsdosisform verwendet werden, eingebracht werden. Ein derartiger Film ermöglicht die Freisetzung der Enzyme nach Kontakt mit Wasser. Wie hierin verwendet, bezieht sich "wasserlöslich" auf eine Filmstruktur, die vorzugsweise vollständig wasserlöslich ist. Bevorzugt besteht ein solcher Film aus (vollständig oder teilweise hydrolysiertem) Polyvinylalkohol (PVA).

Erfindungsgemäße Mittel können einen oder mehrere reversible Enyzminhibitor(en)/- stabilisator(en) umfassen. Erfindungsgemäße Mittel können den/die reversiblen Enyzminhibitor(en)/- stabilisator(en) in einer Konzentration von 0,1 bis 2 Gew.-%, vorzugsweise 0,3 bis 1,5 Gew.-%, enthalten, bezogen auf das Gesamtgewicht des Mittels. Falls mehrere Inhibitoren/Stabilisatoren enthalten sind, beziehen sich diese Angaben auf die Gesamtkonzentration. Diese können insbesondere ausgewählt sein aus der aus Polyolen, wie Glycerin oder 1,2-Ethylenglycol, Benzamidinhydrochlorid, Borax, Borsäuren, Boronsäuren oder deren Salze oder Ester oder Derivate, insbesondere Phenylboronsäurederivate oder 4-Formylphenylboronsäure (4-FPBA), Antioxidantien, speziellen Peptidverbindungen und Kombinationen davon bestehenden Gruppe.

In bevorzugten Ausführungsformen ist das erfindungsgemäße Mittel im Wesentlichen frei von borhaltigen Verbindungen. "Im Wesentlichen frei von borhaltigen Verbindungen" bedeutet in diesem Zusammenhang, dass die erfindungsgemäßen Mittel weniger als 2 Gew.-%, vorzugsweise weniger als 1 Gew.-%, weiter bevorzugt weniger als 0,5 Gew.-% und besonders bevorzugt weniger als 0,1 Gew.-%, borhaltige Verbindungen, bezogen auf das Gesamtgewicht des Mittels, enthalten. In besonders bevorzugten Ausführungsformen sind die erfindungsgemäßen Mittel frei von borhaltigen Verbindungen, d.h. sie enthalten insbesondere keine Borsäure und/oder Phenylboronsäurederivate.

Ein weiterer Erfindungsgegenstand ist ein Verfahren zur Reinigung von Textilien und/oder harten Oberflächen, insbesondere Geschirr, das dadurch gekennzeichnet ist, dass in mindestens einem Verfahrensschritt ein erfindungsgemäßes Mittel angewendet wird. In verschiedenen Ausführungsformen zeichnet sich das beschriebene Verfahren dadurch aus, dass die Protease bei einer Temperatur von etwa 0°C bis etwa 100°C, bevorzugt etwa 20°C bis etwa 60°C, weiter bevorzugt etwa 20°C bis etwa 40°C und besonders bevorzugt etwa 20°C eingesetzt wird.

Hierunter fallen sowohl manuelle als auch maschinelle Verfahren, wobei maschinelle Verfahren aufgrund ihrer präziseren Steuerbarkeit, was z.B. die eingesetzten Mengen und Einwirkzeiten angeht, bevorzugt sind. Verfahren zur Reinigung von Textilien zeichnen sich im Allgemeinen dadurch aus, dass in mehreren Verfahrensschritten verschiedene reinigungsaktive Substanzen auf das Reinigungsgut aufgebracht und nach der Einwirkzeit abgewaschen werden, oder dass das Reinigungsgut in sonstiger Weise mit einem Waschmittel oder einer Lösung oder Verdünnung dieses Mittels behandelt wird.

Da erfindungsgemäße Proteasen natürlicherweise bereits eine hydrolytische Aktivität besitzen und diese auch in Medien entfalten, die sonst keine Reinigungskraft besitzen wie z.B. in bloßem Puffer, kann ein einzelner und/oder der einzige Schritt eines solchen Verfahrens darin bestehen, dass als einzige reinigungsaktive Komponente eine erfindungsgemäße Protease mit der Anschmutzung in Kontakt gebracht wird, bevorzugt in einer Pufferlösung oder in Wasser. Dies stellt eine weitere Ausführungsform dieses Erfindungsgegenstandes dar.

Alternative Ausführungsformen dieses Erfindungsgegenstandes stellen auch Verfahren zur Behandlung von Textilrohstoffen oder zur Textilpflege dar, bei denen in wenigstens einem Verfahrensschritt eine erfindungsgemäße Protease aktiv wird. Hierunter sind Verfahren für Textilrohstoffe, Fasern oder Textilien mit natürlichen Bestandteilen bevorzugt, und ganz besonders für solche mit Wolle oder Seide.

Ein weiterer Gegenstand der Erfindung betrifft ein Verfahren zur Entfernung von proteasesensitiven Anschmutzungen, insbesondere ei(gelb)-, milch-, fleisch-, blut- und anderen proteinhaltigen Anschmutzungen, bevorzugt ei(gelb)- und/oder milchhaltigen Anschmutzungen, von Textilien und/oder harten Oberflächen, insbesondere Geschirr, wobei in mindestens einem Verfahrensschritt ein Mittel, das eine erfindungsgemäße Protease, wie hierin beschrieben, enthält und/oder eine erfindungsgemäße Protease, wie hierin beschrieben, angewendet wird.

Ein weiterer Gegenstand der Erfindung betrifft ein Verfahren zur Verbesserung der Reinigungsleistung eines Wasch- und Reinigungsmittels, insbesondere flüssigen Textilwaschmittels, an mindestens einer proteasesensitiven Anschmutzung, insbesondere ausgewählt aus ei(gelb)-, milch-, fleisch-, blut- und anderen proteinhaltigen Anschmutzungen, bevorzugt ei(gelb)- und/oder milchhaltigen Anschmutzungen, wobei das Mittel eine erfindungsgemäße Protease, wie hierin beschrieben, enthält.

Schließlich erfasst die Erfindung auch die Verwendung erfindungsgemäßer Proteasen, wie hierin beschrieben, in Wasch- oder Reinigungsmitteln, insbesondere flüssigen Textilwaschmitteln, zur (verbesserten) Entfernung von proteasesensitiven Anschmutzungen, die vorzugsweise aus der aus blut-, ei(gelb)-, milch-, fleisch- und anderen proteinhaltigen Anschmutzungen, bevorzugt ei(gelb)-und/oder milchhaltigen Anschmutzungen, bestehenden Gruppe ausgewählt sind, von Textilien und/oder harten Oberflächen, insbesondere Geschirr.

In einer weiteren bevorzugten Ausführungsform betrifft die Erfindung die Verwendung einer hierin beschriebenen erfindungsgemäßen Protease in einem Wasch- oder Reinigungsmittel, insbesondere flüssiges Textilwaschmittel, zur Verbesserung der Reinigungsleistung eines solchen proteasehaltigen Wasch- oder Reinigungsmittels, insbesondere eines flüssigen Textilwaschmittels, an mindestens einer proteasesensitiven Anschmutzung, die vorzugsweise aus der aus blut-, ei(gelb)-, milch-, fleisch- und anderen proteinhaltigen Anschmutzungen, bevorzugt ei(gelb)- und/oder milchhaltigen Anschmutzungen, bestehenden Gruppe ausgewählt ist, wobei die Verbesserung der Reinigungsleistung eines Mittels mit erfindungsgemäßer Protease gegenüber einem Mittel, welches eine Referenzprotease (insbesondere Wildtyp-Protease gemäß SEQ ID NO:1) enthält, wie in Beispiel 2 beschrieben, bestimmt wird, insbesondere in einem Temperaturbereich von etwa 20°C bis etwa 40°C, bevorzugt 20°C.

In einer weiteren bevorzugten Ausführungsform betrifft die Erfindung die Verwendung einer hierin beschriebenen erfindungsgemäßen Protease in einem Wasch- oder Reinigungsmittel, insbesondere flüssigen Textilwaschmittel, zur Verbesserung der Reinigungsleistung eines solchen proteasehaltigen Wasch- oder Reinigungsmittels, insbesondere eines flüssigen Textilwaschmittels, an mindestens einer proteasesensitiven Anschmutzung, die vorzugsweise aus der aus blut-, ei(gelb)-, milch-, fleisch- und anderen proteinhaltigen Anschmutzungen, bevorzugt ei(gelb)- und/oder milchhaltigen Anschmutzungen, bestehenden Gruppe ausgewählt ist, wobei die Verbesserung der Reinigungsleistung eines Mittels mit erfindungsgemäßer Protease gegenüber einem Mittel, welches eine Referenzprotease (insbesondere Wildtyp-Protease gemäß SEQ ID NO:1) enthält, wie in Beispiel 2 beschrieben, bestimmt wird, insbesondere in einem Temperaturbereich von etwa 20°C bis etwa 40°C, bevorzugt etwa 20°C, wobei die Protease eine Protease ist, proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease bezogen auf die Nummerierung gemäß SEQ ID NO:1 an mindestens einer der Positionen, die den Positionen 97, 99, 127 und 211 entsprechen, mindestens eine Aminosäuresubstitution, die aus der aus N97E, R99E, R99I, R99N, R99V, R99T, D127E und M211C bestehenden Gruppe ausgewählt ist, aufweist.

Alle Sachverhalte, Gegenstände und Ausführungsformen, die für die erfindungsgemäße Protease und sie enthaltende Mittel beschrieben sind, sind auch auf diese Erfindungsgegenstände anwendbar. Daher wird an dieser Stelle ausdrücklich auf die Offenbarung an entsprechender Stelle verwiesen mit dem Hinweis, dass diese Offenbarung auch für die vorstehenden erfindungsgemäßen Verfahren und Verwendungen gilt.

### BEISPIELE

**Tabelle1: Verwendete Reiniqunqsmittelmatrix**

| **Chemischer Name** | **% Aktivsubstanz im Rohstoff** | **Gew.-% Aktivsubstanz in Formulierung** |
|---|---|---|
| Wasser | 100% | Ad 100% |
| Zitronensäure | 100% | 3-5% |
| FAEOS | 70% | 5-8% |
| C12-18 Fettalkoholethoxylat, 7EO | 100% | 8-11 % |
| Alkylbenzolsulfonsäure | 96% | 10-14% |
| C₁₂₋₁₄-Fettsäure | 30% | 2-4% |
| Monoethanolamin | 100% | 6-8% |
| NaOH | 50% | 2% |
| Glycerin | 99,5% | 3% |
| 1,2-Propandiol | 100% | 8% |
| HEDP | 60% | 0,5-2% |
| Weiteres (Antischaum, SRP, Enzyme, Duftstoffe, DTI) | t.q. | Minors |
| pH 8,2-8,4 | | |

**Tabelle 2: Verwendete Proteasen:**

| | **Aminosäuresubstitution(en) gegenüber SEQ ID NO:1** |
|---|---|
| Protease 1 (P1) | SEQ ID NO:1 (Wildtyp) |
| Protease 2 (P2) | D127E |
| Protease 3 (P3) | M211C |
| Protease 4 (P4) | R99E |
| Protease 5 (P5) | R99I |
| Protease 6 (P6) | N97E |
| Protease 7 (P7) | R99N |
| Protease 8 (P8) | R99V |
| Protease 9 (P9) | R99T |
| Protease 10 (P10) | D127E-M211C |
| Protease 11 (P11) | N97E-R99I |

### Beispiel 1: Bestimmung der Proteaseaktivität

Die Aktivität der Protease wird durch die Freisetzung des Chromophors para-Nitroanilin aus dem Substrat Succinyl Alanin-Alanin-Prolin-Phenylalanin-para-Nitroanilid (AAPF-pNA; Bachem L-1400) bestimmt. Die Freisetzung des pNA verursacht eine Zunahme der Extinktion bei 410 nm, deren zeitlicher Verlauf ein Maß für die enzymatische Aktivität ist.

Die Messung erfolgte bei einer Temperatur von 25°C, bei pH 8,6 und einer Wellenlänge von 410 nm. Die Messzeit betrug 5 min bei einem Messintervall von 20 bis 60 Sekunden.

Messansatz:
10 µL AAPF-Lösung (70 mg/mL)
1000 µL Tris/HCl (0,1 M; pH 8,6 mit 0,1% Brij 35)
10 µL verdünnte Proteaselösung
Kinetik erstellt über 5 min bei 25°C (410 nm)

### Beispiel 2: Bestimmung der Reinigungsleistung

### Minispültest

Die Reinigungsleistung wurde in Miniwaschtests mit *Bacillus subtilis* Kulturüberständen, welche die exprimierten Protease-Varianten enthalten, bestimmt. Die Überstände wurde aktivitätsgleich zum Benchmark (= Wildtyp gemäß SEQ ID NO:1) mit einer marktüblichen Konzentration für Proteasen in einer Waschmittelformulierung A (gemäß Tabelle 1) (28,6 mg/Job aktives Enzymprotein in der Waschlauge) eingesetzt.
Bedingungen: 20°C bzw. 40°C, 16°dH Wasser, 1h Waschzeit
Dosierung in Waschlauge: 3,17 g/L

Anschmutzungen:
1. CS38 (Eigelb/Pigment (eingetrocknet))
2. 10N (Ganzei/Ruß)
3. C05 (Blut/Milch/Tusche)
4. H-MR-B (Milch)
5. PC10 (Milch/Öl)

Ausgestanzte Gewebe mit waschrelevanter Anschmutzung (Durchmesser = 10 mm) wurden in Mikrotiterplatten vorgelegt, die Waschlauge enthaltend ein Mittel gemäß Tabelle 1 wurde auf pH = 8,2 eingestellt und auf 20°C bzw. 40°C vortemperiert, Endkonzentration 3,17 g/L, Lauge und Enzym wurden auf die Anschmutzung gegeben, für 1 h bei 20°C bzw. 40°C und 600 rpm inkubiert, anschließend wurde die Lauge entfernt und die Anschmutzung mehrmals mit klarem Wasser gespült, trocknen gelassen und mit einem Farbmessgerät (MACH 5, Multi area color-measurement, CFTBV.nl) wurde die Helligkeit bestimmt. Je heller das Gewebe, desto besser die Reinigungsleistung. Gemessen wird hier der Y-Wert = Helligkeit, je höher desto heller. Alle gemessenen Y-Werte wurden durch die Leistung der Lauge allein (ohne Protease) korrigiert (Y_{Variante} - Y_{Blank} = ΔY_{Variante}). Für jede Anschmutzung wurde für jede Protease ΔY_{Variante} ermittelt und alle ΔY_{Variante}-Werte der fünf Anschmutzungen pro Variante wurden summiert, um ΣY_{Variante} zu bestimmen. Um die Reinigungsleistung der erfindungsgemäßen Varianten mit dem Benchmark (= Protease P1) zu vergleichen, wurde ΔY_{P1} bzw. ΣY_{P1} (für jede Anschmutzung) auf 100% normiert und die relative Reinigungsleistung der erfindungsgemäßen Varianten berechnet. Eine ≥10%ige Erhöhung der Reinigungsleistung wird als eine signifikante Leistungssteigerung angesehen.

Die Ergebnisse sind in Tabelle 3 zusammengefasst.

**Tabelle 3: Reinigungsleistung bei 20°C und 40°C:**

| | ΔY_{Variante} (Summe) 20°C | ΔY_{Variante} (%) 20°C | ΔY_{Variante} (Summe) 40°C | ΔY_{Variante} (%) 40°C |
|---|---|---|---|---|
| P1 | 48 | 100% | 77 | 100% |
| P2 | 56 | 116,5% | 74 | 95,4% |
| P3 | 54 | 112,3% | 83 | 107,0% |
| P4 | 65 | 135,2% | 92 | 118,7% |
| P5 | 56 | 116,5% | 81 | 104,4 % |
| P6 | 60 | 124,8% | 87 | 112,2% |
| P7 | 58 | 120,6% | 79 | 101,9% |
| P8 | 60 | 124,8% | 85 | 109,6% |
| P9 | 56 | 116,5% | 82 | 105,8% |
| P10 | 55 | 114,4% | 76 | 98,0% |
| P11 | 64 | 133,1% | 91 | 117,4% |

Die erfindungsgemäßen Proteasen (P2 bis P11) zeigen eine vergleichbare bzw. signifikant verbesserte Reinigungsleistung an verschiedenen proteasesensitiven Anschmutzungen im Vergleich zur Referenzprotease (Wildtyp-Protease gemäß SEQ ID NO:1), die verbesserte Reinigungsleistung zeigt sich insbesondere bei einer vergleichsweise niedrigen Temperatur von 20°C. Daraus folgt, dass erfindungsgemäße Proteasen nicht nur für Waschvorgänge bei üblicherweise verwendeten Temperaturen von etwa 40°C verwendet werden können, sondern auch bei niedrigen Temperaturen, d.h. z.B. Schonwaschgängen bei 20°C (oder Raumtemperatur) verwendet werden können und eine gegenüber der Referenzprotease signifikant gesteigerte Waschleistung bei niedrigen Temperaturen zeigen.

### Beispiel 3: Beispielformulierungen

Die erfindungsgemäßen Proteasen können in verschiedenen Wasch- und Reinigungsmittelzusammensetzungen eingesetzt werden und ihren Effekt verwirklichen.

**Tabelle 4: Flüssigqwaschmittel**

| **Chemischer Name** | **Gew.-% Aktivsubstanz in der Formulierung** | | | | | |
|---|---|---|---|---|---|---|
| | **A** | **B** | **C** | **D** | **E** | **F** |
| Wasser demin. | Rest | Rest | Rest | Rest | Rest | Rest |
| LAS | 5,5 | 20 | 15,0 | 5,5 | 21,7 | 23,5 |
| FAEOS | 7,0 | | 5,0 | | | |
| Palmkernölsäure | 3,0 | 8,0 | | | 7,0 | 7,4 |
| FAEO | 5,5 | | 8,0 | | | |
| C_{13/15} Oxoalkohol, 8EO | | 25 | | | | |
| C₁₂₋₁₈ Fettalkoholethoxylat, 7EO | | | | | 22,4 | 23,4 |
| Alkylpolyglykosid | | | 4,0 | | | |
| Nichtionische Tenside | | | | 3,1 | | |
| Seife | | | 1,0 | 0,5 | | |
| HEDP | 0,5 | | | | | |
| DTPMPA 7Na | | 1,0 | 1,0 | 0,2 | 0,5 | 1,7 |
| Zitronensäure | 2,5 | | 3,0 | 0,23 | | |
| NaOH | 3,0 | | | 0,7 | | |
| Glycerin | 3,0 | 5,0 | | 0,5 | 9,4 | 10,2 |
| Ethanol | 1,5 | 3,0 | | | | 3,2 |
| 1,2-Propandiol | | 10,0 | 12,0 | | 5,0 | 5,6 |
| Monoethanolamin | | 6,0 | 7,0 | | 6,0 | 6,1 |
| Borsäure | 1,0 | | 1,0 | 0,5 | | |
| Polyalkoxyliertes Alkanolamin | | | | | 4,5 | |
| Ethoxyliertes Polyethylenimin | | | | | 4,5 | 3,0 |
| Protease | 6 HPE/ml | 6 HPE/ml | 6 HPE/ml | 6 HPE/ml | 6 HPE/ml | 6 HPE/ml |
| Duftstoff(e) | 0,5 | 0,5 | 0,4 | 0,3 | 0,4 | 0,25 |
| DTI, SRP, weitere Enzyme, Entschäumer, u.a. | minors | minors | minors | minors | minors | minors |

**Tabelle 5: Feste Waschmittel**

| **Chemischer Name** | **Gew.-% Aktivsubstanz in der Formulierung** | | |
|---|---|---|---|
| | **A** | **B** | **C** |
| LAS | 12,2 | 12,0 | 10,1 |
| Na-Fettalkoholsulfat, C₁₂₋₁₈ | 4,2 | | |
| Fettalkohol, C₁₂₋₁₈, 7 EO | 4,1 | 2,3 | 1,5 |
| Seifen | 0,4 | | |
| Citrat | 2,0 | | |
| Natriumcarbonat | 2,4 | 17,9 | 25,1 |
| Builder | 23,0 | 7,0 | 7,6 |
| Phosphonat | 1,2 | 1,1 | 1,2 |
| Polyacrylat | 0,12 | 2,8 | 3,0 |
| Carboxymethylcellulose | 2,3 | 2,0 | 1,1 |
| 2Na2 Carbonat 3 H₂O₂ | 18,5 | 15,8 | |
| TAED | 10,9 | 3,5 | |
| Duftstoff(e) | 0,5 | 0,3 | 0,4 |
| Protease | 6 HPE/ml | 6 HPE/ml | 6 HPE/ml |
| Natriumsulfat, Schauminhibitor, optischer Aufheller, Duftstoffe, weitere Enzyme | Rest | Rest | Rest |

**Tabelle 6: Zweiphasiges Geschirrspülmaschinenmittel**

| **Pulverphase (Phase A)** | **A1** | **A2** |
|---|---|---|
| Aktivstoffgehalt in Gew.-% (soweit nichts anderes angegeben), bezogen auf das Gesamtgewicht der Pulverphase | | |
| Natriumpercarbonat | 13,0 | 15,0 |
| Nichtionisches Tenside | 4,0 | 4,0 |
| Sulfonsäuregruppenhaltiges Polymer | 4,0 | 4,0 |
| HEDP (Natriumsalz) | 6,0 | 6,0 |
| Natriumcarbonat (inkl. Natriumhydrogencarbonat) | 24,0 | 28,0 |
| MGDA (Trinatriumsalz) | 0 | 0 |
| Schichtsilikat (SKS 6 Pulver) | 4,0 | 4,0 |
| Natriumcitrat (als wasserfreies Natriumcitrat berechnet) | 21,0 | 21,0 |
| Amylase (Stainzyme^{®} Plus 24 Evity T; Angabe Gew.-% bezogen auf die Menge der eingesetzten Zubereitung, t.q.) | 1,5 | 1,5 |
| Protease (Gesamtaktivprotein) | 40 mg/Job | 40 mg/Job |
| Misc (u.a. Parfüm, Farbstoffe, Konservierungsmittel, Füllstoffe z.B. Natriumsulfat, Bleichkatalysator (MnTACN), Bleichaktivator (TAED), Zinkacetat, Silberschutz, weitere Enzyme) | Add 100 | Add 100 |
| | | |

| **Gelphase (Phase B)** | **B1** | **B2** |
|---|---|---|
| Aktivstoffgehalt in Gew.-% (soweit nichts anderes angegeben), bezogen auf das Gesamtgewicht der Gelphase | | |
| Polymer umfassend Acrylsäure- und Amidopropylsulfonsäurehaltige Monomere | 10,0 | 11,0 |
| Glycerin | 27,0 | 25,0 |
| 1,3-Propandiol | 30,0 | 30,0 |
| PEG 400 | 15,0 | 17,0 |
| PVOH | 15,0 | 14,0 |
| Misc (u.a. Prozesshilfsmittel, pH-Stellmittel, Parfüm, Farbstoff) | Add 100 | Add 100 |
| Gelierzeit / min | weniger als1 | weniger als1 |
| | | |
| Die Phasen A1 bzw. A2 und die Phasen B1 bzw. B2 können beliebig miteinander kombiniert werden. Gesamtgewicht beider Phasen in einer Einmalportion von 18,5 g. | | |

## Patentansprüche

1. Protease, die proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease bezogen auf die Nummerierung gemäß SEQ ID NO:1 an mindestens einer der Positionen, die den Positionen 97, 99, 127 und 211 entsprechen, mindestens eine Aminosäuresubstitution, die aus der aus N97E, R99E, R99I, R99N, R99V, R99T, D127E und M211C bestehenden Gruppe ausgewählt ist, aufweist.

2. Protease nach Anspruch 1, wobei die Protease proteolytische Aktivität aufweist und eine Aminosäuresequenz umfasst, die zu der in SEQ ID NO:1 angegebenen Aminosäuresequenz über deren Gesamtlänge zu mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5% oder 99% identisch ist, wobei die Protease eine Aminosäuresubstitution bzw. Aminosäuresubstitutionskombination aufweist, die aus der aus N97E, R99E, R99I, R99N, R99V, R99T, D127E, M211C, N97E-R99E, N97E-R99I, N97E-R99N, N97E-R99V, N97E-R99T, N97E-D127E, N97E-M211C, R99E-D127E, R99E-M211C, R99I-D127E, R99O-M211C, R99N-D127E, R99N-M211C, R99V-D127E, R99V-M211C, R99T-D127E, R99T-M211C, D127E-M211C, N97E-R99E-D127E, N97E-R99I-D127E, N97E-R99N-D127E, N97E-R99V-D127E, N97E-R99T-D127E, N97E-M211C-D127E, N97E-R99E-M211C, N97E-R991-M211C, N97E-R99N-M211C, N97E-R99V-M211C, N97E-R99T-M211C, N97E-D127E-M211C, R99E-D127E-M211C, R991-D127E-M211C, R99N-D127E-M211C, R99V-D127E-M211C, R99T-D127E-M211C, N97E-R99E-D127E-M211C, N97E-R99I-D127E-M211C, N97E-R99N-D127E-M211C, N97E-R99V-D127E-M211C und N97E-R99T-D127E-M211C, bevorzugt N97E, R99E, R99I, R99N, R99V, R99T, D127E, M211C, D127E-M211C und N97E-R991 bestehenden Kombination ausgewählt ist.

3. Protease nach Anspruch 1 oder 2, wobei die Protease eine Aminosäure bzw. Aminosäuresubstitutionskombination aufweist, die aus der aus N97E, R99E, R99I, R99N, R99V, R99T, D127E, M211C, N97E-R99E, N97E-R99I, N97E-R99N, N97E-R99V, N97E-R99T, N97E-D127E, N97E-M211C, R99E-D127E, R99E-M211C, R991-D127E, R991-M211C, R99N-D127E, R99N-M211C, R99V-D127E, R99V-M211C, R99T-D127E, R99T-M211C, D127E-M211C, N97E-R99E-D127E, N97E-R99I-D127E, N97E-R99N-D127E, N97E-R99V-D127E, N97E-R99T-D127E, N97E-M211C-D127E, N97E-R99E-M211C, N97E-R991-M211C, N97E-R99N-M211C, N97E-R99V-M211C, N97E-R99T-M211C, N97E-D127E-M211C, R99E-D127E-M211C, R991-D127E-M211C, R99N-D127E-M211C, R99V-D127E-M211C, R99T-D127E-M211C, N97E-R99E-D127E-M211C, N97E-R99I-D127E-M211C, N97E-R99N-D127E-M211C, N97E-R99V-D127E-M211C und N97E-R99T-D127E-M211C, bevorzugt N97E, R99E, R99I, R99N, R99V, R99T, D127E, M211C, D127E-M211C und N97E-R99I, bestehenden Gruppe ausgewählt ist, wobei die Nummerierung jeweils bezogen auf die Nummerierung gemäß SEQ ID NO:1 ist und wobei die Protease neben den genannten Aminosäuresubstitutionen keine weiteren Änderungen umfasst.

4. Protease, **dadurch gekennzeichnet, dass**
(a) sie aus einer Protease nach einem der Ansprüche 1 bis 3 als Ausgangsmolekül erhältlich ist durch ein- oder mehrfache konservative Aminosäuresubstitution, wobei die Protease, bezogen auf die Nummerierung gemäß SEQ ID NO:1 an mindestens einer der Positionen, die den Positionen 97, 99, 127 und 211 entsprechen, mindestens eine Aminosäuresubstitution, die aus der aus N97E, R99E, R99I, R99N, R99V, R99T, D127E und M211C bestehenden Gruppe ausgewählt ist, aufweist;
(b) sie aus einer Protease nach einem der Ansprüche 1 bis 3 als Ausgangsmolekül erhältlich ist durch Fragmentierung, Deletions-, Insertions- oder Substitutionsmutagenese und eine Aminosäuresequenz umfasst, die über eine Länge von mindestens 190, 200, 210, 220, 230, 240, 250, 255, 256, 257, 258, 259, 260, 261, 262, 263, 264, 265, 266, 267, 268 oder 269 zusammenhängenden Aminosäuren mit dem Ausgangsmolekül übereinstimmt, wobei die Protease bezogen auf die Nummerierung gemäß SEQ ID NO:1 an mindestens einer der Positionen, die den Positionen 97, 99, 127 und 211 entsprechen, mindestens eine Aminosäuresubstitution, die aus der aus N97E, R99E, R99I, R99N, R99V, R99T, D127E und M211C bestehenden Gruppe ausgewählt ist, aufweist.

5. Verfahren zur Herstellung einer Protease, umfassend das Einbringen mindestens einer Aminosäuresubstitution an mindestens einer der Positionen, die bezogen auf die Nummerierung gemäß SEQ ID NO:1 den Positionen 97, 99, 127 und 211 entsprechen, ausgewählt aus der aus N97E, R99E, R99I, R99N, R99V, R99T, D127E und M211C bestehenden Gruppe, in ein Ausgangsmolekül, das eine Aminosäuresequenz aufweist, die mindestens 70% und zunehmend bevorzugt zu mindestens 71%, 72%, 73%, 74%, 75%, 76%, 77%, 78%, 79%, 80%, 81%, 82%, 83%, 84%, 85%, 86%, 87%, 88%, 89%, 90%, 90,5%, 91%, 91,5%, 92%, 92,5%, 93%, 93,5%, 94%, 94,5%, 95%, 95,5%, 96%, 96,5%, 97%, 97,5%, 98%, 98,5%, 99%, 99,5% oder 100% Sequenzidentität mit der in SEQ ID NO:1 angegebenen Aminosäuresequenz über deren Gesamtlänge aufweist.

6. Verfahren nach Anspruch 5, ferner umfassend einen oder mehreren der folgenden Verfahrensschritte:
(a) Einbringen einer ein- oder mehrfachen konservativen Aminosäuresubstitution, wobei die Protease bezogen auf die Nummerierung gemäß SEQ ID NO:1 an mindestens einer der Positionen, die den Positionen 97, 99, 127 und 211 entsprechen, mindestens eine Aminosäuresubstitution, die aus der aus N97E, R99E, R99I, R99N, R99V, R99T, D127E und M211C bestehenden Gruppe ausgewählt ist, aufweist;
(b) Veränderung der Aminosäuresequenz durch Fragmentierung, Deletions-, Insertions- oder Substitutionsmutagenese derart, dass die Protease eine Aminosäuresequenz umfasst, die über eine Länge von mindestens 190, 200, 210, 220, 230, 240, 250, 255, 256, 257, 258, 259, 260, 261, 262, 263, 264, 265, 266, 267, 268 oder 269 zusammenhängenden Aminosäuren mit dem Ausgangsmolekül übereinstimmt, wobei die Protease bezogen auf die Nummerierung gemäß SEQ ID NO:1 an mindestens einer der Positionen, die den Positionen 97, 99, 127 und 211 entsprechen, mindestens eine Aminosäuresubstitution, die aus der aus N97E, R99E, R99I, R99N, R99V, R99T, D127E und M211C bestehenden Gruppe ausgewählt ist, aufweist.

7. Nukleinsäure codierend für eine Protease nach einem der Ansprüche 1 bis 4 oder codierend für eine nach einem Verfahren der Ansprüche 5 bis 6 erhaltene Protease.

8. Vektor enthaltend eine Nukleinsäure nach Anspruch 7, insbesondere ein Klonierungsvektor oder ein Expressionsvektor.

9. Nicht-menschliche Wirtszelle, die eine Nukleinsäure nach Anspruch 7 oder einen Vektor nach Anspruch 8 beinhaltet, oder die eine Protease nach einem der Ansprüche 1 bis 4 beinhaltet, oder die eine nach einem Verfahren der Ansprüche 5 bis 6 erhaltene Protease beinhaltet, insbesondere eine, die die Protease in das die Wirtszelle umgebende Medium sezerniert.

10. Verfahren zur Herstellung einer Protease umfassend
a) Kultivieren einer Wirtszelle gemäß Anspruch 9 und
b) Isolieren der Protease aus dem Kulturmedium oder aus der Wirtszelle.

11. Wasch- oder Reinigungsmittel, insbesondere flüssiges Textilwaschmittel, **dadurch gekennzeichnet, dass** es mindestens eine Protease nach einem der Ansprüche 1 bis 4 oder eine nach einem Verfahren der Ansprüche 5 bis 6 erhaltene Protease enthält.

12. Verfahren zur Reinigung von Textilien und/oder harten Oberflächen, insbesondere Geschirr, **dadurch gekennzeichnet, dass** in mindestens einem Verfahrensschritt ein Mittel nach Anspruch 11 angewendet wird.

13. Verfahren zur Entfernung von proteasesensitiven Anschmutzungen, insbesondere ei(gelb)-, milch-, blut- und/oder fleischhaltigen Anschmutzungen, bevorzugt ei(gelb)- und/oder milchhaltigen Anschmutzungen, von Textilien und/oder harten Oberflächen, insbesondere Geschirr, **dadurch gekennzeichnet, dass** in mindestens einem Verfahrensschritt ein Mittel nach Anspruch 11 angewendet wird, und/oder dass in mindestens einem Verfahrensschritt eine Protease nach einem der Ansprüche 1 bis 4 oder eine nach einem der Verfahren der Ansprüche 5 bis 6 erhaltene Protease angewendet wird.

14. Verfahren zur Verbesserung der Reinigungsleistung eines Wasch- und Reinigungsmittels, insbesondere flüssigen Textilwaschmittels, an mindestens einer proteasesensitiven Anschmutzung, insbesondere ausgewählt aus ei(gelb)-, milch-, blut- und/oder fleischhaltigen Anschmutzungen, bevorzugt ei(gelb)- und/oder milchhaltigen Anschmutzungen, wobei das Wasch- und Reinigungsmittel, insbesondere flüssiges Textilwaschmittel, eine Protease nach einem der Ansprüche 1 bis 4 oder eine nach einem Verfahren der Ansprüche 5 bis 6 erhaltene Protease enthält.

15. Verfahren nach einem der Ansprüche 12 bis 14, wobei das Verfahren bei einer Temperatur zwischen etwa 20°C und etwa 40°C, bevorzugt bei etwa 20°C, durchgeführt wird.

16. Verwendung einer Protease nach einem der Ansprüche 1 bis 4 oder eine nach einem Verfahren der Ansprüche 5 bis 6 erhaltene Protease in einem Wasch- oder Reinigungsmittel, insbesondere flüssiges Textilwaschmittel, zur Entfernung von proteasesensitiven Anschmutzungen, insbesondere ei(gelb)-, milch-, blut- und/oder fleischhaltigen Anschmutzungen, bevorzugt ei(gelb)- und/oder milchhaltigen Anschmutzungen von Textilien und/oder harten Oberflächen, insbesondere Geschirr, vorzugsweise bei einer Temperatur zwischen etwa 20°C und etwa 40°C, bevorzugt bei etwa 20°C.
